# EUROPEAN PATENT SPECIFICATION

(11) **EP 3 299 028 B1**
(45) Date of publication and mention of the grant of the patent: **30.10.2024**
(21) Application number: 16796570.6
(22) Date of filing: 19.05.2016
(51) Int. Cl.: A61K 45/06, A61K 38/00, A61K 39/39, A61K 39/395, C07K 14/82, A61P 35/00, A61P 35/02, A61P 43/00

(54) **COMBINATION OF WT1 ANTIGEN PEPTIDE, IMMUNOMODULATOR AND WT1 HELPER PEPTIDE**
KOMBINATION AUS WT1-ANTIGENPEPTID, IMMUNMODULATOR UND WT1-HELFERPEPTID
COMBINAISON DE PEPTIDE D'ANTIGÈNE WT1, D'IMMUNOMODULATEUR ET PEPTIDE AUXILIAIRE WT1

(30) Priority: 20.05.2015 JP 2015103145
(43) Date of publication of application: 28.03.2018
(73) Proprietor: Sumitomo Pharma Co., Ltd., Osaka 541-0045 (JP); International Institute of Cancer Immunology, Inc., Suita-shi, Osaka 564-0053 (JP)
(72) Inventor: TAKASU, Hideo, Kyoto-shi, Kyoto 602-0863 (JP); NAKAMURA, Megumi, Osaka-shi Osaka 554-0022 (JP); GOTO, Masashi, Osaka-shi Osaka 554-0022 (JP); SUGINOBE, Natsuko, Osaka-shi Osaka 554-0022 (JP)
(74) Representative: Vossius & Partner Patentanwälte Rechtsanwälte mbB
(86) International application number: PCT/JP2016/064923
(87) International publication number: WO 2016/186177

(56) References cited:
- EP-A1- 1 584 627
- WO-A1-2014/134165
- JP-A- 2013 531 970
- JP-A- 2014 027 935
- JP-A- 2014 169 282
- JP-A- 2014 221 795
- FU, C. ET AL.: "beta-catenin-mediated inhibition of croos-priming: A new mechanism for tumors to evade immunosurveilance", ONCOIMMUNOLOGY, vol. 2, no. 12, 2013, pages e26920, XP055331155, ISSN: 2162-4011
- STATHER, D. ET AL.: "High PD-1 Levels at Baseline are Associated With Unfavourable Clinical Outocome in a Wilms Tumour Gene 1 (Wt1) Peptide Vaccination Setting in Leukaemia Patients", J IMMUNOTHER, vol. 34, no. 9, 2011, pages 704, XP009507550, ISSN: 1524-9557
- RIETHER, C. ET AL.: "Blocking programmed cell death 1 in combination with adoptive cytotoxic T- cell transfer eradicates chronic myelogenous leukemia stem cells", LEUKEMIA, vol. 29, no. 8, February 2015 (2015-02-01), pages 1781 - 5, XP055331159, ISSN: 1476-5551

## Description

### TECHNICAL FIELD

The present invention relates to a combination use of a WT1 antigen peptide and an immunomodulator.

### BACKGROUND

Cellular immunity, particularly cytotoxic T cells (referred to as CTLs hereinafter) involved therein play an important role to remove tumor cells or virus-infected cells in the living body. CTLs are produced by differentiation and proliferation of precursor T cells that recognizes a complex between an antigen peptide on tumor cells (tumor antigen peptide) and an MHC (Major Histocompatibility Complex) class I molecule. CTLs attack cancer cells.

MHC in human is called human leukocyte-type antigen (HLA) and HLA subtypes such as HLA-A, B and Cw are known. Tumor antigen peptides are produced intracellularly from tumor antigen proteins, which are proteins highly expressed in tumor, when the tumor antigen proteins produced within the cells are degraded by proteases. A tumor antigen peptide thus produced forms a complex with an MHC class I antigen in the endoplasmic reticulum and the complex is delivered to and presented on the cell surface. Tumor-reactive CTLs recognize the tumor antigen peptide (killer peptide) presented on the cell surface and show anti-tumor effects through the cytotoxic activity or production of lymphokines.

Tumor antigen proteins or killer peptides derived therefrom have been considered as active ingredients for cancer immunotherapy (such as cancer vaccines) to treat cancer by potentiating cancer-specific CTLs within the bodies of cancer patients. For example, WT1 (Wilm's tumor 1) -targeted cancer immunotherapies are under development. WT1 has been identified as a responsible gene of Wilms tumor, a kidney cancer in children, and encodes a transcriptional factor having zinc finger motifs (non-patent literature 1). The WT1 gene was initially considered as a tumor-suppressing gene, but later identified as a cancer gene in hematopoietic tumors or solid cancers by subsequent research. The WT1 gene is reported to highly express in various malignant tumors (non-patent literature 2). WT1 is considered as a novel cancer antigen protein in leukemia or solid cancers (non-patent literature 3). Thus, cancer vaccine therapies or dendritic cell therapies using WT1 protein or WT1-derived peptides, TCR-like antibodies that recognize a complex between a WT1-derived peptide and an HLA molecule, or chimeric antigen receptor (CAR) T-cell therapies that use genetically engineered T cells expressing CAR derived from TCR-like antibodies are under development.

For WT1 protein, MHC class I-binding killer peptides such as WT1₁₂₆₋₁₃₄ peptide, WT1₂₃₅₋₂₄₃ peptide, WT1₁₀₋₁₈ peptide, WT1₁₈₇-₁₉₅ peptide, WT1₃₀₂-₃₁₀ peptide, and WT1₃₇-₄₅ peptide are reported (patent literature 1, patent literature 2, non-patent literatures 4 and 5).

In addition to CTLs, helper T (Thl) cells are also play an important role in cancer immunotherapies. Typically, antigenic proteins are intracellularly degraded in lysosomes to provide peptide fragments, and a part of the peptide fragments having about 13-17 amino acids binds to MHC class II molecules as antigen peptides (helper peptides). The complex between the antigen peptide and an MHC class II molecule is presented to the TCR-CD3 complex to activate Th1 cells. The activated Thl cells promote induction and activation of CTLs: Human MHC class II molecules such as HLA-DR, DQ and DP are known and WT1-derived helper peptides have been identified (non-patent literatures 6 and 7).

The immunoregulatory system has been reported to involve stimulatory signals that interact with one another to induce immune suppression or tolerance. T cell activation by antigen presenting cells uses the immunoregulatory system, and agents that interact with costimulatory molecules on the surface of antigen presenting cells or T cells have been reported to regulate costimulatory signals (non-patent literature 8).

As an example, tumor shrinkage is not observed in some cases even when CTLs are present in the tumor. One possible reason is that tumor-infiltrating CTLs are exhausted at an early stage and lose the cytotoxic activity against tumor cells, production ability of various cytokines, and proliferative activity, and results in cell death. The exhaustion has been identified to be induced by negative signals from immune checkpoint molecules expressed on the cell surface of CTLs.

Immune checkpoint molecules such as CTLA-4, PD-1, PD-L1, PD-L2, LAG-3, KIR, TIM-3, B7-H3, B7-H4, VISTA/PD-1H, HVEM, BTLA, CD160, GAL9, TIGIT, PVR, BTNL2, BTN1A1, BTN2A2, BTN3A2, and CD244 have been reported (non-patent literatures 8 and 9). For example, PD-1 is a member of the CD28 receptor family expressed on activated lymphocytes (T cells, B cells, NKT cells) and myeloid cells, and binds to a PD-1 ligand (PD-L1 or PD-L2) expressed on antigen presenting cells to negatively regulate the activated lymphocytes by delivering inhibitory signals to the lymphocytes. In addition to antigen presenting cells, PD-L1 has been reported to express in various tumor tissues. Thus, cancer cells escape from attack by CTLs using PD-L1.

Under the circumstances, inhibitory antibodies against immune checkpoint molecules have recently been developed (non-patent literature 9 and 10) . These antibodies release the exhaustion of CTLs. For example, anti-PD-1 antibodies or anit-PD-L1 antibodies inhibit the binding between PD-1 and PD-L1 and restore the cytotoxic activity of CTLs. Practically, clinical trials of an anti-PD-1 or anit-PD-L1 antibody have been conducted in patients such as those having non-small cell lung cancer or melanoma and significant effects have been observed. The PD-1 or PD-L1 antibody therapies, however, are far from satisfactory because patients showing a market response are 20-30% of total patients and severe immune related adverse events have also been observed.

### CITATION LIST

### PATENT DOCUMENT

Patent Document 1: WO00/06602
Patent Document 2: WO00/18795

### NON PATENT DOCUMENT

Non Patent Document 1: Am J Hum Genet. 1993; 52: 192-203
Non Patent Document 2: Blood.1997; 89: 1405-1412
Non Patent Document 3: Immunogenetics. 2000; 51: 99-107
Non Patent Document 4: Clin Cancer Res. 2005; 11: 8799-807
Non Patent Document 5: Blood. 2008 Oct 1; 112(7): 2956-64
Non Patent Document 6: J Immunother. 2007; 30: 282-93
Non Patent Document 7: Cancer Immunol Immunother. 2010; 59: 1467-79
Non Patent Document 8: Nat Rev Cancer. 2012; 12: 252-64
Non Patent Document 9: Nat Rev Drug Discov. 2015 Aug; 14 (8): 561-8
Non Patent Document 10: Nat Rev Drug Discov. 2013 Feb; 12 (2): 130-46

### SUMMARY

An object of the present invention is to provide methods and pharmaceutical compositions for treating or preventing cancer using a WT1 antigen peptide and an immunomodulator and further a WT1 helper peptide.

One reason why the anti-PD-1 or anit-PD-L1 antibodies do not show sufficient effects could be that the CTL level in tumor is low, or that a strong immune suppressive mechanism other than PD-1/PD-L1 exists. Then, combinations with cancer vaccines or inhibitors of immune checkpoint molecules other than PD-1 and PD-L1 have been conceived. The present inventors have examined combinations between cancer vaccines that increase tumor-reactive CTLs in tumor and immune checkpoint inhibitors or other immunomodulators. Trough intensive studies using mice, the inventors have demonstrated that the administration of a WT1 antigen peptide induces expression of immune checkpoint molecules in CD8⁺ T cells, in particular WT1-speciifc killer T cells, and CD4⁺ T cells; and that the activation of the induced WT1-speciifc killer T cells is enhanced by an immunomodulator such as an immune checkpoint inhibitor. Further, trough intensive studies using human peripheral blood mononuclear cells, the inventors have demonstrated that WT1-speciifc killer T cells are efficiently induced from naive T cells when a WT1 antigen peptide is combined with an immunomodulator; and that the activation of the WT1-speciifc killer T cells induced with a WT1 antigen peptide is enhanced by an immunomodulator. The inventors have further tried to improve the effects of the combination, and finally demonstrated that using a cancer vaccine comprising both a WT1 killer peptide and a WT1 helper peptide in the combination induces CTLs that are not suppressed by cancer cells and thus significantly improves the effects of the combination with an immunomodulator.

The present invention relates to the embodiments as characterized in the claims. Thus, it relates to the following items.
1. A pharmaceutical composition for use in treating or preventing cancer, wherein said pharmaceutical composition is to be parenterally administered by injection, comprising:
   (i) a WT1 antigen peptide or a pharmaceutically acceptable salt thereof, wherein the pharmaceutical composition is used in combination with an immunomodulator,
   (ii) an immunomodulator, wherein the pharmaceutical composition is used in combination with a WT1 antigen peptide or a pharmaceutically acceptable salt thereof, or
   (iii)a WT1 antigen peptide or a pharmaceutically acceptable salt thereof and an immunomodulator,
      wherein the pharmaceutical composition further comprises a WT1 helper peptide or a pharmaceutically acceptable salt thereof, or the pharmaceutical composition is used in combination with a WT1 helper peptide or a pharmaceutically acceptable salt thereof,
      wherein said WT1 antigen peptide or a pharmaceutically acceptable salt thereof is
         a peptide consisting of the amino acid sequence selected from
         RMFPNAPYL (SEQ ID NO: 2),
         CMTWNQMNL (SEQ ID NO: 3),
         CYTWNQMNL (SEQ ID NO: 4),
         ALLPAVPSL (SEQ ID NO: 5),
         SLGEQQYSV (SEQ ID NO: 6),
         RVPGVAPTL (SEQ ID NO: 7),
         VLDFAPPGA (SEQ ID NO: 8),
         C-CMTWNQMNL (SEQ ID NO: 9) (wherein the bond within C-C is a disulfide bond),
         C-CYTWNQMNL (SEQ ID NO: 10) (wherein the bond within C-C is a disulfide bond),
         RYFPNAPYL (SEQ ID NO: 21), and
         YMFPNAPYL (SEQ ID NO: 26);
         a peptide comprising an altered amino acid sequence of the amino acid sequence selected from SEQ ID NOS: 2-10, 21 and 26 that comprises deletion, substitution, or addition of one to several amino acids in the amino acid sequence and having a CTL induction activity; or
         a compound selected from the group consisting of the compound of formula (1): 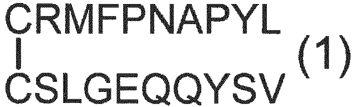
         (wherein the bond within C-C is a disulfide bond),
         the compound of formula (2): 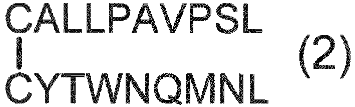
         (wherein the bond within C-C is a disulfide bond), and
         the compound of formula (3): 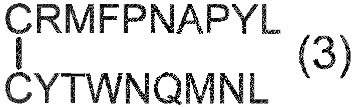
         (wherein the bond within C-C is a disulfide bond);
         or a pharmaceutically acceptable salt thereof, and
      the WT1 helper peptide or a pharmaceutically acceptable salt thereof is a peptide consisting of the amino acid sequence selected from
         KRYFKLSHLQMHSRKH (SEQ ID NO: 11),
         SGQARMFPNAPYLPSCLES (SEQ ID NO: 12),
         RSDELVRHHNMHQRNMTKL (SEQ ID NO: 13),
         PGCNKRYFKLSHLQMHSRKHTG (SEQ ID NO: 14),
         CNKRYFKLSHLQMHSRK (SEQ ID NO: 15),
         CNKRYFKLSHLQMHSRKH (SEQ ID NO: 16),
         CNKRYFKLSHLQMHSRKHTG (SEQ ID NO: 17),
         WAPVLDFAPPGASAYGSL (SEQ ID NO: 18),
         CWAPVLDFAPPGASAYGSL (SEQ ID NO: 19),
         WAPVLDFAPPGASAYGSLC (SEQ ID NO: 20), and
         SGQAYMFPNAPYLPSCLES (SEQ ID NO: 37); or
         a peptide comprising an altered amino acid sequence of the amino acid sequence selected from SEQ ID NOs: 11-20 and 37 that comprises deletion, substitution, or addition of one to several amino acids in the amino acid sequence and having a helper T cell induction activity; or a pharmaceutically acceptable salt thereof, and
      the immunomodulator is at least one agent selected from the group consisting of
         (a) an immune checkpoint inhibitor,
            the immune checkpoint inhibitor is at least one agent directed to a molecule selected from the group consisting of
            (1) CTLA-4,
            (2) PD-1,
            (3) LAG-3,
            (4) BTLA,
            (5) KIR,
            (6) TIM-3,
            (7) PD-L1,
            (8) PD-L2,
            (9) B7-H3,
            (10) B7-H4,
            (11) HVEM,
            (12) GAL9,
            (13) CD160,
            (14) VISTA,
            (15) BTNL2,
            (16) TIGIT,
            (17) PVR,
            (18) BTN1A1,
            (19) BTN2A2,
            (20) BTN3A2, and
            (21) CSF-1R,
         (b) a costimulatory molecule agonist,
            the costimulatory molecule agonist is at least one agent directed to a molecule selected from the group consisting of
            (1) 4-1BB,
            (2) 4-1BB-L,
            (3) OX40,
            (4) OX40-L,
            (5) GITR,
            (6) CD28,
            (7) CD40,
            (8) CD40-L,
            (9) ICOS,
            (10) ICOS-L,
            (11) LIGHT, and
            (12) CD27
         (c) a Toll-like receptor (TLR) agonist,
            the TLR agonist is at least one agent selected from the group consisting of
            (1) a TLR1/2 agonist,
            (2) a TLR2 agonist,
            (3) a TLR3 agonist,
            (4) a TLR4 agonist,
            (5) a TLR5 agonist,
            (6) a TLR6/2 agonist,
            (7) a TLR7 agonist,
            (8) a TLR7/8 agonist,
            (9) a TLR7/9 agonist,
            (10) a TLR8 agonist,
            (11) a TLR9 agonist, and
            (12) a TLR11 agonist, and
         (d) a low-molecular inhibitor
            the low-molecular inhibitor is at least one agent selected from the group consisting of
            (1) a β-catenin inhibitor,
            (2) a IDO inhibitor,
            (3) a COX-2 inhibitor,
            (4) a CXCR4 inhibitor,
            (5) a STAT3 inhibitor, and
            (6) a multikinase inhibitor.
2. The pharmaceutical composition for use according to item 1, wherein said pharmaceutical composition is to be parenterally administered by injection,
   wherein the WT1 antigen peptide or a pharmaceutically acceptable salt thereof is the compound of formula (3): 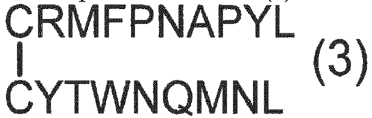
   (wherein the bond within C-C is a disulfide bond);
   or a pharmaceutically acceptable salt thereof.
3. The pharmaceutical composition for use according to item 1 or 2, wherein said pharmaceutical composition is to be parenterally administered by injection,
   wherein the WT1 helper peptide or a pharmaceutically acceptable salt thereof is a peptide consisting of the amino acid sequence selected from
   KRYFKLSHLQMHSRKH (SEQ ID NO: 11),
   SGQARMFPNAPYLPSCLES (SEQ ID NO: 12),
   RSDELVRHHNMHQRNMTKL (SEQ ID NO: 13),
   PGCNKRYFKLSHLQMHSRKHTG (SEQ ID NO: 14),
   CNKRYFKLSHLQMHSRK (SEQ ID NO: 15),
   CNKRYFKLSHLQMHSRKH (SEQ ID NO: 16),
   CNKRYFKLSHLQMHSRKHTG (SEQ ID NO: 17),
   WAPVLDFAPPGASAYGSL (SEQ ID NO: 18),
   CWAPVLDFAPPGASAYGSL (SEQ ID NO: 19),
   WAPVLDFAPPGASAYGSLC (SEQ ID NO: 20), and
   SGQAYMFPNAPYLPSCLES (SEQ ID NO: 37) or
   a pharmaceutically acceptable salt thereof.
4. The pharmaceutical composition for use according to any one of items 1-3, wherein said pharmaceutical composition is to be parenterally administered by injection,
   wherein the WT1 helper peptide or a pharmaceutically acceptable salt thereof is WAPVLDFAPPGASAYGSL (SEQ ID NO: 18),
   or a pharmaceutically acceptable salt thereof.
5. The pharmaceutical composition for use according to any one of items 1-4, wherein said pharmaceutical composition is to be parenterally administered by injection, wherein the pharmaceutical composition is used as a cancer vaccine.
6. The pharmaceutical composition for use according to any one of items 1-5, wherein said pharmaceutical composition is to be parenterally administered by injection, wherein the immune checkpoint inhibitor is at least one agent directed to a molecule selected from the group consisting of CTLA-4, PD-1, LAG-3, BTLA, TIM-3, PD-L1, B7-H4, HVEM, CD160, VISTA, TIGIT and PVR.
7. The pharmaceutical composition for use according to any one of items 1-6, wherein said pharmaceutical composition is to be parenterally administered by injection, wherein the immune checkpoint inhibitor is an antibody.
8. The pharmaceutical composition for use according to item 7, wherein said pharmaceutical composition is to be parenterally administered by injection, wherein the immune checkpoint inhibitor is an antibody against PD-1 or PD-L1.
9. The pharmaceutical composition for use according to item 8, wherein said pharmaceutical composition is to be parenterally administered by injection, wherein the antibody against PD-1 is Nivolumab or Pembrolizumab.
10. The pharmaceutical composition for use according to item 8, wherein said pharmaceutical composition is to be parenterally administered by injection, wherein the antibody against PD-L1 is Durvalumab, MPDL3280A or BMS-936559.
11. The pharmaceutical composition for use according to any one of items 1-5, wherein said pharmaceutical composition is to be parenterally administered by injection, wherein the costimulatory molecule agonist is at least one agent directed to a molecule selected from the group consisting of 4-1BB, OX40, GITR, CD40 and ICOS.
12. The pharmaceutical composition for use according to any one of items 1-5, wherein said pharmaceutical composition is to be parenterally administered by injection, wherein the immune activating agent is at least one of Toll-like receptor (TLR) agonists selected from the group consisting of a TLR3 agonist, a TLR7 agonist, a TLR7/8 agonist and a TLR9 agonist.
13. The pharmaceutical composition for use according to any one of items 1-5, wherein said pharmaceutical composition is to be parenterally administered by injection, wherein the low-molecular inhibitor is a β-catenin inhibitor.
14. The pharmaceutical composition for use according to any one of items 1-13, wherein said pharmaceutical composition is to be parenterally administered by injection, wherein the cancer is selected from the group consisting of leukemia, myelodysplastic syndrome, multiple myeloma, malignant lymphoma, gastric cancer, colorectal cancer, lung cancer, breast cancer, germ cell cancer, liver cancer, skin cancer, urinary bladder cancer, prostate cancer, uterine cancer, cervical cancer, ovarian cancer, brain tumor, bone cancer, pancreatic cancer, cancer of the head or neck, cutaneous or intraocular malignant melanoma, rectal cancer, cancer of the anal region, testicular cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia such as acute myeloid leukemia, chronic myeloid leukemia, acute lymphoblastic leukemia, or chronic lymphocytic leukemia, childhood solid tumor, lymphocytic lymphoma, cancer of the kidney or ureter, carcinoma of the renal pelvis, central nervous system (CNS) tumor, primary CNS lymphoma, tumor angiogenesis, spinal tumor, brainstem glioma, pituitary adenoma, Kaposi's sarcoma, epidermoid cancer, squamous cell cancer, T-cell lymphoma, glioblastoma multiforme, malignant melanoma, non-small cell lung cancer, renal cell carcinoma, and asbestos-induced cancer
15. A kit for use in treating or preventing cancer, comprising the WT1 antigen peptide or a pharmaceutically acceptable salt thereof, the WT1 helper peptide or a pharmaceutically acceptable salt thereof, and the immunomodulator as defined in any one of items 1-14 which are to be parenterally administered by injection.

Further, for reference only, there is disclosed the following:
a method for treating or preventing cancer, comprising administering a WT1 antigen peptide or a pharmaceutically acceptable salt thereof and an immunomodulator to a mammal; a WT1 antigen peptide or a pharmaceutically acceptable salt thereof for use in the treatment or prevention of cancer, wherein the WT1 antigen peptide or a pharmaceutically acceptable salt thereof is used in combination with an immunomodulator;
an immunomodulator for use in the treatment or prevention of cancer, wherein the immunomodulator is used in combination with a WT1 antigen peptide or a pharmaceutically acceptable salt thereof;
use of a WT1 antigen peptide or a pharmaceutically acceptable salt thereof for the manufacture of a medicament for the treatment or prevention of cancer, wherein WT1 antigen peptide or a pharmaceutically acceptable salt thereof is used in combination with an immunomodulator;
use of an immunomodulator for the manufacture of a medicament for the treatment or prevention of cancer, wherein the immunomodulator is used in combination with a WT1 antigen peptide or a pharmaceutically acceptable salt thereof; and
use of a WT1 antigen peptide or a pharmaceutically acceptable salt thereof and an immunomodulator for the manufacture of a medicament for the treatment or prevention of cancer.

Further, for reference only, there is disclosed the following:
a method for treating or preventing cancer, comprising administering a WT1 antigen peptide or a pharmaceutically acceptable salt thereof and an immune checkpoint inhibitor to a mammal;
a WT1 antigen peptide or a pharmaceutically acceptable salt thereof for use in the treatment or prevention of cancer, wherein the WT1 antigen peptide or a pharmaceutically acceptable salt thereof is used in combination with an immune checkpoint inhibitor;
an immune checkpoint inhibitor for use in the treatment or prevention of cancer, wherein the immune checkpoint inhibitor is used in combination with a WT1 antigen peptide or a pharmaceutically acceptable salt thereof;
use of a WT1 antigen peptide or a pharmaceutically acceptable salt thereof for the manufacture of a medicament for the treatment or prevention of cancer, wherein WT1 antigen peptide or a pharmaceutically acceptable salt thereof is used in combination with an immune checkpoint inhibitor;
use of an immune checkpoint inhibitor for the manufacture of a medicament for the treatment or prevention of cancer, wherein the immune checkpoint inhibitor is used in combination with a WT1 antigen peptide or a pharmaceutically acceptable salt thereof; and
use of a WT1 antigen peptide or a pharmaceutically acceptable salt thereof and an immune checkpoint inhibitor for the manufacture of a medicament for the treatment or prevention of cancer.

### BRIEF DESCRIPTION OF DRAWINGS

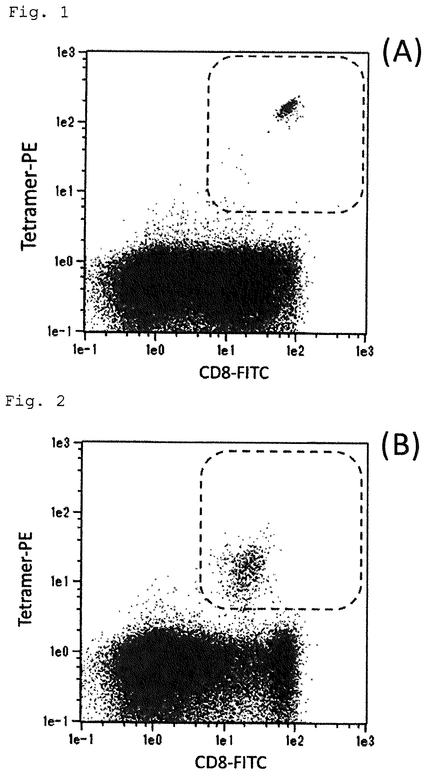

Fig. 1 shows the detection of killer peptide B-specific CTLs in PBMCs treated with an anti-PD-1 antibody. The horizontal axis shows the staining intensity with an anti-CD8 antibody, and the vertical axis shows the staining intensity with an HLA-tetramer. The dots in the dashed-line box show WT1 antigen peptide-specific CTLs.
Fig. 2 shows the detection of killer peptide A-specific CTLs in PBMCs treated with an anit-PD-L1 antibody. The horizontal axis shows the staining intensity with an anti-CD8 antibody, and the vertical axis shows the staining intensity with an HLA-tetramer. The dots in the dashed-line box show WT1 antigen peptide-specific CTLs.

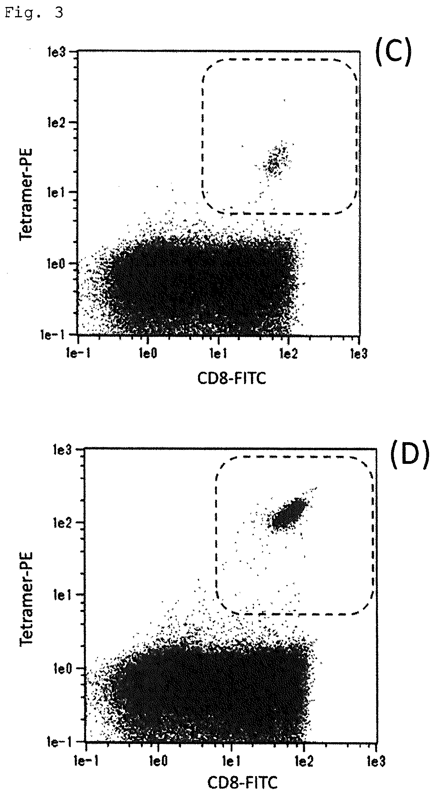

Fig. 3 shows the detection of killer peptide B-specific CTLs in PBMCs treated with an anit-PD-L1 antibody. The horizontal axis shows the staining intensity with an anti-CD8 antibody, and the vertical axis shows the staining intensity with an HLA-tetramer. The dots in the dashed-line box show WT1 antigen peptide-specific CTLs.

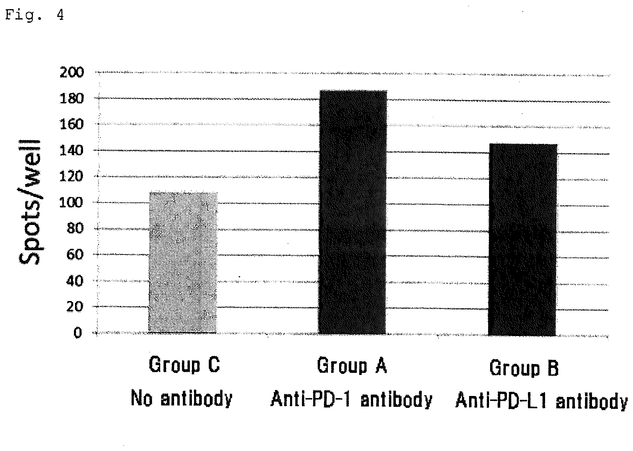

Fig. 4 shows the detection of WT1 antigen peptide-specific CTLs. The vertical axis shows the number of spots detected in the IFN-γ ELISPOT assay.

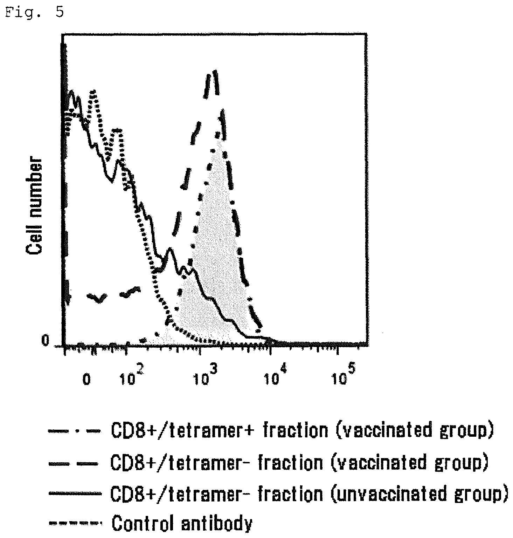

Fig. 5 shows the detection of PD-1 in CD8⁺ T cells. The change of PD-1 expression by vaccine administration in mouse spleen cells is shown. The horizontal axis shows the staining intensity of PD-1 by flow cytometry analysis. The dashed dotted line, dashed line, and solid line indicate the results of analysis of CD8⁺, tetramer⁺ fraction of spleen cells from a vaccinated mouse; CD8⁺, tetramer⁻fraction of spleen cells from a vaccinated mouse; and CD8⁺, tetramer- fraction of spleen cells from an unvaccinated mouse, respectively. The dotted line indicates the result of staining with an isotype control.

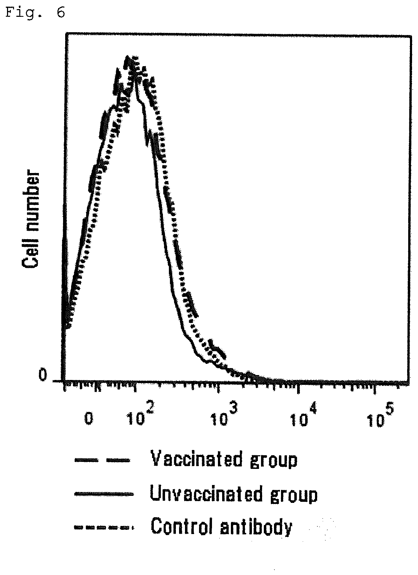

Fig. 6 shows the detection of PD-1 in CD4⁺ T cells. The change of PD-1 expression by vaccine administration in mouse spleen cells is shown. The horizontal axis shows the staining intensity of PD-1 by flow cytometry analysis. The dashed line and solid line indicate the results of analysis of CD4⁺ T cells from spleen cells of a vaccinated mouse and an unvaccinated mouse, respectively. The dotted line indicates the result of staining with an isotype control.

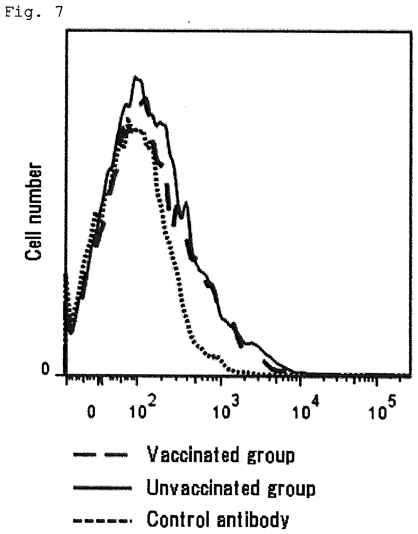

Fig. 7 shows the detection of PD-1 in CD4⁻/CD8⁻ T cells. The change of PD-1 expression by vaccine administration in mouse spleen cells is shown. The horizontal axis shows the staining intensity of PD-1 by flow cytometry analysis. The dashed line and solid line indicate the results of analysis of CD4⁻/CD8⁻ T cells from spleen cells of a vaccinated mouse and an unvaccinated mouse, respectively. The dotted line indicates the result of staining with an isotype control.

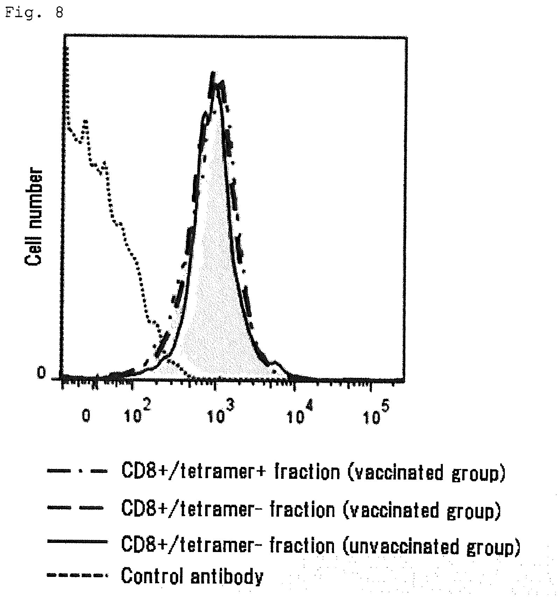

Fig. 8 shows the detection of PD-L1 in CD8⁺ T cells. The change of PD-L1 expression by vaccine administration in mouse spleen cells is shown. The horizontal axis shows the staining intensity of PD-L1 by flow cytometry analysis. The dashed dotted line, dashed line, and solid line indicate the results of analysis of CD8⁺, tetramer⁺ fraction of spleen cells from a vaccinated mouse; CD8⁺, tetramer⁻fraction of spleen cells from a vaccinated mouse; and CD8⁺, tetramer- fraction of spleen cells from an unvaccinated mouse, respectively. The dotted line indicates the result of staining with an isotype control.

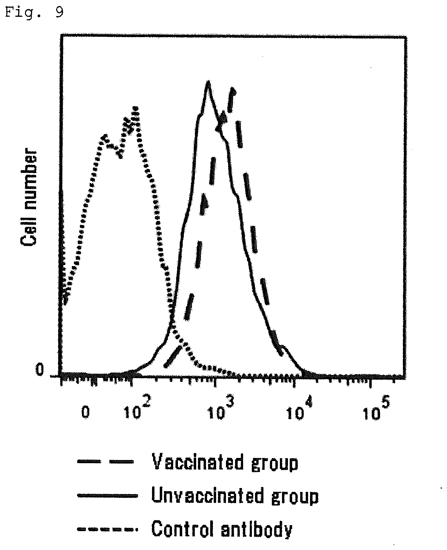

Fig. 9 shows the detection of PD-L1 in CD4⁺ T cells. The change of PD-L1 expression by vaccine administration in mouse spleen cells is shown. The horizontal axis shows the staining intensity of PD-L1 by flow cytometry analysis. The dashed line and solid line indicate the results of analysis of CD4⁺ T cells from spleen cells of a vaccinated mouse and an unvaccinated mouse, respectively. The dotted line indicates the result of staining with an isotype control.

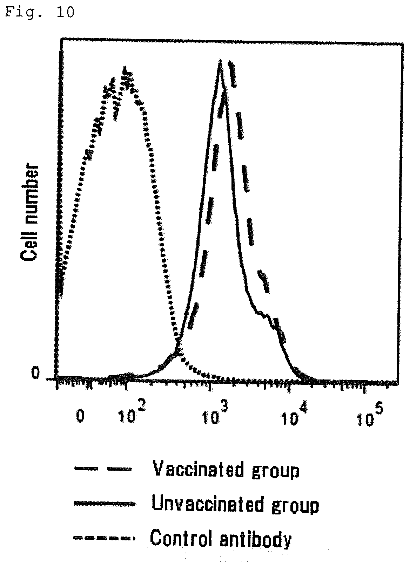

Fig. 10 shows the detection of PD-L1 in CD4⁻/CD8⁻ T cells. The change of PD-L1 expression by vaccine administration in mouse spleen cells is shown. The horizontal axis shows the staining intensity of PD-L1 by flow cytometry analysis. The dashed line and solid line indicate the results of analysis of CD9⁻JCDS⁻ T cells from spleen cells of a vaccinated mouse and an unvaccinated mouse, respectively. The dotted line indicates the result of staining with an isotype control.

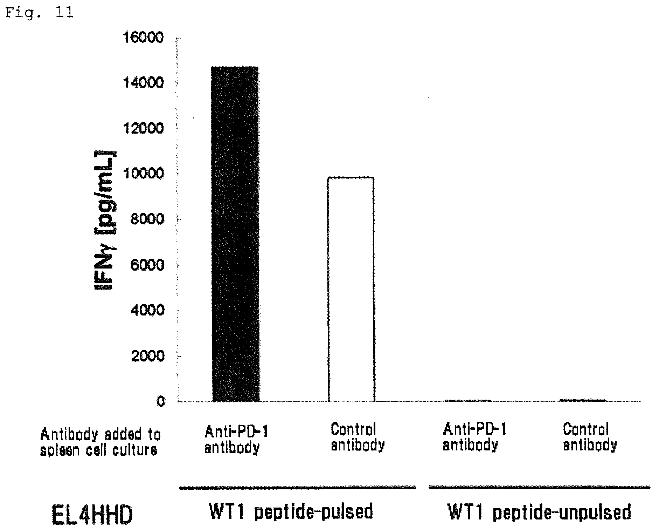

Fig. 11 shows the IFN-γ production by the treatment with an anti-PD-1 antibody. WT1 antigen peptide-specific T cells induced in a vaccinated mouse were treated with an anti-PD-1 antibody, and the IFN-γ production from the cells when cocultured with EL4HHD tumor cells was measured by ELISA.

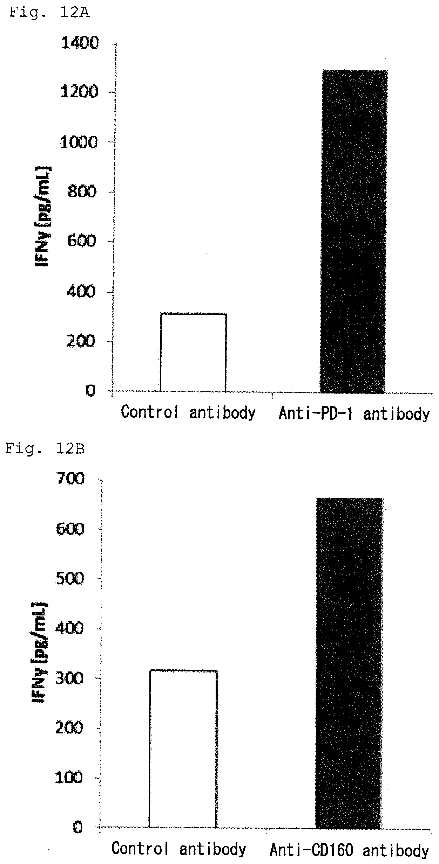

Fig. 12A shows the IFN-γ production by the treatment with an immune checkpoint inhibitor. WT1 antigen peptide-specific T cells induced in a vaccinated mouse were treated with an anti-PD-1 or an isotype control antibody, and the IFN-γ production from the cells when cocultured with a WT1 killer peptide and LLC-HHD-WT1 tumor cells was measured by ELISA.
Fig. 12B shows the IFN-γ production by the treatment with an immune checkpoint inhibitor. WT1 antigen peptide-specific T cells induced in a vaccinated mouse were treated with an anti-CD160 or an isotype control antibody, and the IFN-γ production from the cells when cocultured with a WT1 killer peptide and LLC-HHD-WT1 tumor cells was measured by ELISA.

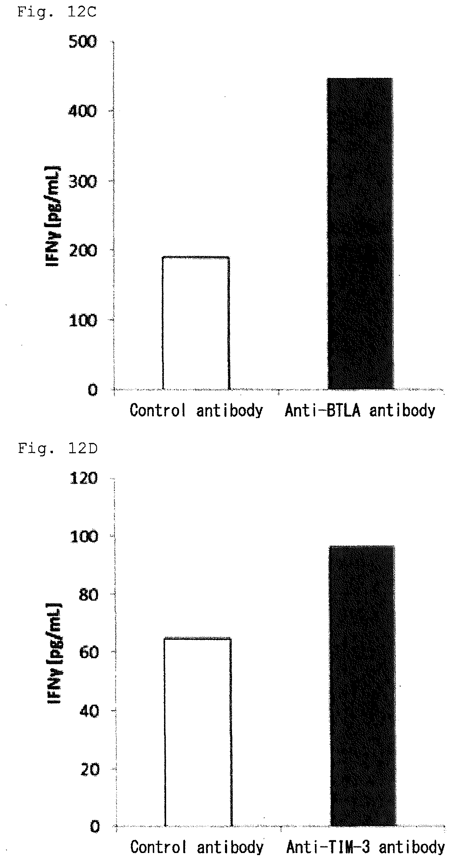

Fig. 12C shows the IFN-γ production by the treatment with an immune checkpoint inhibitor. WT1 antigen peptide-specific T cells induced in a vaccinated mouse were treated with an anti-BTLA or an isotype control antibody, and the IFN-γ production from the cells when cocultured with a WT1 killer peptide and LLC-HHD-WT1 tumor cells was measured by ELISA.
Fig. 12D shows the IFN-γ production by the treatment with an immune checkpoint inhibitor. WT1 antigen peptide-specific T cells induced in a vaccinated mouse were treated with an anti-TIM-3 or an isotype control antibody, and the IFN-γ production from the cells when cocultured with a WT1 killer peptide and LLC-HHD-WT1 tumor cells was measured by ELISA.

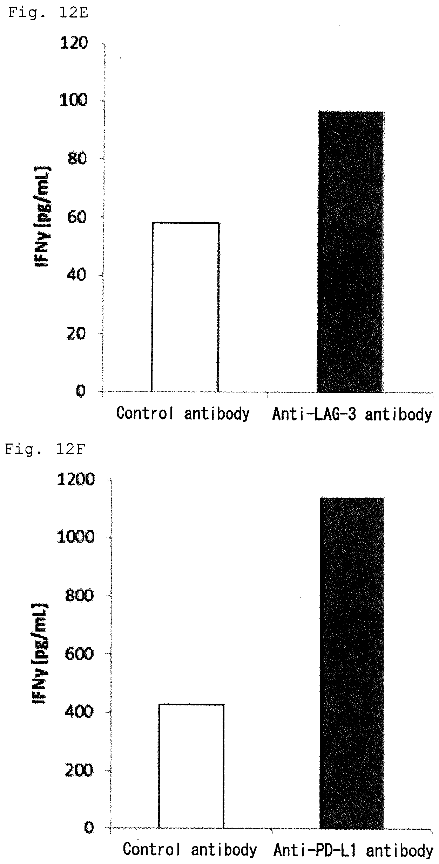

Fig. 12E shows the IFN-γ production by the treatment with an immune checkpoint inhibitor. WT1 antigen peptide-specific T cells induced in a vaccinated mouse were treated with an anti-LAG-3 or an isotype control antibody, and the IFN-γ production from the cells when cocultured with a WT1 killer peptide and LLC-HHD-WT1 tumor cells was measured by ELISA.
Fig. 12F shows the IFN-γ production by the treatment with an immune checkpoint inhibitor. WT1 antigen peptide-specific T cells induced in a vaccinated mouse were treated with an anti-PD-L1 or an isotype control antibody, and the IFN-γ production from the cells when cocultured with a WT1 killer peptide and LLC-HHD-WT1 tumor cells was measured by ELISA.

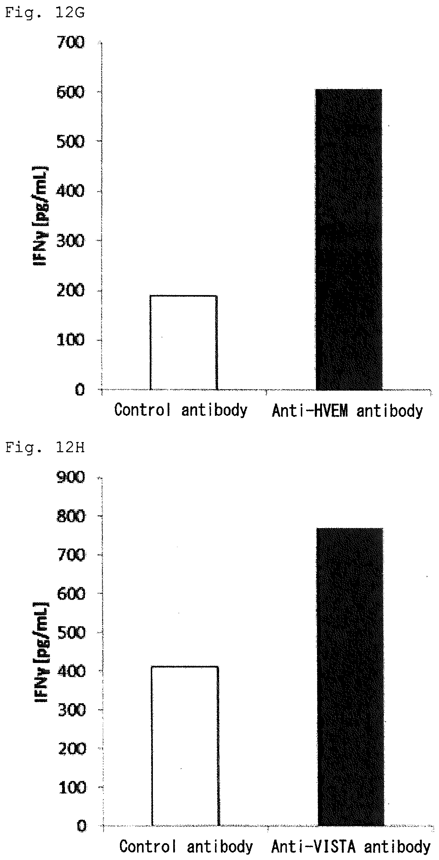

Fig. 12G shows the IFN-γ production by the treatment with an immune checkpoint inhibitor. WT1 antigen peptide-specific T cells induced in a vaccinated mouse were treated with an anti-HVEM or an isotype control antibody, and the IFN-γ production from the cells when cocultured with a WT1 killer peptide and LLC-HHD-WT1 tumor cells was measured by ELISA.
Fig. 12H shows the IFN-γ production by the treatment with an immune checkpoint inhibitor. WT1 antigen peptide-specific T cells induced in a vaccinated mouse were treated with an anti-VISTA or an isotype control antibody, and the IFN-γ production from the cells when cocultured with a WT1 killer peptide and LLC-HHD-MT1 tumor cells was measured by ELISA.

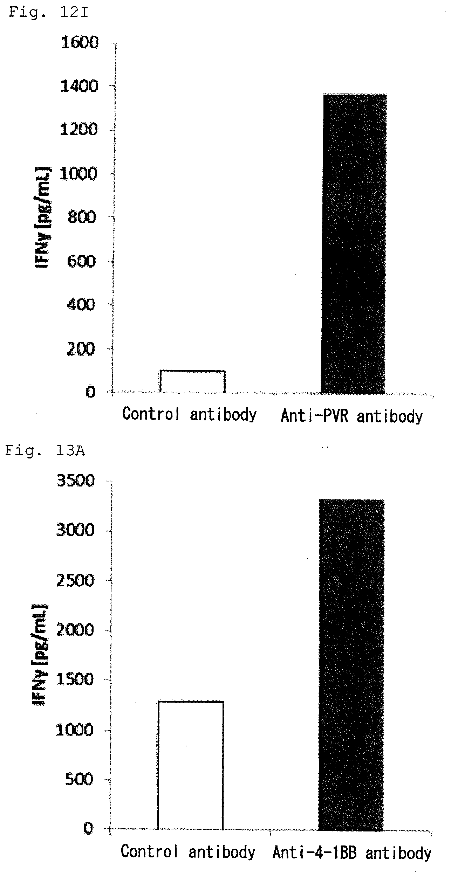

Fig. 12I shows the IFN-γ production by the treatment with an immune checkpoint inhibitor. WT1 antigen peptide-specific T cells induced in a vaccinated mouse were treated with an anti-PVR or an isotype control antibody, and the IFN-γ production from the cells when cocultured with a WT1 killer peptide and LLC-HHD-WT1 tumor cells was measured by ELISA.
Fig. 13A shows the IFN-γ production by the treatment with a costimulatory molecule agonist antibody. WT1 antigen peptide-specific T cells induced in a vaccinated mouse were treated with an anti-4-1BB or an isotype control antibody, and the IFN-γ production from the cells when cocultured with a WT1 killer peptide and LLC-HHD-NT1 tumor cells was measured by ELISA.

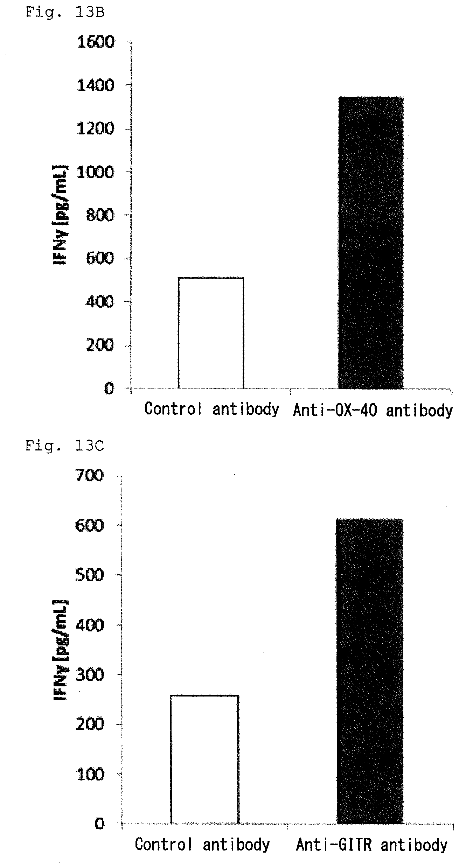

Fig. 13B shows the IFN-γ production by the treatment with a costimulatory molecule agonist antibody. WT1 antigen peptide-specific T cells induced in a vaccinated mouse were treated with an anti-OX-40 or an isotype control antibody, and the IFN-γ production from the cells when cocultured with a WT1 killer peptide and LLC-HHD-WT1 tumor cells was measured by ELISA.
Fig. 13C shows the IFN-γ production by the treatment with a costimulatory molecule agonist antibody. WT1 antigen peptide-specific T cells induced in a vaccinated mouse were treated with an anti-GITR or an isotype control antibody, and the IFN-γ production from the cells when cocultured with a WT1 killer peptide and LLC-HHD-WT1 tumor cells was measured by ELISA.

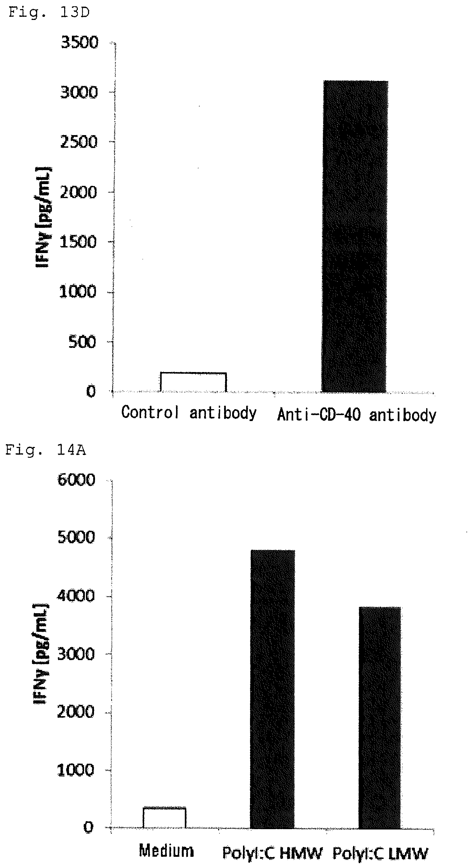

Fig. 13D shows the IFN-γ production by the treatment with a costimulatory molecule agonist antibody. WT1 antigen peptide-specific T cells induced in a vaccinated mouse were treated with an anti-CD40 or an isotype control antibody, and the IFN-γ production from the cells when cocultured with a WT1 killer peptide and LLC-HHD-WT1 tumor cells was measured by ELISA.
Fig. 14A shows the IFN-γ production by the treatment with a Toll-like receptor (TLR) agonist. WT1 antigen peptide-specific T cells induced in a vaccinated mouse were treated with PolyI:C, and the IFN-γ production from the cells when cocultured with a WT1 killer peptide and LLC-HHD-WT1 tumor cells was measured by ELISA.

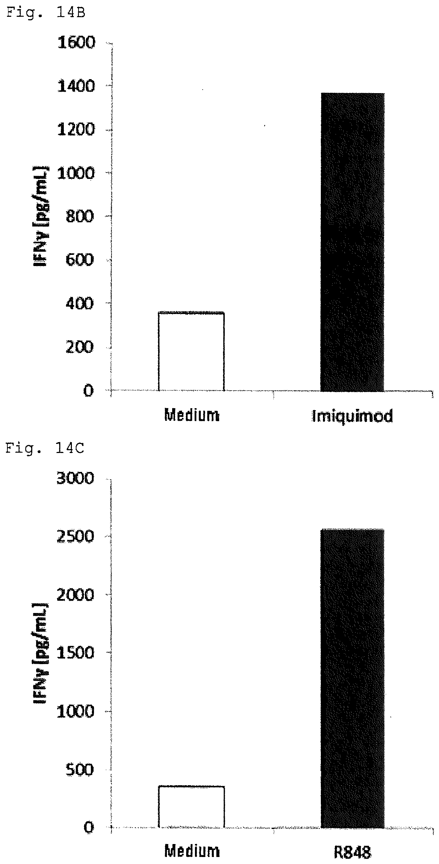

Fig. 14B shows the IFN-γ production by the treatment with a TLR agonist. WT1 antigen peptide-specific T cells induced in a vaccinated mouse were treated with Imiquimod, and the IFN-γ production from the cells when cocultured with a WT1 killer peptide and LLC-HHD-WT1 tumor cells was measured by ELISA.
Fig. 14C shows the IFN-γ production by the treatment with a TLR agonist. WT1 antigen peptide-specific T cells induced in a vaccinated mouse were treated with R848, and the IFN-γ production from the cells when cocultured with a WT1 killer peptide and LLC-HHD-WT1 tumor cells was measured by ELISA.

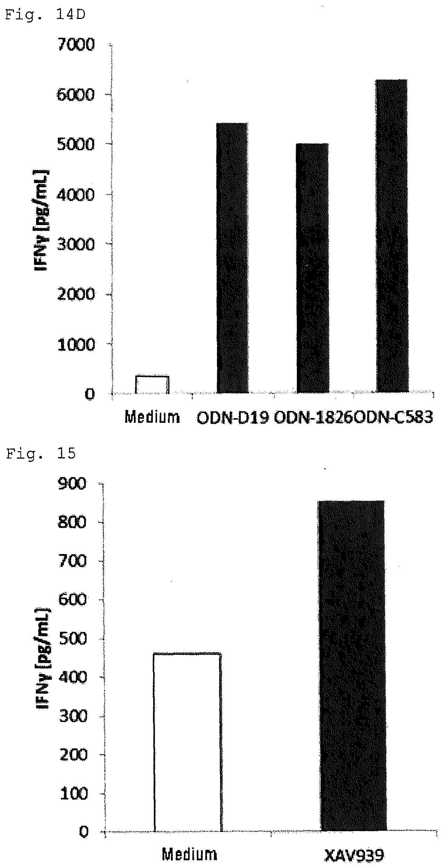

Fig. 14D shows the IFN-γ production by the treatment with a TLR agonist. WT1 antigen peptide-specific T cells induced in a vaccinated mouse were treated with CpG-ODN, and the IFN-γ production from the cells when cocultured with a WT1 killer peptide and LLC-HHD-WT1 tumor cells was measured by ELISA.
Fig. 15 shows the IFN-γ production by the treatment with a β-catenin inhibitor. WT1 antigen peptide-specific T cells induced in a vaccinated mouse were treated with XAV939, and the IFN-γ production from the cells when cocultured with a WT1 killer peptide and I,LC-HHD-WT1 tumor cells was measured by ELISA.

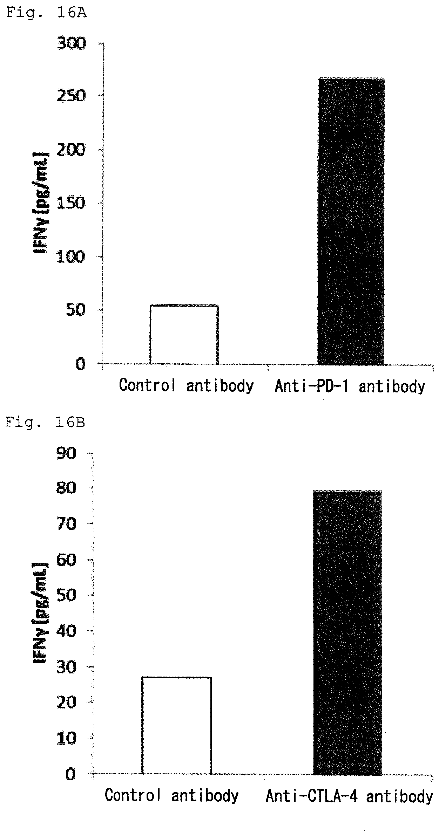

Fig. 16A shows the IFN-γ production by the treatment with an immune checkpoint inhibitor. WT1 antigen peptide-specific T cells induced in a vaccinated tumor-bearing mouse were treated with an anti-PD-1 or an isotype control antibody and followed by culture, and the IFN-γ production from the cells was measured by ELISA.
Fig. 16B shows the IFN-γ production by the treatment with an immune checkpoint inhibitor. WT1 antigen peptide-specific T cells induced in a vaccinated tumor-bearing mouse were treated with an anti-CTLA-4 or an isotype control antibody and followed by culture, and the IFN-γ production from the cells was measured by ELISA.

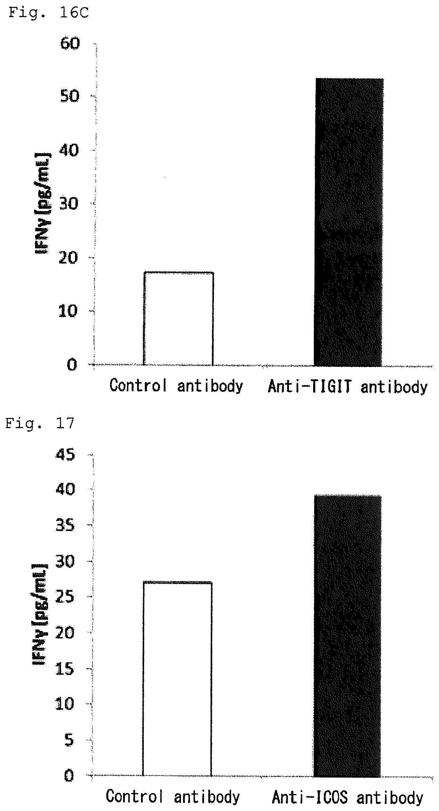

Fig. 16C shows the IFN-γ production by the treatment with an immune checkpoint inhibitor. WT1 antigen peptide-specific T cells induced in a vaccinated tumor-bearing mouse were treated with an anti-TIGIT or an isotype control antibody and followed by culture, and the IFN-γ production from the cells was measured by ELISA.
Fig. 17 shows the IFN-γ production by the treatment with a costimulatory molecule agonist antibody. WT1 antigen peptide-specific T cells induced in a vaccinated tumor-bearing mouse were treated with an anti-ICOS or an isotype control antibody and followed by culture, and the IFN-γ production from the cells was measured by ELISA.

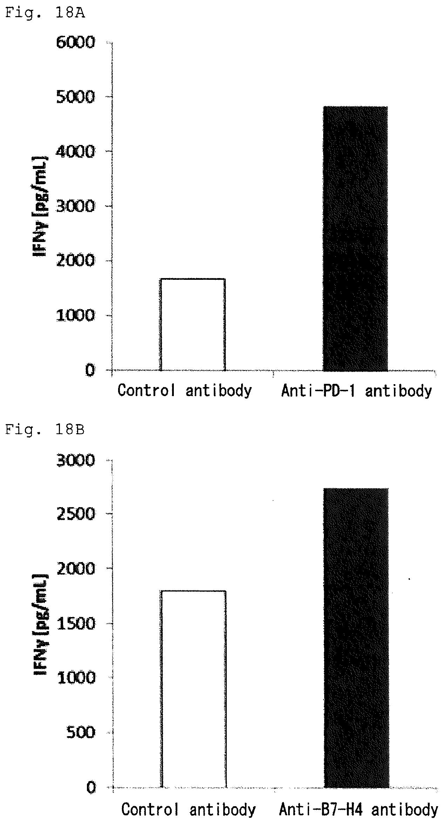

Fig. 18A shows the IFN-γ production by the treatment with an immune checkpoint inhibitor. WT1 antigen peptide-specific T cells induced in a vaccinated mouse were treated with an anti-PD-1 or an isotype control antibody, and the IFN-γ production from the cells when cocultured with a WT1 killer peptide and LLC-HHD-WT1 tumor cells was measured by ELISA.
Fig. 18B shows the IFN-γ production by the treatment with an immune checkpoint inhibitor. WT1 antigen peptide-specific T cells induced in a vaccinated mouse were treated with an anti-B7-H4 or an isotype control antibody, and the IFN-γ production from the cells when cocultured with a WT1 killer peptide and LLC-HHD-WT1 tumor cells was measured by ELISA.

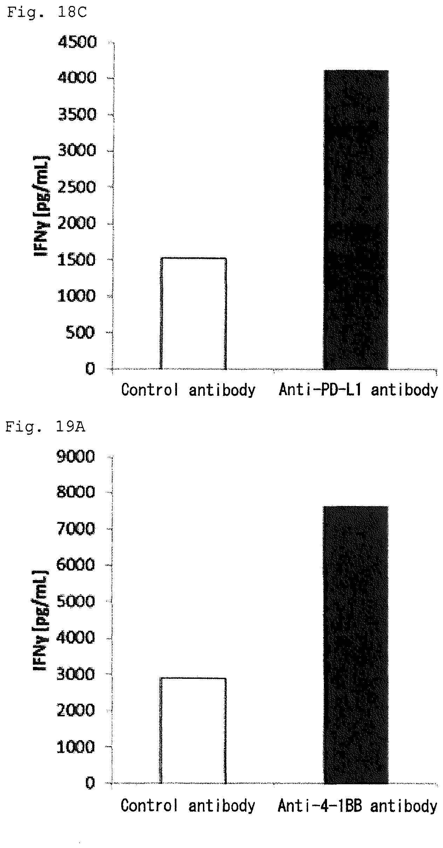

Fig. 18C shows the IFN-γ production by the treatment with an immune checkpoint inhibitor. WT1 antigen peptide-specific T cells induced in a vaccinated mouse were treated with an anti-PD-L1 or an isotype control antibody, and the IFN-γ production from the cells when cocultured with a WT1 killer peptide and LLC-HHD-WT1 tumor cells was measured by ELISA.
Fig. 19A shows the IFN-γ production by the treatment with a costimulatory molecule agonist antibody. WT1 antigen peptide-specific T cells induced in a vaccinated mouse were treated with an anti-4-1BB or an isotype control antibody, and the IFN-γ production from the cells when cocultured with a WT1 killer peptide and LLC-HHD-WT1 tumor cells was measured by ELISA.

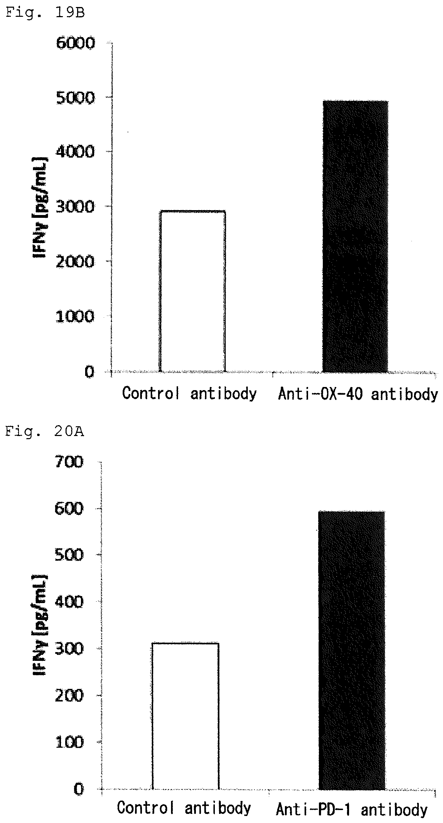

Fig. 19B shows the IFN-γ production by the treatment with a costimulatory molecule agonist antibody. WT1 antigen peptide-specific T cells induced in a vaccinated mouse were treated with an anti-OX-40 or an isotype control antibody, and the IFN-γ production from the cells when cocultured with a WT1 killer peptide and LLC-HHD-NT1 tumor cells was measured by ELISA.
Fig. 20A shows the IFN-γ production by the treatment with an immune checkpoint inhibitor. WT1 antigen peptide-specific T cells induced in a vaccinated mouse were treated with an anti-PD-1 or an isotype control antibody, and the IFN-γ production from the cells when cocultured with a WT1 killer peptide and LLC-HHD-WT1 tumor cells was measured by ELISA.

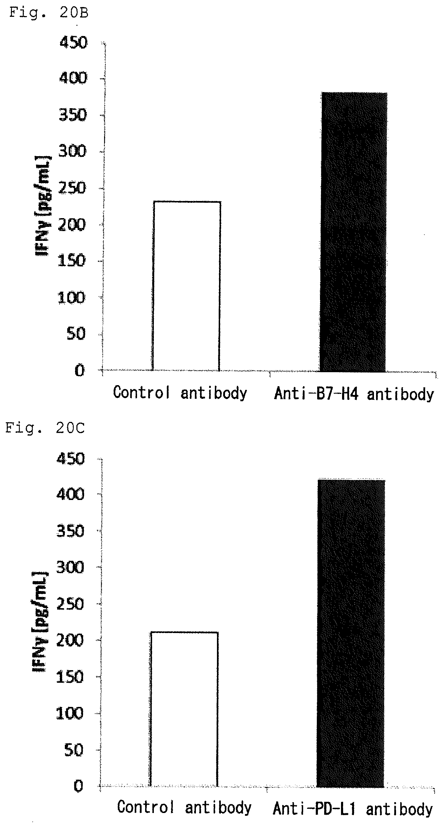

Fig. 20B shows the IFN-γ production by the treatment with an immune checkpoint inhibitor. WT1 antigen peptide-specific T cells induced in a vaccinated mouse were treated with an anti-B7-H4 or an isotype control antibody, and the IFN-γ production from the cells when cocultured with a WT1 killer peptide and LLC-HHD-WT1 tumor cells was measured by ELISA.
Fig. 20C shows the IFN-γ production by the treatment with an immune checkpoint inhibitor. WT1 antigen peptide-specific T cells induced in a vaccinated mouse were treated with an anti-PD-L1 or an isotype control antibody, and the IFN-γ production from the cells when cocultured with a WT1 killer peptide and LLC-HHD-WT1 tumor cells was measured by ELISA.

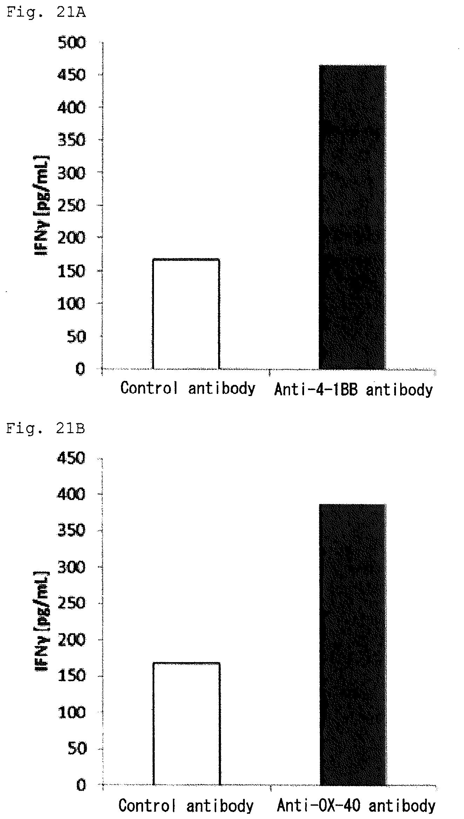

Fig. 21A shows the IFN-γ production by the treatment with a costimulatory molecule agonist antibody. WT1 antigen peptide-specific T cells induced in a vaccinated mouse were treated with an anti-4-1BB or an isotype control antibody, and the IFN-γ production from the cells when cocultured with a WT1 killer peptide and LLC-HHD-WT1 tumor cells was measured by ELISA.
Fig. 21B shows the IFN-γ production by the treatment with a costimulatory molecule agonist antibody. WT1 antigen peptide-specific T cells induced in a vaccinated mouse were treated with an anti-OX-40 or an isotype control antibody, and the IFN-γ production from the cells when cocultured with a WT1 killer peptide and LLC-HHD-WT1 tumor cells was measured by ELISA.

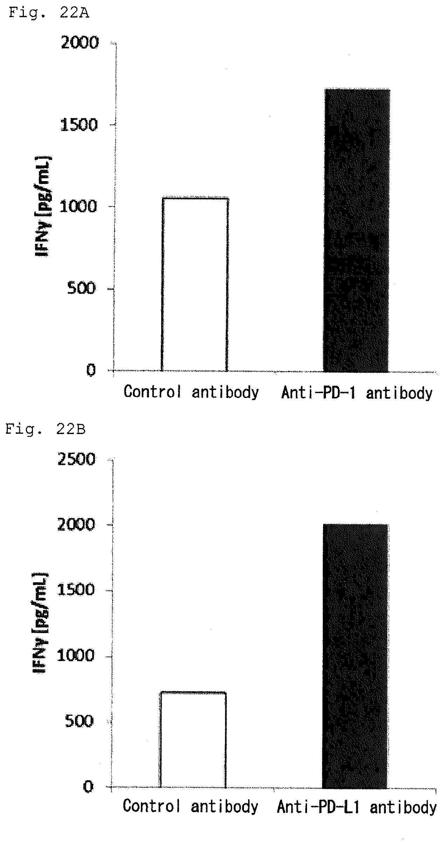

Fig. 22A shows the IFN-γ production by the treatment with an immune checkpoint inhibitor. WT1 antigen peptide-specific T cells induced in a vaccinated mouse were treated with an anti-PD-1 or an isotype control antibody, and the IFN-γ production from the cells when cocultured with a WT1 killer peptide and LLC-HHD-WT1 tumor cells was measured by ELISA.
Fig. 22B shows the IFN-γ production by the treatment with an immune checkpoint inhibitor. WT1 antigen peptide-specific T cells induced in a vaccinated mouse were treated with an anti-PD-L1 or an isotype control antibody, and the IFN-γ production from the cells when cocultured with a WT1 killer peptide and LLC-HHD-WT1 tumor cells was measured by ELISA.

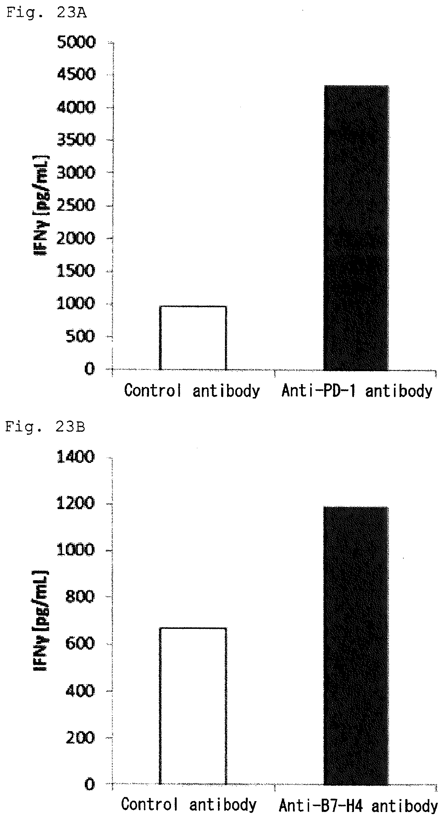

Fig. 23A shows the IFN-γ production by the treatment with an immune checkpoint inhibitor. WT1 antigen peptide-specific T cells induced in a vaccinated mouse were treated with an anti-PD-1 or an isotype control antibody, and the IFN-γ production from the cells when cocultured with a WT1 killer peptide and LLC-HHD-WT1 tumor cells was measured by ELISA.
Fig. 23B shows the IFN-γ production by the treatment with an immune checkpoint inhibitor. WT1 antigen peptide-specific T cells induced in a vaccinated mouse were treated with an anti-B7-H4 or an isotype control antibody, and the IFN-γ production from the cells when cocultured with a WT1 killer peptide and LLC-HHD-WT1 tumor cells was measured by ELISA.

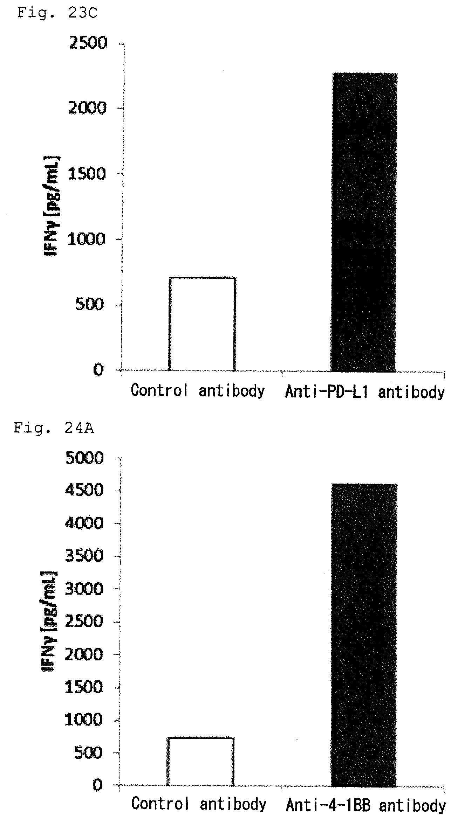

Fig. 23C shows the IFN-γ production by the treatment with an immune checkpoint inhibitor. WT1 antigen peptide-specific T cells induced in a vaccinated mouse were treated with an anti-PD-L1 or an isotype control antibody, and the IFN-γ production from the cells when cocultured with a WT1 killer peptide and LLC-HHD-WT1 tumor cells was measured by ELISA.
Fig. 24A shows the IFN-γ production by the treatment with a costimulatory molecule agonist antibody. WT1 antigen peptide-specific T cells induced in a vaccinated mouse were treated with an anti-4-1BB or an isotype control antibody, and the IFN-γ production from the cells when cocultured with a WT1 killer peptide and LLC-HHD-NT1 tumor cells was measured by ELISA.

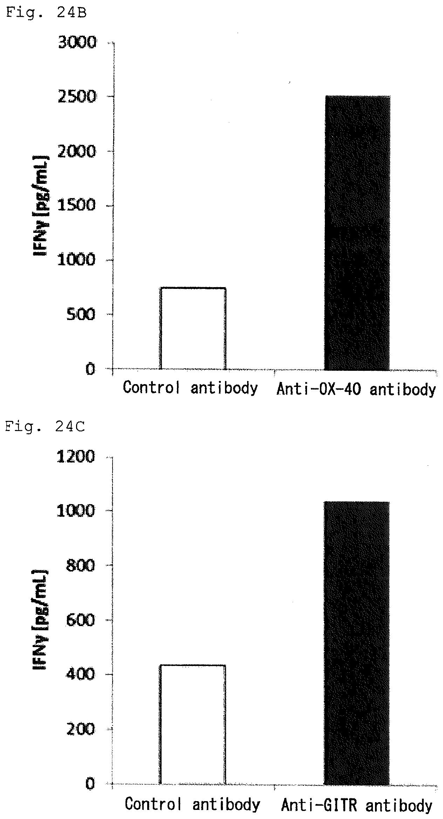

Fig. 24B shows the IFN-γ production by the treatment with a costimulatory molecule agonist antibody. WT1 antigen peptide-specific T cells induced in a vaccinated mouse were treated with an anti-OX-40 or an isotype control antibody, and the IFN-γ production from the cells when cocultured with a WT1 killer peptide and LLC-HHD-WT1 tumor cells was measured by ELISA.
Fig. 24C shows the IFN-γ production by the treatment with a costimulatory molecule agonist antibody. WT1 antigen peptide-specific T cells induced in a vaccinated mouse were treated with an anti-GITR or an isotype control antibody, and the IFN-γ production from the cells when cocultured with a WT1 killer peptide and LLC-HHD-WT1 tumor cells was measured by ELISA.

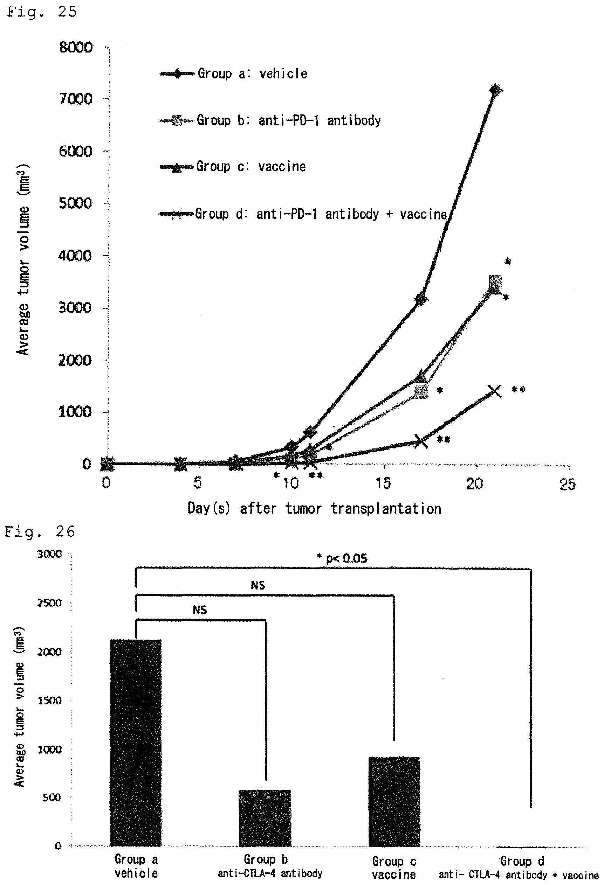

Fig. 25 shows the suppression of tumor proliferation *in* vivo by a combination of an anti-PD-1 antibody and a vaccine. Shown is the average tumor volume in EL4-A24/Kb-WT1 tumor cells-transplanted mice that received a vehicle (Group a), an anti-PD-1 antibody (Group b), a vaccine (Group c), or an anti-PD-1 antibody and a vaccine (Group d).
Fig. 26 shows the suppression of tumor proliferation *in vivo* by a combination of an anti-CTLA-4 antibody and a vaccine. Shown is the average tumor volume in EI,4-A24lKb-WT1 tumor cells-transplanted mice that received a vehicle (Group a), an anti-CTLA-4 antibody (Group b), a vaccine (Group c), or an anti-CTLA-4 antibody and a vaccine (Group d).

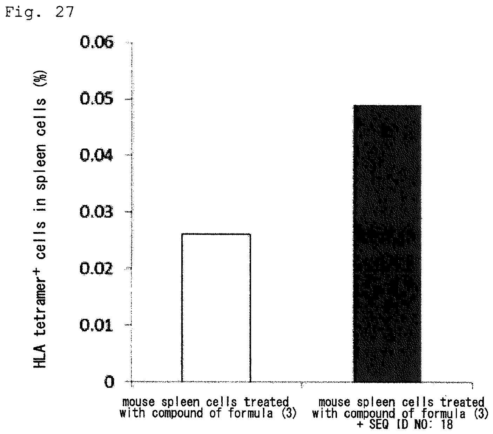

Fig. 27 shows the detection of WT1 antigen peptide-specific CTLs by an HLA tetramer. The vertical axis shows the ratio of WT1 antigen peptide-specific CTLs included in spleen cells from a vaccinated mouse.

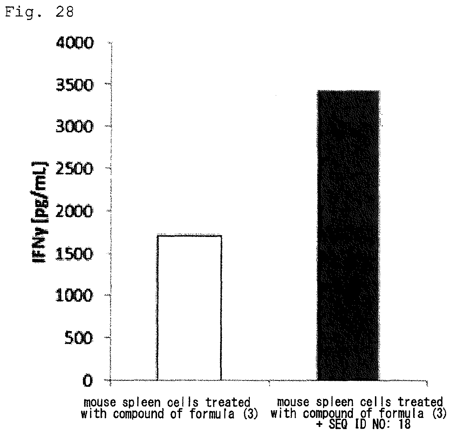

Fig. 28 shows the IFN-γ production by the stimulation with a WT1 antigen peptide. WTl antigen peptide-specific T cells induced in a vaccinated mouse were cultured in the presence of a WT1 killer peptide, and the IFN-γ production from the cells was measured by ELISA.

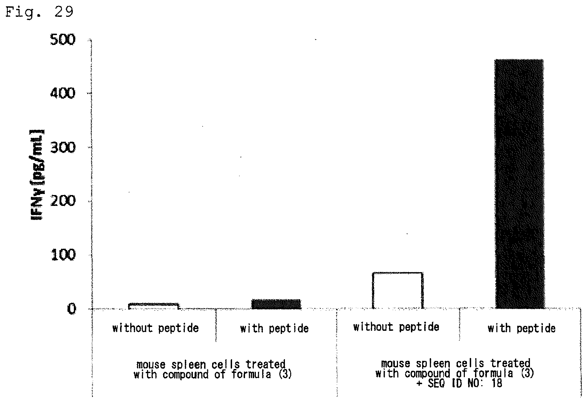

Fig. 29 shows the IFN-γ production by the stimulation with a WT1 antigen peptide. WT1 antigen peptide-specific T cells induced in a vaccinated mouse were cultured with LLC-HHD-WT1 tumor cells in the presence or absence of a WT1 killer peptide, and the IFN-γ production from the cells was measured by ELISA.

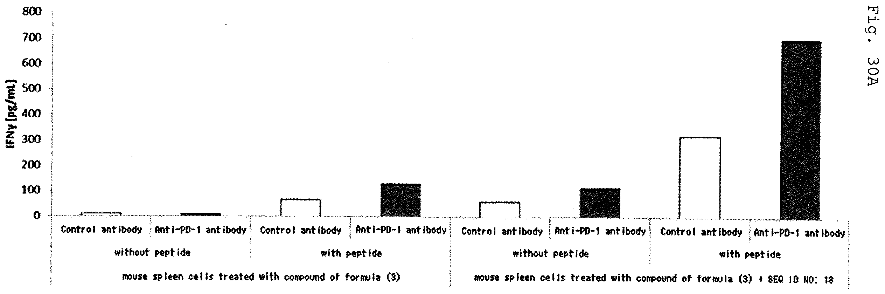

Fig. 30A shows the IFN-γ production by the treatment with an immune checkpoint inhibitor. WT1 antigen peptide-specific T cells induced in a vaccinated mouse were treated with an anti-PD-1 or an isotype control antibody, and the IFN-γ production from the cells when cocultured with LLC-HHD-WT1 tumor cells in the presence or absence of a WT1 killer peptide was measured by ELISA.

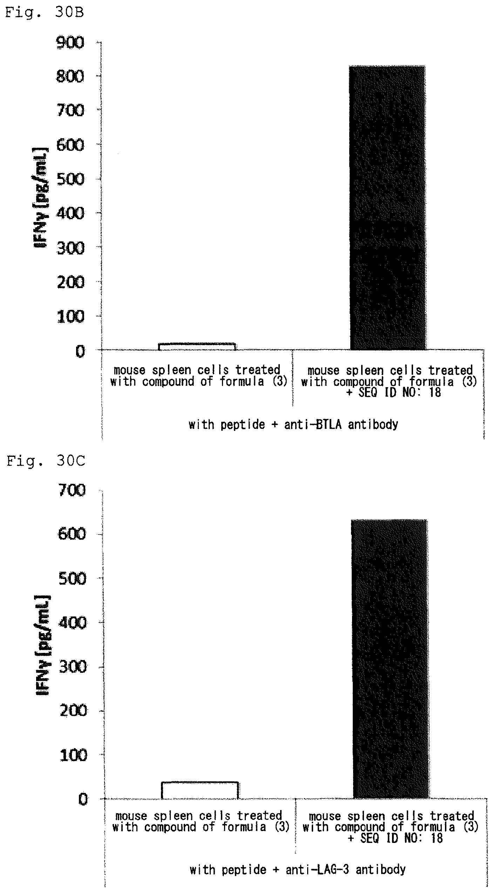

Fig. 30B shows the IFN-γ production by the treatment with an immune checkpoint inhibitor. WT1 antigen peptide-specific T cells induced in a vaccinated mouse were treated with an anti-BTLA antibody, and the IFN-γ production from the cells when cocultured with LLC-HHD-WT1 tumor cells in the presence of a WT1 killer peptide was measured by ELISA.
Fig. 30C shows the IFN-γ production by the treatment with an immune checkpoint inhibitor. WT1 antigen peptide-specific T cells induced in a vaccinated mouse were treated with an anti-LAG-3 antibody, and the IFN-γ production from the cells when cocultured with LLC-HHD-WT1 tumor cells in the presence of a WT1 killer peptide was measured by ELISA.

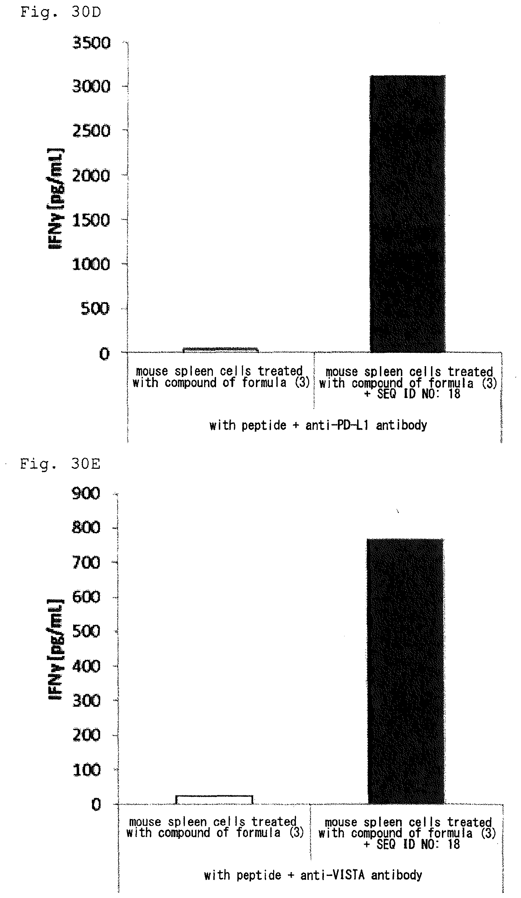

Fig. 30D shows the IFN-γ production by the treatment with an immune checkpoint inhibitor. WT1 antigen peptide-specific T cells induced in a vaccinated mouse were treated with an anti-PD-L1 antibody, and the IFN-γ production from the cells when cocultured with LLC-HHD-WT1 tumor cells in the presence of a WT1 killer peptide was measured by ELISA.
Fig. 30E shows the IFN-γ production by the treatment with an immune checkpoint inhibitor. WT1 antigen peptide-specific T cells induced in a vaccinated mouse were treated with an anti-VISTA antibody, and the IFN-γ production from the cells when cocultured with LLC-HHD-WT1 tumor cells in the presence of a WT1 killer peptide was measured by ELISA.

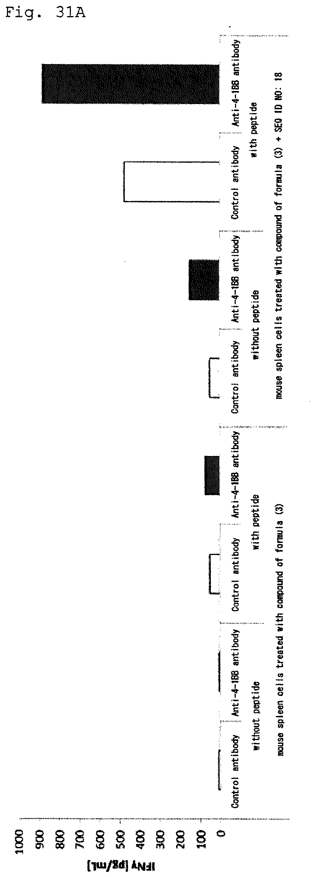

Fig. 31A shows the IFN-γ production by the treatment with a costimulatory molecule agonist antibody. WT1 antigen peptide-specific T cells induced in a vaccinated mouse were treated with an anti-4-1BB or an isotype control antibody, and the IFN-γ production from the cells when cocultured with LLC-HHD-WT1 tumor cells in the presence or absence of a WT1 killer peptide was measured by ELISA.

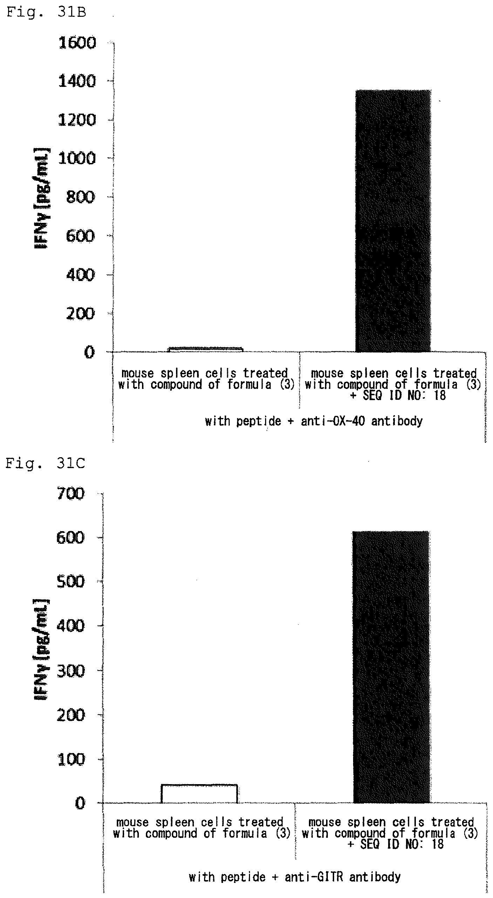

Fig. 31B shows the IFN-γ production by the treatment with a costimulatory molecule agonist antibody. WT1 antigen peptide-specific T cells induced in a vaccinated mouse were treated with an anti-OX-40 antibody, and the IFN-γ production from the cells when cocultured with LLC-HHD-WT1 tumor cells in the presence of a WT1 killer peptide was measured by ELISA.
Fig. 31C shows the IFN-γ production by the treatment with a costimulatory molecule agonist antibody. WT1 antigen peptide-specific T cells induced in a vaccinated mouse were treated with an anti-GITR antibody, and the IFN-γ production from the cells when cocultured with LLC-HHD-WT1 tumor cells in the presence of a WT1 killer peptide was measured by ELISA.

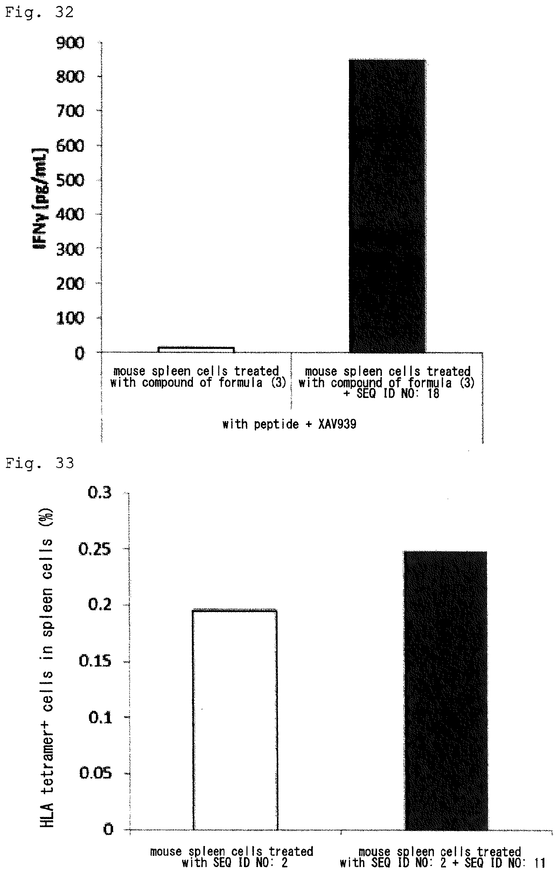

Fig. 32 shows the IFN-γ production by the treatment with a β-catenin inhibitor. WT1 antigen peptide-specific T cells induced in a vaccinated mouse were treated with XAV939, and the IFN-γ production from the cells when cocultured with LLC-HHD-WT1 tumor cells in the presence of a WT1 killer peptide was measured by ELISA.
Fig. 33 shows the detection of WT1 antigen peptide-specific CTLs by an HLA tetramer. The vertical axis shows the ratio of WT1 antigen peptide-specific CTLs included in spleen cells from a vaccinated mouse.

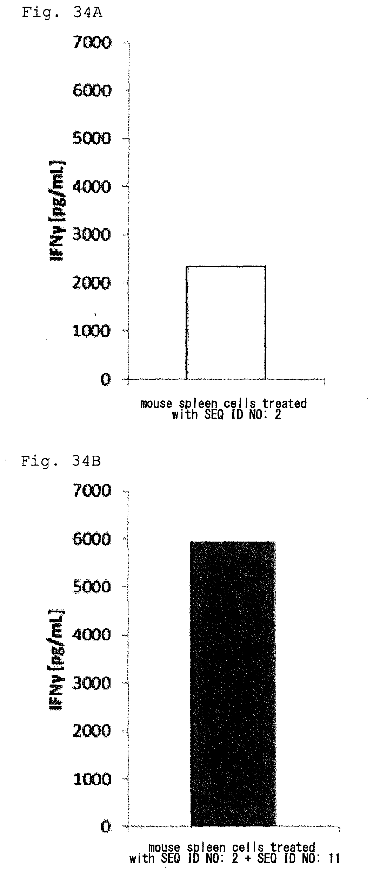

Fig. 34A shows the IFN-γ production by the stimulation with a WT1 antigen peptide. WT1 antigen peptide-specific T cells induced in a mouse vaccinated with a killer vaccine were cultured in the presence of a WT1 killer peptide, and the IFN-γ production from the cells was measured by ELISA.
Fig. 34B shows the IFN-γ production by the stimulation with a WT1 antigen peptide. WT1 antigen peptide-specific T cells induced in a mouse vaccinated with a cocktail vaccine were cultured in the presence of a WT1 killer peptide, and the IFN-γ production from the cells was measured by ELISA.

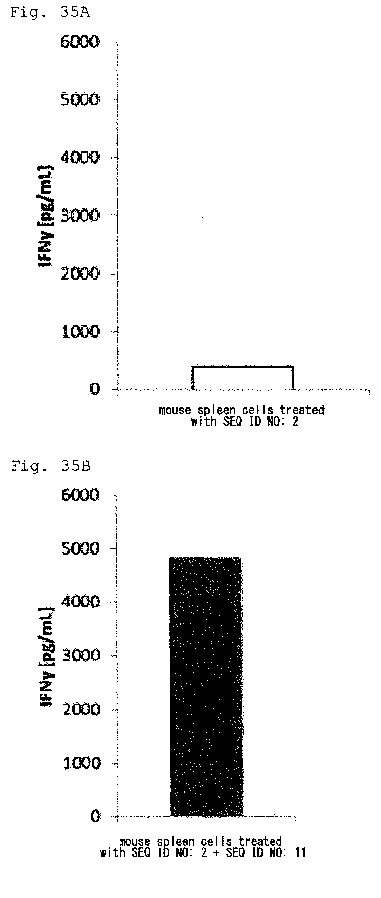

Fig. 35A shows the IFN-γ production by the treatment with an anti-PD-1 antibody. WT1 antigen peptide-specific T cells induced in a mouse vaccinated with a killer vaccine were treated with an anti-PD-1 antibody, and the IFN-γ production from the cells when cocultured with LLC-HHD-WT1 tumor cells in the presence of a WT1 killer peptide was measured by ELISA.
Fig. 35B shows the IFN-γ production by the treatment with an anti-PD-1 antibody. WT1 antigen peptide-specific T cells induced in a mouse vaccinated with a cocktail vaccine were treated with an anti-PD-1 antibody, and the IFN-γ production from the cells when cocultured with LLC-HHD-WT1 tumor cells in the presence of a WT1 killer peptide was measured by ELISA.

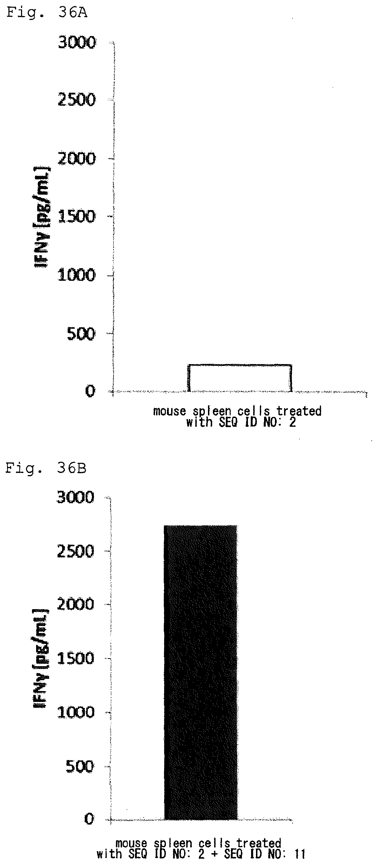

Fig. 36A shows the IFN-γ production by the treatment with an anti-B7-H4 antibody. WT1 antigen peptide-specific T cells induced in a mouse vaccinated with a killer vaccine were treated with an anti-B7-H4 antibody, and the IFN-γ production from the cells when cocultured with LLC-HHD-WT1 tumor cells in the presence of a WT1 killer peptide was measured by ELISA.
Fig. 36B shows the IFN-γ production by the treatment with an anti-B7-H4 antibody. WT1 antigen peptide-specific T cells induced in a mouse vaccinated with a cocktail vaccine were treated with an anti-B7-H4 antibody, and the IFN-γ production from the cells when cocultured with LLC-HHD-WT1 tumor cells in the presence of a WT1 killer peptide was measured by ELISA.

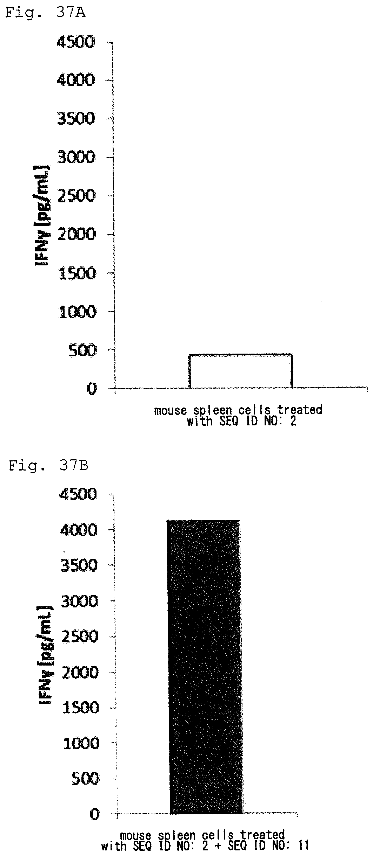

Fig. 37A shows the IFN-γ production by the treatment with an anti-PD-L1 antibody. WT1 antigen peptide-specific T cells induced in a mouse vaccinated with a killer vaccine were treated with an anti-PD-L1 antibody, and the IFN-γ production from the cells when cocultured with LLC-HHD-WT1 tumor cells in the presence of a WT1 killer peptide was measured by ELISA.
Fig. 37B shows the IFN-γ production by the treatment with an anti-PD-L1 antibody. WT1 antigen peptide-specific T cells induced in a mouse vaccinated with a cocktail vaccine were treated with an anti-PD-L1 antibody, and the IFN-γ production from the cells when cocultured with LLC-HHD-WT1 tumor cells in the presence of a WT1 killer peptide was measured by ELISA.

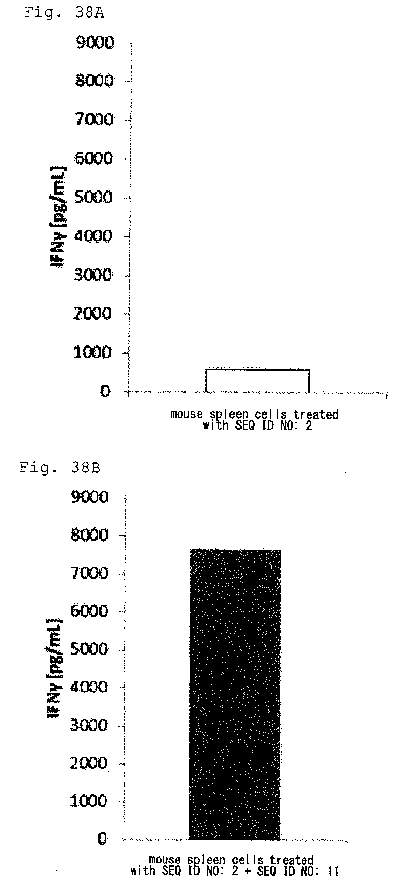

Fig. 38A shows the IFN-γ production by the treatment with an anti-4-1BB antibody. WT1 antigen peptide-specific T cells induced in a mouse vaccinated with a killer vaccine were treated with an anti-4-lBB antibody, and the IFN-γ production from the cells when cocultured with LLC-HHD-WT1 tumor cells in the presence of a WT1 killer peptide was measured by ELISA.
Fig. 38B shows the IFN-γ production by the treatment with an anti-4-1BB antibody. WT1 antigen peptide-specific T cells induced in a mouse vaccinated with a cocktail vaccine were treated with an anti-4-1BB antibody, and the IFN-γ production from the cells when cocultured with LLC-HHD-WT1 tumor cells in the presence of a WT1 killer peptide was measured by ELISA.

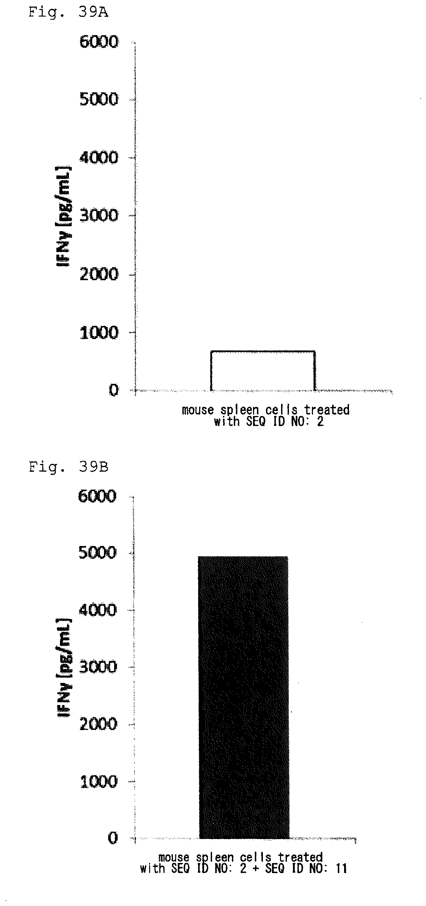

Fig. 39A shows the IFN-γ production by the treatment with an anti-OX-40 antibody. WT1 antigen peptide-specific T cells induced in a mouse vaccinated with a killer vaccine were treated with an anti-OX-40 antibody, and the IFN-γ production from the cells when cocultured with LI,C-HHD-WT1 tumor cells in the presence of a WT1 killer peptide was measured by ELISA.
Fig. 39B shows the IFN-γ production by the treatment with an anti-ox-40 antibody. WTl antigen peptide-specific T cells induced in a mouse vaccinated with a cocktail vaccine were treated with an anti-OX-40 antibody, and the IFN-γ production from the cells when cocultured with LLC-HHD-WT1 tumor cells in the presence of a WT1 killer peptide was measured by ELISA.

### DESCRIPTION OF EMBODIMENTS

Embodiments of the present invention are explained in detail hereinafter.

The present invention relates to a combination use of a WTl antigen peptide and an immunomodulator and further a WT1 helper peptide.

As used herein, a "WT1 antigen peptide" refers to a peptide that has an amino acid sequence derived from WT1 protein, and binds to an MHC class I or class II molecule to be presented on the cell surface as a complex with the MHC molecule and induces killer T cells or helper T cells. As used herein, a WT1 antigen peptide that binds to an MHC class I molecule to induce killer T cells is referred as a WT1 killer peptide, and a WT1 antigen peptide that binds to an MHC class II molecule to induce helper T cells is referred to as a "WT1 helper peptide". WT1 protein may be, but not limited to, a mouse or human WT1 protein, and preferably is a human WT1 protein. The human WT1 protein has the amino acid sequence of SEQ ID NO: 1. As used herein, the term "WT1 antigen peptide" include a pharmaceutically acceptable salt thereof as long as it is appropriate in the context.

The WT1 antigen peptide may be modified at a part or all of the amino acid residues in its amino acid sequence. Such modified peptides may be prepared by any method known in the art. For example, the modified peptide may be prepared by modification such as esterification, alkylation, halogenation, phosphorylation, sulfonation, or amidation of the functional group of the side chain of the amino acid residue(s) constituting the peptide. Also, a variety of substances may be bound to the peptide at the N- and/ or C-terminus. For example, an amino acid, a peptide, or an analog thereof may be bound to the peptide. When such a substance is bound to the WT1 antigen peptide, the substance may be removed by any process, for example by an enzymatic reaction *in vivo* or by intracellular processing, such that the WT1 antigen peptide is finally generated. The substance may be a substance that regulates the solubility of the peptide; improves the stability of the peptide such as protease resistance; delivers the peptide to a specific tissue or organ; or increases an uptake of the peptide by antigen presenting cells. The substance may be a substance that increases the CTL inducing activity, such as a killer or helper peptide different from the WT1 antigen peptide or a pharmaceutically acceptable salt thereof.

The WT1 antigen peptide may comprise a bond other than a peptide bond such as a carbon-carbon bond, a carbonnitrogen bond, or a carbon-sulfur bond. Also, the WT1 antigen peptide may comprise one or more D-amino acids.

The modified peptides as described above are illustrative only and those skilled in the art can easily conceive, prepare, examine and use other variations of the peptides.

The "amino acid residue" as used herein refers to a single unit in the amino acids constituting a peptide or protein molecule. The "amino acid residue" may be a natural or non-natural α-amino acid residue, β-amino acid residue, γ-amino acid residue or δ-amino acid residue. Specifically, The "amino acid residue" may be a natural α-amino acid residue, ornithine residue, homoserine residue, homocysteine residue, β-alanine, γ-aminobutanoic acid or 8-aminopentanoic acid.

The "amino acid residue" as used herein may be represented by the following abbreviations.
Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
Ile or I: isoleucine residue
Leu or L: leucine residue
Lys or K: lysine residue
Met or M: methionine residue
Phe or F: phenylalanine residue
Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (also referred to as α-aminobutyric acid residue)
Orn: ornithine residue
Cit: citrulline residue

The amino acid sequence of a peptide disclosed herein is described according to the conventional method, wherein the amino acid residue of the N-terminal amino acid is positioned on the left side, and the amino acid residue of the C-terminal amino acid is positioned on the right side. In a peptide, unless otherwise indicated, the amino group of the N-terminal amino acid residue binds to a hydrogen atom, and the carbonyl group of the C-terminal amino acid residue binds to a hydroxyl group. A divalent peptide group means a group that binds at the amino group of the N-terminal amino acid residue and the carbonyl group of the C-terminal amino acid residue. For example, in the peptide comprised in the compounds of formulae (1) to (3), the amino group of the N-terminal amino acid residue binds to a hydrogen atom, and the carbonyl group of the C-terminal amino acid residue binds to a hydroxyl group.

MHC in human is called human leukocyte-type antigen (HLA). HLA subtypes corresponding to MHC class I-molecules include HLA-A, B, Cw, F and G. The expression "MHC class I-restricted" as used herein means the property of inducing killer T cells by binding to an MHC class I molecule. Preferred examples of "MHC class I-restricted" peptides include HLA-A-restricted peptides, HLA-B-restricted peptides, and HLA-Cw-restricted peptides.

Allelic Polymorphism is known for each HLA subtype. The polymorphism in HLA-A has 27 or more types such as HLA-A1, HLA-A2, and HLA-A24. The polymorphism in HLA-B has 59 or more types such as HLA-B7, HLA-B40, and HLA-B44. The polymorphism in HLA-Cw has 10 or more types such as HLA-Cw0301, HLA-Cw0401, and HLA-Cw0602. Among such polymorphism, HLA-A0201 and HLA-A24 are preferred.

In one embodiment, the WT1 antigen peptide is a killer peptide, which binds to an MHC class I molecule and induces killer T cells (cytotoxic T cells, CTLs). The WT1 killer peptide induces WT1-specific killer T cells when presented on the cell surface in a form of a complex with an MHC class I molecule.

In one embodiment, the WT1 killer peptide is a peptide consisting of contiguous 7-30 amino acids in the amino acid sequence of human WT1 protein of SEQ ID NO: 1 or a variant thereof. The WT1 killer peptide may be a peptide comprising the amino acid sequence selected from
RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6),
RVPGVAPTL (SEQ ID NO: 7),
VLDFAPPGA (SEQ ID NO: 8),
C-CMTWNQMNL (SEQ ID NO: 9) (wherein the bond within C-C is a disulfide bond), and
C-CYTWNQMNL (SEQ ID NO: 10) (wherein the bond within C-C is a disulfide bond),
or a peptide comprising an altered amino acid sequence of the amino acid sequence selected from SEQ ID NOS: 2-10 that comprises amino acid alteration in the amino acid sequence and having the CTL inducing activity. Preferably, the WT1 killer peptide is a peptide consisting of the amino acid sequence selected from SEQ ID NOS: 2-10, or a peptide consisting of an altered amino acid sequence of the amino acid sequence selected from SEQ ID NOS: 2-10 that comprises amino acid alteration in the amino acid sequence and having the CTL inducing activity. More preferably, the WT1 killer peptide is a peptide consisting of the amino acid sequence selected from SEQ ID NOS: 2-10. Even more preferably, the WT1 killer peptide is a peptide consisting of the amino acid sequence selected from SEQ ID NOS: 2-6, 8 and 10.

As used herein, a peptide "comprising" a given amino acid sequence means, as usually understood, a peptide that may comprise a further amino acid(s) added to the N-terminal and/or C-terminal amino acid of the given amino acid sequence.

As used herein, the "peptide comprising an altered amino acid sequence of the amino acid sequence of ... that comprises amino acid alteration in the amino acid sequence and having the CTL inducing activity" is also called as an altered killer peptide. The "altered killer peptide" means a peptide that consists of an amino acid sequence wherein one to several, preferably one to three amino acids are deleted from, substituted in, and/or added to the original amino acid sequence, and binds to MHC class I to induce CTLs. The position of the amino acid to be substituted may be position 1 (N-terminal), position 2, position 3 or position 9 for a peptide consisting of 9 amino acid residues. The number of amino acids to be added (or inserted, since "addition" encompasses "insertion") is preferably 1 or 2, more preferably 1. A preferable position for addition is the C-terminal. The number of amino acids to be deleted is preferably 1. In the alteration, the amino acid to be added or substituted may be a non-natural amino acid other than the 20 genetically encoded amino acids.

The regularity in the amino acid sequence (binding motif) has been known in peptides that bind to an HLA antigen for each type of polymorphism in an HLA subtype. For example, to make the binding motif for HLA-A24, in a peptide consisting of 8 to 11 amino acid residues, the amino acid at position 2 is Tyr, Phe, Met or Trp, and the amino acid at the C-terminus is Phe, Leu, Ile, Trp or Met (J. Immunol., 152, p3913, 1994; J. Immunol., 155, p4307, 1994; Immunogenetics, 41, p178, 1995). Thus, in a peptide consisting of 9 amino acid residues, the amino acid at position 2 may be replaced with Tyr, Phe, Met or Trp and/or the amino acid at position 9 may be replaced with Phe, Leu, Ile, Trp or Met. A peptide containing such amino acid alteration is a preferred altered killer peptide. Similarly, to make the binding motif for HLA-A2, in a peptide consisting of 8 to 11 amino acid residues, the amino acid at position 2 is Leu or Met, and the amino acid at the C-terminus is Val or Leu. Thus, in a peptide consisting of 9 amino acid residues, the amino acid at position 2 may be replaced with Leu or Met and the amino acid at the C-terminus may be replaced with Val or Leu. A peptide containing such amino acid alteration is a preferred altered killer peptide

Examples of altered killer peptides include an altered killer peptide of RMFPNAPYL (SEQ ID NO: 2) such as
RYFPNAPYL (SEQ ID NO: 21) (WO03/106682)
FMFPNAPYL (SEQ ID NO: 22),
RLFPNAPYL (SEQ ID NO: 23),
RMMPNAPYL (SEQ ID NO: 24),
RMFPNAPYV (SEQ ID NO: 25) and
YMFPNAPYL (SEQ ID NO: 26) (WO2009/072610);
an altered killer peptide of CMTWNQMNL (SEQ ID NO: 3) such as
CYTWNQMNL (SEQ ID NO: 4) (WO02/79253),
Xaa-Met-Thr-Trp-Asn-Gln-Met-Asn-Leu (SEQ ID NO: 27), (wherein Xaa is Ser or Ala) and
Xaa-Tyr-Thr-Trp-Asn-Gln-Met-Asn-Leu (SEQ ID NO: 28) (wherein Xaa is Ser, Ala, Abu, Arg, Lys, Orn, Cit, Leu, Phe or Asn) (WO2004/026897);
an altered killer peptide of ALLPAVPSL (SEQ ID NO: 5) such as
AYLPAVPSL (SEQ ID NO: 29) (WO2003/106682);
an altered killer peptide of SLGEQQYSV (SEQ ID NO: 6) such as
FLGEQQYSV (SEQ ID NO: 30),
SMGEQQYSV (SEQ ID NO: 31) and
SLMEQQYSV (SEQ ID NO: 32) (WO2009/072610); and
an altered killer peptide of RVPGVAPTL (SEQ ID NO: 7) such as
RYPGVAPTL (SEQ ID NO: 33) (WO2003/106682) .

In one embodiment, the WT1 killer peptide is the compound of formula (1): 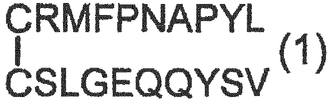 (wherein the bond within C-C is a disulfide bond), the compound of formula (2) : 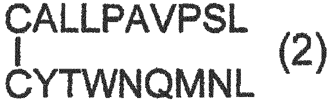 (wherein the bond within C-C is a disulfide bond), or the compound of formula (3): 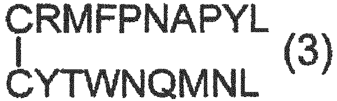 (wherein the bond within C-C is a disulfide bond).

In addition to the peptides and compounds as described above, examples of WT1 antigen peptides (killer or helper peptides) include the compounds disclosed in WO2014/157692.

In one embodiment, the WT1 antigen peptide is a helper peptide, which binds to an MHC class II molecule and induces helper T cells (CD4⁺ T cells). The WT1 helper peptide induces WT1-specific helper T cells when presented on the cell surface in a form of a complex with an MHC class II molecule. The WT1-specific helper T cells produce cytokines such as IL-2, IL-4, IL-5, IL-6 or interferons (IFNs) and promote proliferation, differentiation, or maturation of B cells or other subsets of T cells. Thus, the WT1 helper peptide activates helper T cells to induce or maintain CTLs or to activate effector cells such as macrophages, being useful to efficiently treat or prevent cancer.

HLA subtypes corresponding to the MHC class II-molecules include HLA-DR, DQ and DP. The expression "MHC class II-restricted" as used herein means the property of inducing helper cells by binding to an MHC class II molecule. Preferred examples of the "MHC class II-restricted" peptides include HLA-DR-restricted peptides, HLA-DQ-restricted peptides and HLA-DP-restricted peptides.

The WT1 helper peptide may be a peptide consisting of contiguous 7-30 amino acids, preferably 14-30 amino acids, in the amino acid sequence of human WT1 protein of SEQ ID NO: 1 or a variant thereof. The WT1 helper peptide may be a peptide comprising the amino acid sequence selected from
KRYFKLSHLQMHSRKH (SEQ ID NO: 11),
SGQARMFPNAPYLPSCLES (SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20),
SGQARMFPNAPYLPSC (SEQ ID NO: 34),
SGQAYMFPNAPYLPSC (SEQ ID NO: 35),
SGQARMFPNAPYLPSCLES (SEQ ID NO: 36),
SGQAYMFPNAPYLPSCLES (SEQ ID NO: 37),
PGCNKRYFKLSHLQMHSRK (SEQ ID NO: 38),
PGCNKRYFKLSHLQMHSRKH (SEQ ID NO: 39),
PGCNKRYFKLSHLQMHSRKHTG (SEQ ID NO: 40),
QARMFPNAPYLPSCL (SEQ ID NO: 44),
LKGVAAGSSSSVKWT (SEQ ID NO: 45) and
RYFKLSHLQMHSRKH (SEQ ID NO: 46),
or a peptide comprising an altered amino acid sequence of the amino acid sequence selected from SEQ ID NOS: 11-20, 34-40 and 44-46 that comprises amino acid alteration in the amino acid sequence and having the helper T cell inducing activity. Preferably, the WT1 helper peptide is a peptide consisting of the amino acid sequence selected from SEQ ID NOS: 11-20 and 34-40 or a peptide consisting of an altered amino acid sequence of the amino acid sequence selected from SEQ ID NOS: 11-20 and 34-40 that comprises amino acid alteration in the amino acid sequence, or a peptide consisting of an altered amino acid sequence of the amino acid sequence selected from SEQ ID NOS: 11-20 and 34-40 that comprises amino acid alteration in the amino acid sequence and having the helper T cell inducing activity. More preferably, the WT1 helper peptide is a peptide consisting of the amino acid sequence selected from SEQ ID NOS: 11-20 and 34-40. Even more preferably, the WT1 helper peptide is a peptide consisting of the amino acid sequence selected from SEQ ID NOS: 11-20.

As used herein, the "peptide comprising an altered amino acid sequence of the amino acid sequence of ... that comprises amino acid alteration in the amino acid sequence and having the helper T cell inducing activity" is also called as an altered helper peptide. The "altered helper peptide" means a peptide that consists of an amino acid sequence wherein one to several, preferably one to three amino acids are deleted from, substituted in, and/or added to the original amino acid sequence, and binds to MHC class II to induce CTLs. In the alteration, the amino acid to be added or substituted may be a non-natural amino acid other than the 20 genetically encoded amino acids.

Examples of altered helper peptides include an altered helper peptide of SGQARMFPNAPYLPSCLES (SEQ ID NO: 36) such as
SGQAYMFPNAPYLPSCLES (SEQ ID NO: 37) (WO2O07/120673),
SGQARMFPNAPYLPSC (SEQ ID NO: 34), and
SGQAYMFPNAPYLPSC (SEQ ID NO: 35); and
an altered helper peptide of PGCNKRYFKLSHLQMHSRKHTG (SEQ ID NO: 40) such as
PGCNKRYFKLSHLQMHSRK (SEQ ID NO: 38),
PGCNKRYFKLSHLQMHSRKH (SEQ ID NO: 39),
KRYFKLSHLQMHSRKH (SEQ ID NO: 11),
CNKRYFKLSHLQMHSRK (SEQ ID NO: 15),
CNKRYFKLSHLQMHSRKH (SEQ ID NO: 16) and
CNKRYFKLSHLQMHSRKHTG (SEQ ID NO: 17).

The WT1 antigen peptide may be prepared according to any method generally used in the peptide synthesis. Examples of such methods include those described in the references such as Peptide Synthesis, Interscience, New York, 1966; The Proteins, Vol 2, Academic Press Inc., New York, 1976; peptide synthesis, Maruzen Co., LTD., 1975; Basics and Experiment of Peptide Synthesis, Maruzen Co., LTD., 1985; and Development of Pharmaceutical Product subsequent vol. 14, Peptide Synthesis, Hirokawa Shoten, 1991. For example, the peptide may be prepared by the Fmoc method or the Boc method using a solid phase synthesizer, or by sequential condensation of Boc-amino acid or Z-amino acid in the liquid phase synthesis (wherein Fmoc is a 9-fluorenylmethoxycarbonyl group, Boc is a t-butoxycarbonyl group, and Z is a benzyloxycarbonyl group).

A functional group in an intermediate to prepare the WT1 antigen peptide such as an amino, carboxy, or mercapto group may be protected by a suitable protecting group and deprotected as necessary using protection and deprotection techniques. Preferred protecting groups, protection methods, and deprotection methods are described in detail, for example, in "Protective Groups in Organic Synthesis 2nd Edition (John Wiley & Sons, Inc.; 1990)". Examples of mercapto-protecting groups include an acetamidomethyl group and a trityl group.

When the WT1 antigen peptide has a disulfide bond, the disulfide bond may be formed between two different peptides each containing a cysteine residue, or between a peptide containing a cysteine residue and cysteine, according to any method generally used in peptide chemistry. Examples of methods to form a disulfide bond include those described in the references such as Peptide Synthesis, Interscience, New York, 1966; The Proteins, Vol. 2, Academic Press Inc., New York, 1976; peptide synthesis, Maruzen Co., LTD., 1975; Basics and Experiment of peptide synthesis, Maruzen Co., LTD., 1985; and Development of Pharmaceutical Product sequential vol. 14, Peptide Synthesis; Hirokawa Shoten, 1991.

Specifically, when the peptide contains one cysteine residue, a compound having a disulfide bond (also referred to as "a disulfide compound") may be prepared by removing all protecting groups including the mercapto-protecting group on the cysteine side chain and oxidizing the peptide in an inert solvent. In addition, a disulfide compound may be prepared by mixing two intermediates each having a mercapto group in a suitable solvent and oxidizing the mixture. The method for the oxidation may be selected as appropriate from known methods for disulfide bond formation in usual peptide synthesis. For example, the oxidation may be iodine oxidation; air oxidation under alkali conditions; or oxidation with an oxidant under alkaline or acidic conditions to form a disulfide bond. Examples of the oxidant include iodine, dimethyl sulfoxide (DMSO), and potassium ferricyanide. Examples of the solvent include water, acetic acid, methanol, chloroform, DMF, and DMSO, or a mixture thereof. The oxidation reaction often provides a mixture of symmetric and asymmetric disulfide compounds. The desired asymmetric disulfide compound may be obtained by purification using techniques such as various types of chromatography or recrystallization. Alternatively, a disulfide bond may be selectively formed by mixing an intermediate having an activated mercapto group and another intermediate having a mercapto group. Examples of the intermediate having an activated mercapto group include an intermediate comprising a mercapto group bonded with an Npys group (3-nitro-2-pyridinesulphenyl group). Alternatively, one intermediate is mixed with an agent to activate the mercapto group, for example, 2,2'-dithiobis(5-nitropyridine), and then the other intermediate is added thereto, whereby a disulfide bond may be selectively formed (Tetrahedron Letters. Vol. 37. No. 9, pp. 1347-1350).

The methods as described above also may be used when the peptide contains two or more cysteine residues. In this case, isomers having different disulfide bonding patterns are obtained. Using a specific combination of protecting groups for the side chains of cysteines provides a desired dimer having a disulfide bond between specific cysteine residues. Examples of the combination of protecting groups include MeBzl (methylbenzyl) group and Acm (acetamidomethyl) group, Trt (trityl) group and Acm group, Npys (3-nitro-2-pyridylthio) group and Acm group, and S-But (S-tert-butyl) group and Acm group. For example, when the combination of MeBzl group and Acm group is used, MeBzl groups and protecting groups other than those of cysteine side chains are removed and then a solution containing the peptide monomers is subjected to air oxidation reaction to form a disulfide bond between the deprotected cysteine residues. Then, after the deprotection with iodine and oxidation, a disulfide bond between the cysteine residues protected with Acm groups is formed.

The WT1 antigen peptide may be a compound composed of a killer peptide and a helper peptide or two different killer or helper peptides conjugated via a disulfide bond. Such peptides may be synthesized by a method comprising the following steps (1) to (3).

The step (1) uses Fmoc-C(Mmt)A-SBn and a first antigen peptide. The step (1) synthesizes a peptide wherein the carbonyl group of the C-terminal amino acid of C(Mmt)A binds to the N-terminal amino group of the first antigen peptide. The "Fmoc" is a 9-fluorenylmethoxycarbonyl group. The "Mmt" is a monomethoxytrityl group. The "SBn" is a thiobenzyl group.

The step (2) uses the peptide obtained in step (1) and a second antigen peptide that has a cysteine residue protected by Npsy group at the N-terminus. The step (2) synthesizes a peptide wherein the thioether group of the cysteine residue of the first antigen peptide in the peptide obtained in step (1) binds to the thioether group of the cysteine residue added to the N-terminus of the second antigen peptide. The "Nspy" is a 3-nitro-2-pyridylthio group.

The step (3) uses the peptide obtained in step (2) and a third antigen peptide that has a cysteine residue protected by Spy group at the N-terminus. The step (3) synthesizes a peptide wherein the thioether group of the N-terminus cysteine residue of the second antigen peptide in the peptide obtained in step (2) binds to the thioether group of the cysteine residue of the third antigen peptide. The Spy" is a 2-pyridylsulfide group.

The WT1 antigen peptide thus obtained may be purified according to any method known to those of ordinary skill in the art or generally used for peptide chemistry. For example, The WT1 antigen peptide may be purified by techniques such as various types of chromatography (e.g., silica gel column chromatography, ion exchange column chromatography, gel filtration or reversed-phase chromatography) and recrystallization. For example, the recrystallization solvent may be an alcohol solvent such as methanol, ethanol or 2-propanol; ether solvent such as diethyl ether; ester solvent such as ethyl acetate; aromatic hydrocarbon solvent such as benzene or toluene; ketone solvent such as acetone; hydrocarbon solvent such as hexane; aprotonic solvent such as dimethylformamide or acetonitrile; water; or a mixture thereof. Different purification methods described in Jikken Kagaku Kouza {The Chemical Society of Japan ed., Maruzen) vol. 1 or other references also may be used.

Purification methods for disulfide compounds are described in the references such as Peptide Synthesis, Interscience, New York, 1966; The Proteins, Vol. 2, Academic Press Inc., New York, 1976; peptide synthesis, Maruzen Co., LTD., 1975; Basics and Experiment of Peptide Synthesis, Maruzen Co., LTD., 1985; and Development of Pharmaceutical Product sequential vol. 14, peptide synthesis, Hirokawa Shoten, 1991. Among these methods, HPLC is preferred.

When the WT1 antigen peptide has one or more asymmetric points, the peptide may be prepared according to a general method using starting materials (amino acids) having the asymmetric point(s). To increase the optical purity of the WT1 antigen peptide, processes such as optical resolution may be performed at a suitable stage of the preparation. Examples of methods for optical resolution include a diastereomer method, which forms a salt of the WT1 antigen peptide or an intermediate thereof with an optically active acid (e.g., monocarboxylic acid such as mandelic acid, N-benzyloxyalanine, or lactic acid, dicarboxylic acid such as tartaric acid, o-diisopropylidenetartaric acid, or malic acid, or sulfonic acid such as camphorsulfonic acid or bromocamphorsulfonic acid) in an inert solvent (e.g., alcohol solvent such as methanol, ethanol, or 2-propanol, ether solvent such as diethyl ether, ester solvent such as ethyl acetate, hydrocarbon solvent such as toluene, aprotonic solvent such as acetonitrile, or a mixture thereof). When the WT1 antigen peptide or intermediate has an acidic functional group such as carboxy group, optical resolution may also be performed following the formation of a salt with an optically active amine (e.g., organic amine such as a-phenethylamine, kinin, quinidine, cinchonidine, cinchonine, or strychnine).

The temperature for forming the salt is selected from the range of room temperature to the boiling point of the solvent. To improve the optical purity, it is desirable to once raise the temperature to around the boiling point of the solvent. When a precipitated salt is collected by filtration, the yield may be increased as necessary by cooling. The optically active acid or amine may be used in an amount of about 0.5 - about 2.0 equivalents, preferably about 1 equivalent, relative to the substrate. Where necessary, crystals may be recrystallized in an inert solvent (e.g., alcohol solvent such as methanol, ethanol, or 2-propanol, ether solvent such as diethyl ether, ester solvent such as ethyl acetate, hydrocarbon solvent such as toluene, aprotonic solvent such as acetonitrile, or a mixture thereof) to provide an optically active salt with high purity. Where necessary, the optically resolved salt may be treated with an acid or base by a general method to give a free form.

The "pharmaceutically acceptable salt" as used herein may be an acid addition salt or a base addition salt. The acid addition salt may be an inorganic acid salt such as hydrochloride, hydrobromide, sulfate, hydroiodide, nitrate, and phosphate, or an organic acid salt such as citrate, oxalate, acetate, formate, propionate, benzoate, trifluoroacetate, maleate, tartrate, methanesulfonate, benzenesulfonate, or p-toluenesulfonate. The base addition salt may be a salt with an inorganic base such as sodium salt, potassium salt, calcium salt, magnesium salt, or ammonium salt, a salt with an organic base such as triethylammonium salt, triethanolammonium salt, pyridinium salt, or diisopropylammonium salt. Also, the salt may be an amino acid salt with a basic or acidic amino acid such as arginine, aspartic acid, and glutamic acid.

The pharmaceutically acceptable salt of a WT1 killer peptide may be an acid addition salt or a base addition salt of a peptide consisting of the amino acid sequence selected from
RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6),
RVPGVAPTL (SEQ ID NO: 7),
VLDFAPPGA, (SEQ ID NO: 8),
C-CMTWNQMNL (SEQ ID NO: 9) (wherein the bond within C-C is a disulfide bond), and
C-CYTWNQMNL (SEQ ID NO: 10) (wherein the bond within C-C is a disulfide bond), or
a peptide comprising an altered amino acid sequence of the amino acid sequence selected from SEQ ID NOS: 2-10 that comprises one to several amino acid deletions, substitutions, or additions in the amino acid sequence and having the CTL inducing activity; or
a compound represented by any one of the formulae (1) to (3). The acid addition salt may be an inorganic acid salt such as hydrochloride, hydrobromide, sulfate, hydroiodide, nitrate, and phosphate, or an organic acid salt such as citrate, oxalate, acetate, formate, propionate, benzoate, trifluoroacetate, maleate, tartrate, methanesulfonate, benzenesulfonate, or p-toluenesulfonate. The base addition salt may be a salt with an inorganic base such as sodium salt, potassium salt, calcium salt, magnesium salt, or ammonium salt, a salt with an organic base such as triethylammonium salt, triethanolammonium salt, pyridinium salt, or diisopropylammonium salt. Also, the salt may be an amino acid salt with a basic or acidic amino acid such as arginine, aspartic acid, and glutamic acid.

The pharmaceutically acceptable salt of a WT1 helper peptide may be an acid addition salt or a base addition salt of a peptide consisting of the amino acid sequence selected from
KRYFKLSHLQMHSRKH (SEQ ID NO: 11),
SGQARMFPNAPYLPSCLES(SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19) and
WAPVLDFAPPGASAYGSLC (SEQ ID NO: 20); or
a peptide comprising an altered amino acid sequence of the amino acid sequence selected from SEQ ID NOS: 11-20 that comprises one to several amino acid deletions, substitutions, or additions in the amino acid sequence and having the helper T cell inducing activity. The acid addition salt may be an inorganic acid salt such as hydrochloride, hydrobromide, sulfate, hydroiodide, nitrate, and phosphate, or an organic acid salt such as citrate, oxalate, acetate, formate, propionate, benzoate, trifluoroacetate, maleate, tartrate, methanesulfonate, benzenesulfonate, or p-toluenesulfonate. The base addition salt may be a salt with an inorganic base such as sodium salt, potassium salt, calcium salt, magnesium salt, or ammonium salt, a salt with an organic base such as triethylammonium salt, triethanolammonium salt, pyridinium salt, or diisopropylammonium salt. Also, the salt may be an amino acid salt with a basic or acidic amino acid such as arginine, aspartic acid, and glutamic acid.

The pharmaceutically acceptable salt of a WT1 killer peptide may be, but not limited to,
RMFPNAPYL acetate,
CMTWNQMNL acetate,
CYTWNQMNL acetate,
ALLPAVPSL acetate,
SLGEQQYSV acetate,
RVPGVAPTL acetate,
VLDFAPPGA acetate,
C-CMTWNQMNL acetate,
C-CYTWNQMNL acetate, 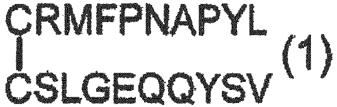
acetate, 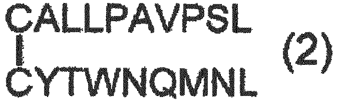
acetate, 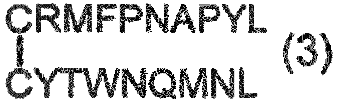
acetate,
KRYFKLSHLQMHSRKH acetate,
SGQARMFPNAPYLPSCLES acetate,
RSDELVRHHNMHQRNMTKL acetate,
PGCNKRYFKLSHLQMHSRKHTG acetate,
CNKRYFKLSHLQMHSRK acetate,
CNKRYFKLSHLQMHSRKH acetate,
CNKRYFKLSHLQMHSRKHTG acetate,
WAPVLDFAPPGASAYGSL acetate,
CWAPVLDFAPPGASAYGSL acetate,
WAPVLDFAPPGASAYGSLC acetate,
RMFPNAPYL trifluoroacetate,
CMTWNQMNL trifluoroacetate,
CYTWNQMNL trifluoroacetate,
ALLPAVPSL trifluoroacetate,
SLGEQQYSV trifluoroacetate,
RVPGVAPTL trifluoroacetate,
VLDFAPPGA trifluoroacetate,
C-CMTWNQMNL trifluoroacetate,
C-CYTWNQMNL trifluoroacetate, 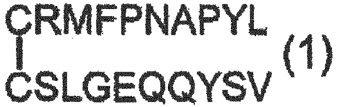 trifluoroacetate, 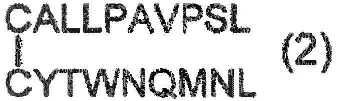
trifluoroacetate, 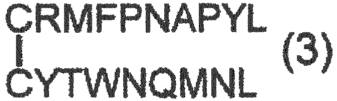
trifluoroacetate,
KRYFKLSHLQMHSRKH trifluoroacetate,
SGQARMFPNAPYLPSCLES trifluoroacetate,
RSDELVRHHNMHQRNMTKL trifluoroacetate,
PGCNKRYFKLSHLQMHSRKHTG trifluoroacetate,
CNKRYFKLSHLQMHSRK trifluoroacetate,
CNKRYFKLSHLQMHSRKH trifluoroacetate,
CNKRYFKLSHLQMHSRKHTG trifluoroacetate,
WAPVLDFAPPGASAYGSL trifluoroacetate,
CWAPVLDFAPPGASAYGSL trifluoroacetate, or
WAPVLDFAPPGASAYGSLC trifluoroacetate.

The present invention also encompasses any hydrate and solvate, such as ethanol solvate, of the WT1 antigen peptide or a pharmaceutically acceptable salt thereof. Further, the present invention encompasses any possible stereoisomer, such as diastereomer or enantiomer, or any crystal form of the WT1 antigen peptide.

The CTL inducing activity may be confirmed by measuring the number of CTLs by the HLA tetramer method (Int. J. Cancer: 100, 565-570 (2002)) or the limiting dilution method (Nat. Med.: 4, 321-327 (1998)). To determine HLA-A24-restricted CTL inducting activity, the HLA-A24 model mouse described in WO 02/47474 and Int. J. Cancer: 100, 565-570 (2002) may be used. The helper T cell inducing activity may be confirmed by any method known in the art, such as the method described in Cancer Immunol. Immunother. 51:271 (2002).

As used herein, the term "immunomodulator" means any agent that controls transmission of costimulatory signals during activation of T cells with antigen-presenting cells by interacting with molecules involved in the transmission of the costimulatory signals and present on the antigen-presenting cells and/or T cells, as well as any agent that directly or indirectly controls function of molecules involved in establishment of immune tolerance (immunosuppression) in the immune system. Examples of "immunomodulator" include, but are not limited to, antibodies, nucleic acids, proteins, peptides, and small compounds. As used in the context of "immunomodulator", the term "antibody" includes antibody fragments. Examples of the antibody fragment include heavy and light chain variable regions of an antibody (VH and VL), F(ab')2, Fab', Fab, Fv, Fd, sdFv, and scFV. As used in the context of "immunomodulator", the term "protein" means any protein other than antibodies. Examples of the term "immunomodulator" include immune checkpoint inhibitors, costimulatory molecule agonists, immune activating agents, and low-molecular inhibitors.

The "immune checkpoint inhibitor" inhibits immunosuppressive effect induced by cancer cells or antigen presenting cells. Examples of the immune checkpoint inhibitor include, but are not limited to, agents against a molecule selected from the group consisting of: (1) CTLA-4 (e.g., ipilimumab and tremelimumab); (2) PD-1 (e.g., nivolumab, pembrolizumab, AMP-224, AMP-514 (MEDI0680), and pidilizumab (CT-011)); (3) LAG-3 (e.g., IMP-321 and BMS-986016); (4) BTLA; (5) KIR (e.g., IPH2101); (6) TIM-3; (7) PD-L1 (e.g., durvalumab (MEDI4736), MPDL3280A, BMS-936559, and avelumab (MSB0010718C)); (8) PD-L2; (9) B7-H3 (e.g., MGA-271); (10) B7-H4; (11) HVEM; (12) GAL9; (13) CD160; (14) VISTA; (15) BTNL2; (16) TIGIT; (17) PVR; (18) BTN1A1; (19) BTN2A2; (20) BTN3A2 (Nat Rev Drug Discov. 2013; 12: 130-146; Nikkei Medical Cancer Review 2014; 9; Nat Rev Immunol. 2014; 14: 559-69); and (21) CSF1-R.

The "costimulatory molecule agonist" activates T-cells by transmitting an auxiliary signal via a costimulatory molecule on the T cells and/or antigen presenting cells to attenuate the immunosuppressive effect of cancer cells or antigen presenting cells. Examples of the costimulatory molecule agonist include, but are not limited to, agents against a molecule selected from the group consisting of: (1) 4-1BB; (2) 4-1BB-L; (3) OX40 (9) OX40-L; (5) GITR; (6) CD28; (7) CD40; (8) CD40-L; (9) ICOS; (10) ICOS-L; (11) LIGHT; and (12) CD27.

The "immune activating agent" efficiently stimulates killer T cells in the lymph nodes by directly or indirectly activating immune cells such as T cells and dendritic cells. Examples of the immune activating agent include, but are not limited to, Toll-like receptor (TLR) agonists, stimulator of interferon genes (STING) agonists, cytokines, and agents against heat shock protein (HSP).

Examples of the "Toll-like receptor (TLR) agonist" include, but are not limited to, TLR1/2 agonists, TLR2 agonists, TLR3 agonists (e.g., PolyI:C), TLR4 agonists (e.g., S-type lipopolysaccharide, paclitaxel, lipid A, and monophosphoryl lipid A), TLR5 agonists (e.g., flagellin), TLR6/2 agonists (e.g., MALP-2), TLR7 agonist, TLR7/8 agonists (e.g., gardiquimod, imiquimod, loxoribine, and resiquimod (R848)), TLR7/9 agonists (e.g., hydroxychloroquine sulfate), TLR8 agonists (e.g., motolimod (VTX-2337)), TLR9 agonists (e.g., CpG-ODN), and TLR11 agonists (e.g., profilin).

Examples of the "cytokine" include, but are not limited to, IL-1α, IL-1β, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (INF)-α, INF-β, INF-γ, SCF, GM-CSF, G-CSF, M-CSF, erythropoietin, thrombopoietin, macrophage inflammatory protein (MIP), and monocyte chemoattractant protein (MCP).

Examples of the "heat shock protein (HSP)" include, but are not limited to, HSP70, HSP90, HSP90α, H3P90β, HSP105, HSP72, and HSP40. Agents against a heat shock protein include HSP inhibitors. For example, examples of HSP90 inhibitor include, but are not limited to, tanespimycin (17-AAG), luminespib (AUY-922, NVP-AUY922), alvespimycin (17-DMAG) hydrochloride, ganetespib (STA-9090), BIIB021, onalespib (AT13387), geldanamycin, NVP-BEP800, SNX-2112 (PF-04928473), PF-4929113 (SNX-5422), KW-2478, XL8B8, VER155008, VER-50589, CH5138303, VER-49009, NMS-E973, PU-H71, HSP990 (NVP-HSP990) and KNK437.

Examples of the "low-molecular inhibitor" include, but are not limited to, histone deacetylase inhibitors, histone demethylase inhibitors, histone acetyltransferase inhibitors, histone methyltransferase inhibitors, DNA methyltransferase inhibitors, anthracycline antibiotics, platinum formulations, MAPK inhibitors, β-catenin inhibitors, STAT3 inhibitors, NF-kB inhibitors, JAK inhibitors, mTOR inhibitors, IDO inhibitors, COX-2 inhibitors, CXCR4 inhibitors, and arginase inhibitors.

Examples of the "histone deacetylase inhibitor" include, but are not limited to, vorinostat (SAHA, MK0683), entinostat (MS-275), panobinostat (LBH589), trichostatin A (TSA), mocetinostat (MGCD0103), BG45, BRD73954, belinostat (PXD101), romidepsin (FK228, depsipeptide), 4SC-202, HPOB, LMK-235, CAY10603, tasquinimod, TMP269, nexturastat A, rocilinostat (ACY-1215), RGFP966, RG2833 (RGFP109), scriptaid, tubastatin A, pracinostat (SB939), CUDC-101, M344, PCI-34051, dacinostat (LAQ824), tubastatin A hydrochloride, abexinostat (PCI-24781), CUDC-907, AR-42, sodium phenylbutyrate, resminostat, tubacin, quisinostat (JNJ-26481585) dihydrochloride, MC1568, givinostat (ITF2357), droxinostat, chidamide (C S055, HBI-8000), CHR-2485, CHR-3996, DAC-060, FRM-0334 (EVP-0334), MGCD-290, CXD-101 (AZD-9468), CG200745, arginine butyrate, sulforaphane, SHP-141, CUDC-907, YM753 (OBP-801), sodium valproate, apicidin, and CI994 (tacedinaline).

Examples of the "histone demethylase inhibitor" include, but are not limited to, GSK J4 HCl, OG-L002, JIB-04, IOXl, SP2509, ORY-1001 (RG-6016), GSK J1, ML324, and GSK-LSD1 2HCl.

Examples of the "histone acetyltransferase inhibitor" include, but are not limited to, C646, MG149, remodelin, and anacardic acid.

Examples of the "histone methyltransferase inhibitor" include, but are not limited to, pinometostat (EPZ5676), EPZ005678, GSK343, BIX01294, tazemetostat (EPZ6438), 3-deazaneplanocin A (DZNeP) HCl, UNC1999, MM-102, SGC0946, entacapone, EPZ015666, UNC0379, EI1, MI-2 (menin-MLL inhibitor), MI-3 (menin-MLL inhibitor), PFI-2, GSK126, EPZ04777, BRD4770, GSK-2816126, and UNC0631.

Examples of the "DNA methyltransferase inhibitor" include, but are not limited to, decitabine, azatidine, RG108, thioguanine, zebularine, SGI-110, CC-486, SGI-1027, lomeguatrib, and procainamide hydrochloride.

The "anthracycline antibiotic" is intercalated between DNA strands to inhibit DNA relaxation. Examples of the anthracycline antibiotic include, but are not limited to, doxorubicin, liposomal doxorubicin, daunorubicin, pirarubicin, epirubicin, idarubicin, aclarubicin, amrubicin, aloin, and mitoxantrone.

Examples of the "platinum formulation" include, but are not limited to, cisplatin, carboplatin, miboplatin, nedaplatin, satraplatin (JM-126), oxaliplatin (ELOXATIN), triplatin tetranitrate, and DDS formulations thereof.

Examples of the "MAPK inhibitor" include, but are not limited to, SB203580, doramapimod (BIRB796), SB202190 (FHPI), LY2228820, VX-702, SB239063, pexmetinib (ARRY-614), PH-797804, VX-745, and TAK-715.

Examples of the "β-catenin inhibitor" include, but are not limited to, XAV-939, ICG-001, IWR-1-endo, Wnt-C59 (C59), LGK-974, KY02111, IWP-2, IWP-L6, NIKI4, and FH535.

Examples of the "STAT3 inhibitor" include, but are not limited to, S3I-201, Stattic, niclosamide, nifuroxazide, napabucasin (BBI-608), cryptotanshinone, HO-3867, WHI-P154, FLLL32, STA-21, WP1066, and SH-4-54.

Examples of the "NF-kB inhibitor" include, but are not limited to, QNZ (EVP4593), sodium 4-aminosalicylate, JSH-23, phenethyl caffeate, sodium salicylate, andrographolide, and SC75741.

Examples of the "JAK inhibitor" include, but are not limited to, ruxolitinib (INCB018424), tofacitinib (CP-690550) citrate, AZD1480, fedratinib (SAR302503, TG101348), AT9283, tyrphostin B42 (AG-490), momelotinib (CYT387), tofacitinib (CP-690550, tasocitinib), WP1066, TG101209, gandotinib (LY2784544), NVP-BSK805 2HCl, baricitinib (LY3009104, INCB02850), AZ960, CEP-33779, pacritinib (SB1518), WHI-P154, XL019, S-ruxolitinib (INCB018424), ZM39923 HCl, decernotinib (VX-509), cerdulatinib (PRT062070, PRT2070), filgotinib (GLPG0634), FLLL32, peficitinib (ASP015K, JNJ-54781532), GLPG0634 analogue, Go6976, and Curcumol.

Examples of the "mTOR inhibitor" include, but are not limited to, sirolimus (rapamycin), deforolimus (AP23573, MK-8669), everolimus (RAD-001), temsirolimus (CCI-779, NSC683864), zotarolimus (ABT-578), biolimus A9 (umirolimus), AZD8055, KU-0063794, voxtalisib (XL765, SAR245409), MHY1485, dactolisib (BEZ235, NVP-BEZ235), PI-103, and torkinib (PP242).

Examples of the "IDO inhibitor" include, but are not limited to, NLG919, INCB024360 analog, indoximod (NLG-8189), and epacadostat (INCB024360).

Examples of the "COX-2 inhibitor" include, but are not limited to, valdecoxib, rofecoxib, carprofen, celecoxib, lumiracoxib, tolfenamic acid, nimesulide, niflumic acid, asaraldehyde, lornoxicam, sodium meclofenamate, amfenac sodium hydrate, diclofenac sodium, ketoprofen, ketorolac, naproxen sodium, indomethacin, ibuprofen, aspirin, mefenamic acid, bromfenac sodium, oxaprozin, zaltoprofen, and nepafenac.

Examples of the "CXCR4 inhibitor" include, but are not limited to, WZ811, plerixafor (AMD3100), and plerixafor 8HCl (AMD3100 SHCl).

The WT1 antigen peptide disclosed herein produces anti-cancer effect when combined with an immunomodulator. The effect may be further enhanced or the QOL of the patient may be improved by combining the WT1 antigen peptide with at least one additional medicament (multidrug therapy).

The WT1 antigen peptide disclosed herein may be used in combination with one or more drugs selected from the group consisting of "hormone therapy agent", "immunotherapeutic agent", "biopharmaceutical", "cell growth factor", "cell growth factor inhibitor", "cell growth factor receptor inhibitor", "radiotherapeutic agent", "auxiliary agent", and "chemotherapeutic agent". Preferably, the WTl antigen peptide disclosed herein may be used in combination with one to five drugs selected from the group. More preferably, the WT1 antigen peptide disclosed herein may be used in combination with one to three drugs selected from the group. Particularly preferably, the WT1 antigen peptide disclosed herein may be used in combination with one drug selected from the group. The drug that may be combined with the WT1 antigen peptide disclosed herein and the immunomodulator is hereinafter referred to as "concomitant drug". The dose of the concomitant drug may be appropriately determined on the basis of the generally employed clinical dose.

Examples of the "hormone therapy agent" include adrenal cortical hormone agents (e.g., steroidal antiinflammatory agents, estrogen preparations, progesterone preparations, and androgen preparations), anti-estrogen agents, estrogen-controlling agents, estrogen synthesis inhibitors, anti-androgen agents, androgen-controlling agents, androgen synthesis inhibitors, LH-RH agonist preparations, LH-RH antagonist preparations, aromatase inhibitors, steroid-lactonase inhibitors, contraceptive pills, retinoids, and agents which delay metabolism of a retinoid.

Examples of the "hormone therapy agent" include fosfestrol, diethylstilbestrol, fluoxymesterol, chlorotrianisene, methyl testosterone, medroxyprogesterone acetate, megestrol acetate, chlormadinone acetate, cyproterone acetate, danazol, allylestrenol, gestrinone, mepartricin, raloxifene, ormeloxifene, levormeloxifene, tamoxifen citrate, toremifene citrate, iodoxyfene, pill formulations, mepitiostane, testololactone, aminoglutethimide, goserelin acetate, buserelin, leuprorelin, leuprolide, droloxifene, epitiostanol, ethinylestradiol sulfonate, estramustine, fadrozole hydrochloride, anastrozole, terorazole, ketoconazole, letrozole, exemestane, vorozole, formestane, exemestane, flutamide, bicalutamide, nilutamide, enzalutamide, mifepristone, finasteride, dexamethasone, prednisolone, betamethasone, triamcinolone, abiraterone, liarozole, bexarotene, and DNl01.

Examples of the "immunotherapeutic agent" include picibanil, krestin, sizofiran, lentinan, ubenimex, interferon (IFN)-α, interferon (IFN)-β, interferon (IFN)-γ, interleukin, macrophage colony stimulating factor, granulocyte-colony stimulating factor, erythropoietin, lymphotoxin, BCG vaccine, *Corynebacterium parvum,* levamisole, polysaccharide K, procodazole, anti-CTLA4 antibody, anti-PD-1 antibody, and TLR agonists (e.g., TLR7 agonists, TLR8 agonists, TLR9 agonists).

Examples of the "biopharmaceutical" include, but are not limited to, interleukin-2 (aldesleukin), interferon-α, interferon-β, interferon-γ, erythropoietin (EPO), granulocyte-colony stimulating factor (filgrastim), granulocyte-macrophage-colony stimulating factor (sargramostim), IL13-PE38QQR, Bacille Calmette-Guerin, levamisole, octreotide, CPG7909, Provenge, GVAX, Myvax, Favld, lenalidomide, trastuzumab, rituximab, gemtuzumab ozogamicin, alemtuzumab, endostatin, ibritumomab tiuxetan, tositumomab, cetuximab, zanolimumab, ofatumumab, HGS-ETR1, pertuzumab, M200, SGN-30, matuzumab, adecatumumab, denosumab, zalutumumab, MDX-060, nimotuzumab, MORAb-003, Vitaxin, MDX-101, MDX-010, DPC4 antibodies, NF-1 antibodies, NF-2 antibodies, Rb antibodies, p53 antibodies, WT1 antibodies, BRCA1 antibodies, BRCA2 antibodies, ganglioside (GM2), prostate specific antigens (PSA), α-fetoprotein (AFP), carcinoembryonic antigens (CEA), melanoma-associated antigens (MART-1, gap100, MAGE 1,3 tyrosine), papilloma virus E6 and E7 fragments, and DDS formulations thereof.

Regarding the "cell growth factor", the "cell growth factor inhibitor", and the "cell growth factor receptor inhibitor", the cell growth factor is an agent that promotes cell proliferation. For example, the cell growth factor may be a peptide that has molecular weight of not more than 20,000 and can produce the effect at low concentration through binding to a receptor.

Examples of the "cell growth factor" include, but are not limited to, epidermal growth factor (EGF), insulin-like growth factor (IGF (e.g., insulin, IGF-1, and IGF-2)), transforming growth factor (TGF (e.g., TGF-α and TGF--β)), nerve growth factor (NGF), brain-derived neurotrophic factor (BDNF), vascular endothelial growth factor (VEGF), colony stimulating factor (CSF (e.g., granulocyte-colony stimulating factor (G-CSF)), granulocyte-macrophage-colony stimulating factor (GM-CSF)), platelet-derived growth factor (PDGF), erythropoietin (EPO), fibroblast growth factor (FGF (e.g., acidic FGF, basic FGF, keratinocyte growth factor (KGK), and FGF-10)), hepatocyte growth factor (HGF), heregulin, and angiopoietin. The term "cell growth factor" is synonymous with the term "growth factor".

Examples of the "cell growth factor inhibitor" include, but are not limited to, epidermal growth factor inhibitors (EGF inhibitors), insulin-like growth factor inhibitors (IGF inhibitors), nerve growth factor inhibitors (NGF inhibitors), brain-derived neurotrophic factor inhibitors (BDNF inhibitors), vascular endothelial cell growth factor inhibitors (VEGF inhibitors), colony stimulating factor inhibitors (CSF inhibitors), platelet-derived growth factor inhibitors (PDGF inhibitors), erythropoietin inhibitors (EPO inhibitors), fibroblast growth factor inhibitors (FGF inhibitors), hepatocyte growth factor inhibitors (HGF inhibitors), heregulin inhibitors, and angiopoietin inhibitors. The term "cell growth factor inhibitor" is synonymous with the term "growth factor inhibitor".

Examples of the "cell growth factor receptor inhibitor" include, but are not limited to, epidermal growth factor receptor inhibitors (EGFR inhibitors), insulin-like growth factor receptor inhibitors (IGFR inhibitors), nerve growth factor receptor inhibitors (NGFR inhibitors), brain-derived neurotrophic factor receptor inhibitors (BDNFR inhibitors), vascular endothelial cell growth factor inhibitors (VEGF inhibitors), colony stimulating factor inhibitors (CSF inhibitors), platelet-derived growth factor receptor inhibitors (PDGFR inhibitors), erythropoietin receptor inhibitors (EPOR inhibitors), fibroblast growth factor receptor inhibitors (FGFR inhibitors), hepatocyte growth factor receptor inhibitors (HGFR inhibitors), heregulin receptor inhibitors, and angiopoietin receptor inhibitors. The term "cell growth factor receptor inhibitor" is synonymous with the term "growth factor receptor inhibitor".

Examples of the "radiotherapeutic agent" include, but are not limited to, radioactive materials and radiosensitizers.

The "auxiliary agent" is used for suppressing an adverse effect caused by an anticancer agent such as a side effect or vomiting. Examples of the auxiliary agent include, but are not limited to, aprepitant, ondansetron, lorazepam, dexamethasone, diphenhydramine, ranitidine, cimetidine, ranitidine, famotidine, cimetidine, Procrit, epoetin alfa, filgrastim, oprelvekin, leucovorin, and granulocyte-macrophage-colony stimulating factor (GM-CSF).

Examples of the "chemotherapeutic agent" include, but are not limited to, alkylating agents, platinum formulations, antimetabolites, topoisomerase inhibitors, DNA intercalators, antimitotic agents, antitumor antibiotics, plant-derived anticancer agents, epigenome drugs, immunomodulators, molecular targeted therapeutics, angiogenesis inhibitors, and other chemotherapeutic agents. Some typical examples of the chemotherapeutic agent are listed below.

Examples of the "alkylating agent" include, but are not limited to, nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, procarbazine, ranimustine, estramustine sodium phosphate, triethylenemelamine, carmustine, lomustine, streptozocin, pipobroman, etoglucid, altretamine, ambamustine, dibrospidium hydrochloride, fotemustine, prednimustine, bendamustine, uramustine, semustine, pumitepa, ribomustin, temozolomide, treosulfan, trofosfamide, zinostatin stimalamer, adozelesin, cystemustine, bizelesin, mechlorethamine, uracil mustard, streptozocin, trabectedin, becaterin, chlormethine, mannosulfan, triaziquone, procarbazine, canfosfamide, nitrosoureas, and DDS formulations thereof.

Examples of the "platinum formulation" include, but are not limited to, cisplatin, carboplatin, miboplatin, nedaplatin, satraplatin, oxaliplatin, triplatin tetranitrate, and DDS formulations thereof.

Examples of the "antimetabolite" include, but are not limited to, antifolates, pyrimidine metabolism inhibitors, purine metabolism inhibitors, ribonucleotide reductase inhibitors, and nucleotide analogs.

Examples of the "antimetabolite" include, but are not limited to, mercaptopurine, 6-mercaptopurine riboside, thioinosine, methotrexate, pemetrexed, eoshitabin, enocitabine, cytarabine, cytarabine ocfosfate, ancitabine hydrochloride, 5-FU agents (e.g., fluorouracil, Carzona1, Bennan, Lunachol, Lunapon, tegafur, tegafur-uracil, tegafur-gimeracil-oteracil potassium (TS-1), UFT, doxifluridine, carmofur, gallocitabine, emitefur, and capecitabine), aminopterin, nelarabine, leucovorin calcium, Tabloid, butocine, folinate calcium, levofolinate calcium, cladribine, emitefur, fludarabine, gemcitabine, hydroxycarbamide, pentostatin, piritrexim, idoxuridine, mitoguazone, tiazofurine, ambamustine, bendamustine, floxuridine, nelarabine, leucovorin, hydroxyurea, thioguanine, asparaginase, bortezomib, raltitrexed, clofarabine, enocitabine, sapacitabine, azacytidine, sulfadiazine, sulfamethoxazole, trimethoprim, Liproxstatin-1, D4476, Xanthohumol, Epacadostat (INCB024360), Vidofludimus, P7C3, GMX1778 (CHS828), NCT-501, SW033291, Ro61-8048, and DDS formulations therof.

Examples of the "topoisomerase inhibitor" include, but are not limited to, doxorubicin, daunorubicin, epirubicin, idarubicin, anthracenedione, mitoxantrone, mitomycin C, bleomycin, dactinomycin, plicatomycin, irinotecan, camptothecin, rubitecan, belotecan, etoposide, teniposide, topotecan, amsacrine, and DDS formulations thereof.

Examples of the "DNA intercalator" include, but are not limited to, proflavine, doxorubicin (adriamycin), daunorubicin, dactinomycin, thalidomide, and DDS formulations thereof.

Examples of the "antimitotic agent" include, but are not limited to, paclitaxel, paclitaxel derivatives (e.g., DHA paclitaxel, paclitaxel polyglutamate, nab-paclitaxel, micellar paclitaxel, 7α-glucosyloxyacetylpaclitaxel, and BMS-275183), docetaxel, vinorelbine, vincristine, vinblastine, vindesine, vinzolidine, etoposide, teniposide, ixabepilone, larotaxel, ortataxel, tesetaxel, ispinesib, colchicine, vinflunine, and DDS formulations thereof.

Examples of the "antitumor antibiotic" include, but are not limited to, actinomycin D, actinomycin C, mitomycin C, chromomycin A3, mithramycin A, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, amrubicin hydrochloride, neocarzinostatin, zinostatin stimalamer, mithramycin, sarkomycin, carzinophilin, mitotane, zorubicin hydrochloride, mitoxantrone hydrochloride, idarubicin hydrochloride, liposomal doxorubicin, and DDS formulations thereof.

Examples of the "plant-derived anticancer agent" include, but are not limited to, irinotecan, nogitecan, etoposide, etoposide phosphate, eribulin, sobuzoxane, vinblastine sulfate, vincristine sulfate, vindesine sulfate, teniposide, paclitaxel, paclitaxel injection, docetaxel, DJ-927, vinorelbine, topotecan, and DDS formulations thereof.

Examples of the "epigenome drug" include, but are not limited to, DNA methylation inhibitors, histone deacetylase (HDAC) inhibitors, DNA methyl transferase (DNMT) inhibitors, histone deacetylase activators, histone demethylase inhibitors, and methylated nucleotides.

Examples of the "epigenome drug" include, but are not limited to, vorinostat, belinostat, mocetinostat (MGCD0103), entinostat (SNDX-275), romidepsin, azacytidine, decitabine, GSK2879552 2H1, SGC707, ORY-1001 (RG-6016), PFI-4, SirReal2, GSK2801, CPI-360, GSK503, AMI-1, CPI-169, and DDS formulations thereof.

Examples of the "immunomodulator" include, but are not limited to, thalidomide, lenalidomide, pomalidomide, and DDS formulations thereof.

The "molecular targeted therapeutics" may be a small compound or an antibody. Examples of the "molecular targeted therapeutics" include, but are not limited to, kinase inhibitors, proteasome inhibitors, monoclonal antibodies, mTOR inhibitors, TNF inhibitors, and T-cell inhibitors.

Examples of the "kinase inhibitor" include, but are not limited to, tyrosine kinase inhibitors, serine/threonine kinase inhibitors, Raf kinase inhibitors, cyclin-dependent kinase (CDK) inhibitors, and mitogen-activated protein kinase (MEK) inhibitors.

Specific examples of the "kinase inhibitor" include, but are not limited to, imatinib, gefitinib, erlotinib, afatinib, dasatinib, bosutinib, vandetanib, sunitinib, axitinib, pazopanib, lenvatinib, lapatinib, nintedanib, nilotinib, crizotinib, ceritinib, alectinib, ruxolitinib, tofacitinib, ibrutinib, sorafenib, vemurafenib, dabrafenib, palbociclib, trametinib, regorafenib, cedivanib, lestaurtinib, bandetinib, vatalanib, seliciclib, tivantinib, canertinib, pelitinib, tesevatinib, cediranib, motesanib, midostaurin, foretinib, cabozantinib, selumetinib, neratinib, volasertib, saracatinib, enzastaurin, tandutinib, semaxanib, alvocidib, ICR-62, AEE788, PD0325901, PD153035, TK787, BBI503, E6201, E7050, and DDS formulations thereof.

Examples of the "proteasome inhibitor" include, but are not limited to, bortezomib, carfilzomib, and DDS formulations thereof.

Examples of the "monoclonal antibody" include, but are not limited to, anti-CD22 antibodies, anti-CD20 antibodies, anti-CD25 antibodies, anti-CD30 antibodies, anti-CD33 antibodies, anti-CD5 antibodies, anti-CD52 antibodies, anti-epidermal growth factor receptor antibodies (EGFR antibodies), anti-vascular endothelial cell growth factor antibodies (VEGF antibodies), anti-TNF-α antibodies, anti-IL-1 receptor antibodies, anti-IL-2 receptor antibodies, anti-IL-5 receptor antibodies, anti-IL-6 receptor antibodies, anti-HER2 antibodies, anti-IgE antibodies, anti-IgG antibodies, anti-RS virus antibodies, anti-CCR4 antibodies, anti-cytotoxic T lymphocyte-associated antigen 4 (CTLA-4, CD152) antibodies, anti-PD-1 antibodies, anti-receptor activator of nuclear factor xB ligand (RANKL) antibodies, anti-c-Met antibodies, and anti-CXCR4 antibodies.

Specific examples of the "monoclonal antibody" include, but are not limited to, ibritumomab tiuxetan, rituximab, cetuximab, infliximab, basiliximab, brentuximab vedotin, tocilizumab, trastuzumab, bevacizumab, omalizumab, mepolizumab, gemtuzumab, ozogamicin, palivizumab, ranibizumab, certolizumab, ocrelizumab, mogamulizumab, eculizumab, pertuzumab, alemtuzumab, inotuzumab, panitumumab, ofatumumab, golimumab, adalimumab, ramucirumab, nivolumab, anakinra, denosumab, ipilimurnab, pembrolizumab, matuzumab, farletuzumab, MORAb-004, MORA-b009, and DDS formulations thereof.

Examples of the "mTOR inhibitor" include, but are not limited to, everolimus (RAD001), rapamycin (sirolimus), AZD8055, temsirolimus (CCI-779, NSC683864), KU-0063794, voxtalisib (XL-765, SAR245409), MHY1485, dactolisib (BEZ235), PI-103, torkinib (PP242), ridaforolimus (deforolimus, MK-8669), INK-128 (MLN0128), Torin1, omipalisib (GSK2126458, GSK458), OSI-027, PF-04691502, apitolisib (GDC-0980, RG7422), GSK1059615, gedatolisib (PF-05212384, PKI-587), WYE-132, PP121, WYE-354, AZD2014, Torin2, WYE-687, CH5132799, WAY-600, ETP-46464, GDC-0349, XL388, zotarolimus (ABT-578), tacrolimus (FK506), BGT226 (NVP-BGT226), Palomid 529 (P529), chrysophanic acid, and DDS formulations thereof.

Examples of the "TNF inhibitor" include, but are not limited to, etanercept, lenalidomide (CC-5013), pomalidomide, thalidomide, necrostatin-1, and QNZ (EVP4593).

Examples of the "T-cell inhibitor" include, but are not limited to, abatacept.

Examples of the "angiogenesis inhibitor" include, but are not limited to, CM101, IFN-α, IL-12, platelet factor-4, suramin, semaxanib, thrombospondin, VEGFR antagonists, combinations of an angiostatic steroid and heparin, cartilage-derived angiogenesis inhibitors, matrix metalloproteinase inhibitors, batimastat, marimastat, angiostatin, endostatin, 2-methoxyestradiol, tecogalan, thrombospondin, αVβ3 inhibitors, linomide, ADH-1, E7820, and DDS formulations thereof.

Examples of the "other chemotherapeutic agent" include, but are not limited to, finasteride, sobuzoxane, obatoclax, efaproxiral, tipifarnib, and lonafarnib.

The pharmaceutical composition of the invention may comprise a further ingredient such as, but not limited to, a pharmaceutically acceptable carrier in addition to the active ingredient(s), the WT1 antigen peptide and/or the immunomodulator. The WT1 antigen peptide in the pharmaceutical composition induces WTl-specific CTLs and/or WT1-specific helper T cells, and thus the pharmaceutical composition may also comprise or may be administered with a suitable adjuvant to increase the induction efficiency.

The pharmaceutically acceptable carrier is not toxic to the cell or mammal which receives the carrier at the amount and concentration applied to the cell or mammal. The pharmaceutically acceptable carrier may often be a pH buffered aqueous solution. Examples of pharmaceutically acceptable carriers include: buffering agents (such as phosphate, citrate, lactate, tartarate, trifluoroacetate and other organic acids); antioxidants (such as ascorbic acid); low molecular weight polypeptides (less than about 10 residues); proteins (such as serum albumin, gelatin or immunoglobulin); hydrophilic polymers (such as polyvinylpyrrolidone); amino acids (such as glycine, glutamine, asparagine, arginine, methionine or lysine ); monosaccharides, disaccharides and other carbohydrates (such as glucose, mannose or dextrin); chelating agents (such as EDTA); sugar alcohols (such as mannitol, trehalose or sorbitol); stabilizers (such as diethylenetriaminepentaacetic acid); salt forming counterion (such as sodium); solubilizing agents (such as polysorbate 80^{®}, and/or nonionic surfactants (such as TWEEN^{®}, polyethylene glycol (PEG) and PLURONICS^{®}). The pharmaceutically acceptable carrier may be a macromolecule that is large and slowly metabolized, such as a protein, polypeptide, liposome, polysaccharide, polylactide, polyglycolic acid, polymeric amino acid, amino acid copolymer, or inactive virus particle. The WT1 antigen peptide may be administered in the form of a liposome preparation, a particulate preparation comprising the peptide bound to a bead with a diameter of several micrometers, or a preparation comprising the peptide bound to a lipid.

The pharmaceutical composition may also comprise or may be administered with a suitable adjuvant to efficiently establish the cellular immunity. The adjuvant may be an agent described in Clin. Microbiol. Rev., 7: 277-289, 1994. Specifically, the adjuvant may be any of fungus-derived components, GM-CSF, cytokines such as interleukin-2, interleukin-7, and interleukin-12, plant-derived components, marine organism-derived components, mineral gels such as aluminum hydroxide, lysolecithin, surfactants such as pluronic polyol, polyanion, peptides, and oil emulsions (emulsion preparation). Examples of the fungus-derived components include lipid A, monophosphoryl lipid A, which is a derivative of lipid A, dead bacteria (Mycobacterium bacteria such as BCG bacteria), bacterium-derived proteins, polynucleotides, Freund's incomplete adjuvant, Freund's complete adjuvant, cell wall skeleton components (e.g., BCG-CWS), trehalose dimycolate (TDM).

Also, the adjuvant may be a sedimentary adjuvant or an oil adjuvant. The sedimentary adjuvant is a suspension of an inorganic substance that absorbs peptides. Examples of the sedimentary adjuvant include sodium hydroxide, aluminum hydroxide (Alum), calcium phosphate, aluminum phosphate, aluminum salts, Pepesu, and carboxyvinyl polymer. The oil adjuvant emulsifies aqueous solutions comprising peptides by forming micelles with mineral oil. Examples of the oil adjuvant include, but not limited to, liquid paraffin, lanolin, Freund's adjuvant (Freund's complete adjuvant, Freund's incomplete adjuvant), Montanide, and W/O emulsion (see WO2006/078059).

The pharmaceutical composition of the present invention may comprise an excipient selected from, but not limited to, a sugar alcohol, such as mannitol, trehalose, or lactose; a pH adjusting agent selected from the group consisting of hydrochloric acid, sulfuric acid, nitric acid, acetic acid, citric acid, tartaric acid, lactic acid, maleic acid, phosphoric acid, sodium hydroxide, potassium hydroxide, aqueous ammonia, sodium acetate hydrate, anhydrous sodium acetate, sodium citrate hydrate, sodium dihydrogen citrate, sodium tartrate, disodium phosphate, dipotassium phosphate, sodium dihydrogen phosphate, potassium dihydrogen phosphate, trisodium phosphate, and any other pH adjusting agents conventionally used in pharmaceutical compositions; a filler; a buffer; a suspending agent; a wetting agent; a solubilizer; a dispersant; a preservative; and/or a coloring agent.

The pharmaceutical composition may be provided as a solid or liquid dosage form for oral administration, or a dosage form for parenteral administration by injection, topical application, inhalation or transnasal application, or as a suppository. Examples of the solid dosage form for oral administration include a tablet, a pill, a capsule (including a hard capsule and a soft capsule), a powder, and a granule. The pharmaceutical composition may also be formulated into such a tablet form as a sublingual tablet, a buccal tablet, or a rapidly disintegrating oral tablet.

The solid oral dosage form may be prepared in accordance with a conventionally known preparation method, and may comprise one or more active agents either alone or in combination with a filler (such as lactose, mannitol, glucose, microcrystalline cellulose, or starch), a binder (such as hydroxypropyl cellulose, polyvinyl pyrrolidone, or magnesium aluminometasilicate), a disintegrating agent (such as calcium cellulose glycolate), a lubricant (such as magnesium stearate), a stabilizer, a solubilizing aid (such as glutamic acid, or aspartic acid), or any other appropriate excipient(s). The solid oral dosage form may optionally be coated with sucrose, gelatin, hydroxypropyl cellulose, hydroxypropylmethyl cellulose phthalate, or any other appropriate coating agent(s). Two or more layers of coating may be applied on the dosage form. The solid oral dosage form may be a capsule which is formed of a digestible substance, such as gelatin. The solid oral dosage form may additionally comprise a preservative, an antioxidant, a coloring agent, a sweetener, or any other appropriate additive(s).

The pharmaceutical composition in the form of a sublingual tablet may be prepared in accordance with a conventionally known preparation method. In particular, the sublingual tablet may be prepared by incorporating one or more active agents with a filler (such as lactose, mannitol, glucose, microcrystalline cellulose, colloidal silica, or starch), a binder (such as hydroxypropyl cellulose, polyvinyl pyrrolidone, or magnesium aluminometasilicate), a disintegrating agent (such as starch, L-hydroxypropyl cellulose, carboxymethyl cellulose, croscarmellose sodium, or calcium cellulose glycolate), a lubricant (such as magnesium stearate), a swelling agent (such as hydroxypropyl cellulose, hydroxypropylmethyl cellulose, carbopol, carboxymethyl cellulose, polyvinyl alcohol, xanthan gum, or guar gum), a swelling aid (such as glucose, fructose, mannitol, xylitol, erythritol, maltose, trehalose, phosphate, citrate, silicate, glycine, glutamic acid, or arginine), a stabilizer, a solubilizing aid (such as polyethylene glycol, propylene glycol, glutamic acid, or aspartic acid), a flavoring agent (such as an orange, strawberry, mint, lemon, or vanilla flavor), or any other appropriate excipient(s) . The sublingual tablet may optionally be coated with sucrose, gelatin, hydroxypropyl cellulose, hydroxypropylmethyl cellulose phthalate, or any other appropriate coating agent(s). Two or more layers of coating may be applied on the tablet. The sublingual tablet may additionally comprise a preservative, an antioxidant, a coloring agent, a sweetener, or any other appropriate additive (s) .

The pharmaceutical composition in the form of a buccal tablet may be prepared in accordance with a conventionally known preparation method. In particular, the buccal tablet may be prepared by incorporating one or more active agents with a filler (such as lactose, mannitol, glucose, microcrystalline cellulose, colloidal silica, or starch), a binder (such as hydroxypropyl cellulose, polyvinyl pyrrolidone, or magnesium aluminometasilicate), a disintegrating agent (such as starch, L-hydroxypropyl cellulose, carboxymethyl cellulose, croscarmellose sodium, or calcium cellulose glycolate), a lubricant (such as magnesium stearate), an adhesive agent (such as hydroxypropyl cellulose, hydroxypropylmethyl cellulose, carbopol, carboxymethyl cellulose, polyvinyl alcohol, xanthan gum, or guar gum), an adhesive aid (such as glucose, fructose, mannitol, xylitol, erythritol, maltose, trehalose, phosphate, citrate, silicate, glycine, glutamic acid, or arginine), a stabilizer, a solubilizing aid (such as polyethylene glycol, propylene glycol, glutamic acid, or aspartic acid), a flavoring agent (such as an orange, strawberry, mint, lemon, or vanilla flavor), or any other appropriate excipient(s). The buccal tablet may optionally be coated with sucrose, gelatin, hydroxypropyl cellulose, hydroxypropylmethyl cellulose phthalate, or any other appropriate coating agent(s). Two or more layers of coating may be applied on the tablet. The buccal tablet may additionally comprise a preservative, an antioxidant, a coloring agent, a sweetener, or any other appropriate additive(s).

The pharmaceutical composition in the form of a rapidly disintegrating oral tablet may be prepared in accordance with a conventionally known preparation method. In particular, for preparing the rapidly disintegrating oral tablet, one or more active agents may be provided in a powder or granule form, which may optionally be coated with a coating agent (such as ethyl cellulose, hydroxypropyl cellulose, hydroxypropylmethyl cellulose, or an acrylic acid/methacrylic acid copolymer) or a plasticizer (such as polyethylene glycol, or triethyl citrate). Then, the powder or granule form of active agents may be incorporated with a filler (such as lactose, mannitol, glucose, microcrystalline cellulose, colloidal silica, or starch), a binder (such as hydroxypropyl cellulose, polyvinyl pyrrolidone, or magnesium aluminometasilicate), a disintegrating agent (such as starch, L-hydroxypropyl cellulose, carboxymethyl cellulose, croscarmellose sodium, or calcium cellulose glycolate), a lubricant (such as magnesium stearate), a disintegrating aid (such as glucose, fructose, mannitol, xylitol, erythritol, maltose, trehalose, phosphate, citrate, silicate, glycine, glutamic acid, or arginine), a stabilizer, a solubilizing aid (such as polyethylene glycol, propylene glycol, glutamic acid, or aspartic acid), a flavoring agent (such as an orange, strawberry, mint, lemon, or vanilla flavor), or any other appropriate excipient(s), thereby forming a rapidly disintegrating oral tablet. The rapidly disintegrating oral tablet may optionally be coated with sucrose, gelatin, hydroxypropyl cellulose, hydroxypropylmethyl cellulose phthalate, or any other appropriate coating agent(s) . Two or more layers of coating may be applied on the tablet. The rapidly disintegrating oral tablet may additionally comprise a preservative, an antioxidant, a coloring agent, a sweetener, or any other appropriate additive(s) .

The pharmaceutical composition in a liquid dosage form for oral administration may be in the form of a solution, a suspension, an emulsion, a syrup, or an elixir, in which one or more active agents are dissolved, dispersed or emulsified in a conventionally used vehicle (such as purified water, ethanol, or a mixture thereof). The liquid oral dosage form may optionally comprise a wetting agent, a dispersant, an emulsifier, a sweetener, a flavoring agent, a preservative, a buffer, or any other appropriate additive (s) .

The pharmaceutical composition in a dosage form for topical application may be in the form of an ointment, a gel, a cream, a plaster, a patch, a liniment, a spray, an inhalant, an aerosol, an eye drop, or a nasal drop, which may be prepared in accordance with a conventionally known preparation method, and may comprise one or more active agents.

The ointment may be prepared in accordance with a conventionally known preparation method. In particular, the ointment may be prepared by incorporating one or more active agents with an ointment base by grinding or melting. For preparing the ointment, any conventionally used ointment base may be used, which may comprise a higher fatty acid or fatty acid ester (such as adipic acid, myristic acid, palmitic acid, stearic acid, or oleic acid, or an ester thereof), a wax (such as beeswax, spermaceti, or ceresin), a surfactant (such as polyoxyethylene alkyl ether phosphate), a higher alcohol (such as cetanol, stearyl alcohol, or cetostearyl alcohol), a silicone oil (such as dimethylpolysiloxane), a hydrocarbon (such as a hydrophilic petrolatum, white petrolatum, purified lanolin, or liquid paraffin), a glycol (such as ethylene glycol, diethylene glycol, propylene glycol, polyethylene glycol, or macrogol), a vegetable oil (such as castor oil, olive oil, sesame oil, or turpentine oil), an animal oil (such as mink oil, egg-yolk oil, squalane, or squalene), water, an absorption enhancer, a skin protective agent, or a combination thereof. The ointment may additionally comprise a humectant, a preservative, a stabilizer, an antioxidant, a fragrance, or any other appropriate additive(s).

The pharmaceutical composition in a gel form may be prepared in accordance with a conventionally known preparation method. In particular, the gel may be prepared by incorporating one or more active agents with a gel base by melting. For preparing the gel, any conventionally used pharmaceutical gel base may be used, which may comprise a lower alcohol (such as ethanol, or isopropyl alcohol), a gelling agent (such as carboxymethyl cellulose, hydroxyethyl cellulose, hydroxypropyl cellulose, or ethyl cellulose), a neutralizing agent (such as triethanolamine, or diisopropanolamine), a surfactant (such as polyoxyethylene glycol monostearate), a gum, water, an absorption enhancer, a skin protective agent, or a combination thereof. The gel may additionally comprise a preservative, an antioxidant, a fragrance, or any other appropriate additive(s). The pharmaceutical composition in a cream form may be prepared in accordance with a conventionally known preparation method. In particular, the cream may be prepared by incorporating one or more active agents with a pharmaceutical cream base by melting or emulsification. For preparing the cream, any conventionally used pharmaceutical cream base may be used, which may comprise a higher fatty acid ester, a lower alcohol, a hydrocarbon, a polyhydric alcohol (such as propylene glycol, or 1,3-butylene glycol), a higher alcohol (such as 2-hexyldecanol, or cetanol), an emulsifier (such as a polyoxyethylene alkyl ether, or a fatty acid ester), water, an absorption enhancer, a skin protective agent, or a combination thereof. The cream may additionally comprise a preservative, an antioxidant, a fragrance, or any other appropriate additive(s).

The pharmaceutical composition in the form of a plaster may be prepared in accordance with a conventionally known preparation method. In particular, the plaster may be prepared by incorporating one or more active agents with a plaster base by melting, and applying the mixture onto a support. For preparing the plaster, any conventionally used pharmaceutical plaster base may be used, which may comprise a thickening agent (such as polyacrylic acid, polyvinyl pyrrolidone, gum arabic, starch, gelatin, or methyl cellulose), a humectant (such as urea, glycerol, or propylene glycol), a filler (such as kaolin, zinc oxide, talc, calcium, or magnesium), water, a solubilizing aid, a tackifier, a skin protective agent, or a combination thereof. The plaster may additionally comprise a preservative, an antioxidant, a fragrance, or any other appropriate additive(s).

The pharmaceutical composition in the form of a patch may be prepared in accordance with a conventionally known preparation method. In particular, the patch may be prepared by incorporating one or more active agents with a patch base by melting, and applying the mixture onto a support. For preparing the patch, any conventionally used pharmaceutical patch base may be used, which may comprise a polymer, an oil or fat, a higher fatty acid, a tackifier, a skin protective agent, or a combination thereof. The patch may additionally comprise a preservative, an antioxidant, a fragrance, or any other appropriate additive(s).

The pharmaceutical composition in the form of a liniment may be prepared in accordance with a conventionally known preparation method. In particular, the liniment may be prepared by dissolving, dispersing or emulsifying one or more active agents in a vehicle which may comprise water, an alcohol (such as ethanol, or polyethylene glycol), a higher fatty acid, glycerol, soap, an emulsifier, a dispersant, or a combination thereof. The liniment may additionally comprise a preservative, an antioxidant, a fragrance, or any other appropriate additive(s).

The pharmaceutical composition in the form of a spray, or an inhalant may comprise active agent(s), and optionally a stabilizing agent such as sodium hydrogen sulfite, or a tonicity agent or buffer, such as sodium chloride, sodium citrate or citric acid, in a vehicle.

The pharmaceutical composition in a dosage form for injection may be in the form of a solution, a suspension, or an emulsion, which comprises one or more active agents dissolved, dispersed or emulsified in a liquid for injection, or may be provided as a solid formulation comprising active agent(s) to be dissolved or dispersed in a liquid for injection at use. The liquid for injection for preparing an injectable preparation may comprise water for injection, physiological saline, a vegetable oil, propylene glycol, polyethylene glycol, an alcohol such as ethanol, or a combination thereof. The injectable preparation may additionally comprise a stabilizer, a solubilizing aid (such as glutamic acid, aspartic acid, or polysorbate 80^{®}), a dispersant, an emulsifier, an analgesic, a buffer, a preservative, or any other appropriate additive(s). For providing the injectable preparationas a sterilized preparation, it may be subjected to sterilization in the final step of its production, or produced aseptically throughout its production. The solid formulation for injection may be provided as a sterilized solid formulation or, in particular, lyophilized formulation, which may be dissolved in sterilized water for injection or any other appropriate sterilized liquid at use.

The pharmaceutical composition in a dosage form for inhalation may be in the form of an aerosol, an inhalable powder, or an inhalable liquid, or may be provided as a liquid concentrate which is to be dissolved or dispersed in water or any other appropriate vehicle to form an inhalable preparation at use. The preparation for inhalation may be prepared in accordance with a conventionally known preparation method. The inhalable liquid may optionally comprise a preservative (such as benzalkonium chloride, or paraben), a coloring agent, a buffer (such as sodium phosphate, or sodium acetate), a tonicity agent (such as sodium chloride, or concentrated glycerin), a thickening agent (such as a carboxyvinyl polymer), an absorption enhancer, or any other appropriate additive(s).

The inhalable powder may optionally comprise a lubricant (such as stearic acid, or a salt thereof), a binder (such as starch, or dextrin), a filler (such as lactose, or cellulose), a coloring agent, a preservative (such as benzalkonium chloride, or paraben), an absorption enhancer, or any other appropriate additive(s).

For administrating the inhalable liquid, a conventionally used spray device (such as an atomizer, or a nebulizer) may be used. The inhalable powder may be dispensed from a conventionally used powder inhalation device.

The pharmaceutical composition in the form of a spray may comprise active agent(s), and optionally a stabilizing agent (such as sodium hydrogen sulfite), or a tonicity agent or buffer (such as sodium chloride, sodium citrate, or citric acid) in a vehicle. The spray may be prepared in accordance with a preparation method as described, for example, in US 2,868,691, or US 3,095,355.

The pharmaceutical composition may be prepared in any other parenteral dosage form comprising one or more active agents by a conventionally known preparation method. Examples of other parenteral dosage forms include a rectal suppository, and a vaginal pessary.

In one embodiment, the pharmaceutical composition comprising a WT1 antigen peptide comprises one or more pharmaceutically acceptable carriers selected from the group consisting of trehalose, mannitol, methionine, citric acid, lactic acid, tartaric acid, acetic acid, trifluoroacetic acid and a pH adjusting agent.

In one embodiment, the pharmaceutical composition comprising an immunomodulator comprises one or more pharmaceutically acceptable carriers selected from the group consisting of mannitol, sodium citrate hydrate, sodium chloride, diethylenetriamine pentaacetic acid, polysorbate 80^{®} and a pH adjusting agent.

The WT1 antigen peptide and the immunomodulator may be administered in any route depending on the factors such as the disease to be treated, the condition of the subject, and the target site. The administration includes intravenous, intramuscular, intradermal, intraperitoneal, subcutaneous or intraspinal administration by injection or infusion, and other parenteral administration. The term "parenteral administration" as used herein is a common mode of administration by injection or infusion other than enteral administration and topical administration, and may be, but not limited to, intravenous, intramuscular, intraarterial, intrathecal, intracapsular, intraorbital, intracardiac, intradermal, intraperitoneal, intratracheal, subcutaneous, subepidermal, intraarticular, subcapsular, subarachnoidal, intraspinal , epidural or infrasternal injection or infusion. The WT1 antigen peptide may be administered in the lymphocyte therapy or DC (dendritic cell) therapy. The immunomodulator may be administered by transdermal administration or transmucosal administration such as intranasal, buccal, vaginal, rectal, or sublingual administration.

In addition to the immunomodulator, The WT1 antigen peptide may be combined with a non-drug therapy to treat or prevent cancer more effectively. The non-drug therapy may be surgery, radiotherapy, gene therapy, hyperthermia, cryotherapy, or laser burning therapy, and two or more non-drug therapies may be combined. For example, the pharmaceutical composition of the present invention, or a combination of the pharmaceutical composition and a concomitant drug, may be used before or after a non-drug therapy such as surgery, or before or after the treatment with two or three non-drug therapies, to prevent the development of resistance, prolong Disease-Free Survival, prevent metastasis or recurrence of cancer, or prolong survival.

The dosage amount, dosage form, and frequency of administration may be appropriately selected for a WT1 antigen peptide or an immunomodulator depending on the factors such as the disease to be treated, the condition of the subject, and the target site. The dosage amount of a WT1 antigen peptide per administration is generally 0.0001 mg-1000 mg, preferably 0.001 mg-1000 mg, more preferably 0.1 mg-10 mg. The dosage amount of an immunomodulator per kg body weight is generally 0.0001 mg-1000 mg, preferably 0.001 mg-1000 mg, more preferably 0.1 mg-10 mg.

The term "effective amount" as used herein means an amount of a WT1 antigen peptide or an immunomodulator, or a combination of two or more WT1 antigen peptides or immunomodulators, that completely or partially inhibits the progression of cancer, or that at least partially remits one or more symptoms of cancer. The effective amount may be a therapeutically or prophylactically effective amount. The effective amount is determined based on the factors such as the age or sex of the subject, the condition to be treated, the severity of the condition, and the desired result. An effective amount for a certain subject may be determined by any method known to the skilled person.

For reference only, it is disclosed that the present disclosure may be used for the treatment or prevention (including prevention of recurrence) of cancer expressing WT1 gene or cancer accompanied by an elevated expression level of WT1 gene. Thus, in one embodiment, the cancer may be hematologic cancer such as leukemia, myelodysplastic syndrome, multiple myeloma, and malignant lymphoma, and solid tumor such as gastric cancer, colorectal cancer, lung cancer, breast cancer, germ cell cancer, liver cancer, skin cancer, urinary bladder cancer, prostate cancer, uterine cancer, cervical cancer, ovarian cancer, glioblastoma multiforme, malignant melanoma, non-small cell lung cancer, renal cell carcinoma or brain tumor.

Further, as an immunomodulator is used in combination, the present invention is expected to increase the T cell activation threshold and activate responses to tumors within the host. Thus, in another embodiment, the cancer may be bone cancer, pancreatic cancer, cancer of the head or neck, cutaneous or intraocular malignant melanoma, rectal cancer, cancer of the anal region, testicular cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia such as acute myeloid leukemia, chronic myeloid leukemia, acute lymphoblastic leukemia, or chronic lymphocytic leukemia, childhood solid tumor, lymphocytic lymphoma, cancer of the kidney or ureter, carcinoma of the renal pelvis, central nervous system (CNS) tumor, primary CNS lymphoma, tumor angiogenesis, spinal tumor, brainstem glioma, pituitary adenoma, Kaposi's sarcoma, epidermoid cancer, squamous cell cancer, T-cell lymphoma, environmentally induced cancers including asbestos-induced cancer, and combinations of the cancers as described above. The present invention is also expected to be useful for the treatment of metastatic cancers, particularily metastatic cancers that express PD-L1 (Iwai et al. (2005) Int. Immunol. 17:133-144).

The term "mammal" as used herein encompasses human and non-human animals. The non-human animal may be, but not limited to, a mammal such as non-human primate, ovine, canine, feline, equine, and bovine. Among such mammals, a human subject, particularly a human subject who needs potentiation of immune response is preferred. Thus, the present invention is particularly suitable to treat a human subject suffering from a disease that is expected to be treated by promotion of T cell-mediated immune response.

The WTl antigen peptide and the immunomodulator and a WT1 helper peptide as disclosed herein may be comprised in separate formulations or in a single formulation. Thus, the pharmaceutical composition of the present invention may be a pharmaceutical composition comprising a WTl antigen peptide that is used in combination with an immunomodulator, a pharmaceutical composition comprising an immunomodulator that is used in combination with a WTl antigen peptide, or a pharmaceutical composition comprising a WTl antigen peptide and an immunomodulator (that is, a combination preparation). The pharmaceutical composition of the present invention may be provided in the form of a kit. For example, the kit may comprise a pharmaceutical composition comprising a WT1 antigen peptide and a pharmaceutical composition comprising an immunomodulator. The pharmaceutical composition and the kit of the present invention may be provided with a package insert showing the information such as dosage amount or dosage regimen for the combined use of a WT1 antigen peptid and an immunomodulator, package, or instruction manual. In one embodiment, the pharmaceutical composition and the kit of the present invention may be provided as a medical product for treating cancer.

As disclosed for reference only, the WT1 antigen peptide and the immunomodulator may be administered simultaneously or separately. Also, the WT1 antigen peptide and the immunomodulator may be administered simultaneously or separately with a further concomitant drug. The expression "administered simultaneously" as used herein mean that the active ingredients are administered in the same dosage regimen, and the active ingredients may be comprised in a single formulation or in separate formulations. When the active ingredients are comprised in separate formulations, the formulations may be administered in a single dose, or sequentially. The expression "administered separately" means that the active ingredients are administered in different dosage regimens. Thus, the administration of an active ingredient is followed by the administration of a different active ingredient after an interval, and there is no limitation in the order of administration of the active ingredients and the length of the interval. The frequency of administration may be the same or different between the active ingredients. For example, the frequency of administration of an active ingredient may be once a day while that of a different active ingredient may be two or more times per day.

When the active ingredients are comprised in a single formulation, the ratio of the active ingredients may be determined appropriately based on the factor(s) such as the subject, route of administration, disease to be treated, conditions of the subject, or combination thereof. For example, when the subject is human, the ratio of a WT1 antigen peptide to an immunomodulator or a concomitant drug may be 1 parts by weight to 0.01-100 parts by weight.

The pharmaceutical composition of the present invention may be used in combination with a further agent such as an antiemetic agent, sleep-inducing agent, or anticonvulsant to reduce side effects

WT1 antigen peptides increase tumor-reactive CTLs in tumor. Thus, when a WT1 antigen peptide is combined with an immunomodulator, the dosage amount of the immunomodulator, and thus adverse events, may be reduced. Therefore, the combination of a WT1 antigen peptide and an immunomodulator would provide more effective therapies with higher safety.

### EXAMPLES

The present invention is specifically explained hereinafter by referring to Examples, but not limited by the same in any sense.

### Example 1:

### Effect of combination use of immune checkpoint inhibitor on induction of tumor antigen peptide-specific cytotoxic T cells from human peripheral blood mononuclear cells

Cryopreserved peripheral blood mononuclear cells (PBMCs) of an HLA-A*02:01 positive adult (C.T.L) were reconstituted, suspended in a culture medium and seeded on a U-bottom 96 well plate at 1.5 × 10⁵ cells/well. The cells were cultured with AIM V medium (Life Technologies) supplemented with 5% human serum (Lonza) and 1% MEM Non-Essential Amino Acids Solution (100x) (Life Technologies) at 37 C°, 5% CO₂. To the PBMCs, an anti-human PD-1 antibody (Group A) or an anti-human Pb-L1 antibody (Group B), or none of the anti-human PD-1 and PD-L1 antibodies (Group C, control group) was added. Twenty four samples (24 wells) were included in each group. The anti-human PD-1 antibody was clone EH12.2H7 (BioLegend), and the anti-human PD-L1 antibody was 29E.243 (BioLegend). Mouse IgG1, κ (BioLegend) and Mouse IgG2b, κ (BioLegend) were used as isotype controls of the anti-human PD-1 and PD-L1 antibodies, respectively.

In Group A, the anti-human PD-1 antibody and the isotype control Mouse IgG2b, κ were added at a final concentration of 10 µg/ml, respectively. In Group B, the anti-human PD-L1 antibody and the isotype control Mouse IgG1, κ were added at a final concentration of 10 µg/ml, respectively. In Group C, the isotype controls Mouse IgG1, κ and Mouse IgG2b, κ were added at a final concentration of 10 µg/ml, respectively. At Day 1 of culture (one day after the start of culture), a WT1 killer peptide (compound of formula (3)) and a WT1 helper peptide (SEQ ID NO: 18) were added to the cultured cells at a final concentration of 20 µg/ml, respectively. At Day 2 of culture, human IL-2 (Shionogi & Co., Ltd.) was added at a final concentration of 50 U/ml. At Day 5 and Day 9 of culture, a half of the culture medium was replaced with a culture medium containing 100 U/ml human IL-2. At Day 13 of culture, the cells of 24 samples in each group were collected and stained with a combination of a PE-labeled HLA tetramer (MBL) against killer peptide A (SEQ ID NO: 2) and an FITC-labeled anti-CD8 antibody (BD), or a combination of a PE-labeled HLA tetramer (MBL) against killer peptide B (SEQ ID NO: 8) and an FITC-labeled anti-CD8 antibody (BD), and analyzed for killer peptide-specific CTLs with a flow cytometer MACSQuant Analyzer (Miltenyi Biotec).

In Group C, to which none of the anti-human PD-1 antibody nor the anti-human PD-L1 antibody were added, CTLs specific to the killer peptide A or B were not detected in any of the 24 samples. In Group A, to which the anti-human PD-1 antibody was added, CTLs specific to the killer peptide B were detected in one of the 24 samples (Fig. 1) . In Group B, to which the anti-human PD-L1 antibody was added, CTLs specific to the killer peptide A were detected in one of the 24 samples (Fig. 2) and CTLs specific to the killer peptide B were detected in two of the 24 samples (Fig. 3). The results are shown in Figs .1-3. These results demonstrate that the immune checkpoint inhibitors, anti-human PD-1 and anti-human PD-L1 antibodies, enhance the CTL induction from PBMCs stimulated with a WT1 peptide in vitro.

### Example 2

### Effect of immune checkpoint inhibitor on immune response to WT1 antigen peptide of WT1 antigen peptide-specific CTLs

Cryopreserved peripheral blood mononuclear cells (PBMCs) of an HLA-A*02:01 positive adult (C.T.L) were reconstituted, suspended in a culture medium and seeded on a U-bottom 96 well plate. The cells were cultured with AIM V medium (Life Technologies) supplemented with 5% human serum (Lonza) and 1% MEM Non-Essential Amino Acids Solution (100×) (Life Technologies) and containing 20 µg/ml killer peptide B and 100 U/ml human IL-2 (Shionogi & Co., Ltd.). At Day 3, Day 7 and Day 11 of culture, a half of the culture medium was replaced with a fresh culture medium. At Day 13 of culture, the cells of each well were collected and stained with a PE-labeled HLA tetramer (MBL) against the killer peptide B and an FITC-labeled anti-CD8 antibody (BD), and analyzed for killer peptide B-specific CTLs with a flow cytometer MACSQuant Analyzer (Miltenyi Biotec). The cells collected from the wells in which killer peptide B-specific CTLs were detected were mixed to provide a WT1 antigen peptide-specific CTL line. The WT1 antigen peptide-specific CTL line was cryopreserved with a cryopreservation medium CELLBANKER (Nippon Zenyaku Kogyo Co.,Ltd.).

The cryopreserved WT1 antigen peptide-specific CTL line was reconstituted and cultured overnight with a culture medium containing 100 U/ml human IL-2 (Shionogi & Co., Ltd.). The cells were collected, washed with a culture medium, and cultured for 2 hours with the anti-human PD-1 antibody (EH12.2H7) (Group A) or the anti-human PD-L1 antibody (29E.243) (Group B), or without the anti-human PD-1 and anti-human PD-L1 antibodies (Group C, control group). In Group A, the anti-human PD-1 antibody and the isotype control Mouse IgG2b, κ were added at a final concentration of 10 µg/ml, respectively. In Group B, the anti-human PD-L1 antibody and the isotype control Mouse IgG1, κ were added at a final concentration of 10 µg/ml, respectively. In Group C, the isotype controls Mouse IgG1, κ and Mouse IgG2b, κ were added at a final concentration of 10 µg/ml, respectively. The antibodies were those used in Example 1. Peptide-specific cellular response was measured using IFN-γ ELISPOT Set (BD). The cells were placed in ELISPOT plates and cultured for 18 hours with or without killer peptide B (SEQ ID NO: 8) at a final concentration of 40 µg/ml. In each group, two samples were prepared. Then, the plates were processed according to the manufacture's protocol and developed with AGE Substrate Set (BD). The spots were counted with ELISPOT analyzer (C.T.L). For each of Groups A, B and C, the response was calculated by subtracting the average number of spots from the samples without peptide addition from the average number of spots from the samples with peptide addition.

Compared to the control Group C, Groups A and B, which were treated with the anti-human PD-1 antibody and the anti-human PD-L1 antibody, respectively, developed 1.7 times and 1.4 times more spots than the control Group C, respectively. The results are shown in Fig. 4. These results demonstrate that the immune checkpoint inhibitors enhance the response to WT1 antigen peptides of WT1 antigen peptide-specific CTLs.

### Example 3

### Expression change of immune checkpoint molecules in mouse spleen cells that received cocktail vaccine of WT1 killer peptide and WT1 helper peptide

The HLA-A*02:01 transgenic mouse (C57BL/6CrHLA-A2.1DR1) used in this example is deficient in mouse MHC molecules but expresses a chimeric HLA molecule of human MHC HLA-A02:01 and mouse MHC H-2D^{b} as well as HLA-DRB1˙01:01 (Eur J Immunol. 2004; 34: 3060-9). In this mouse, CTLs may be induced with human HLA-A*02:01-binding peptides. Also, helper T cells may be induced with human HLA-DRB1˙01:01-binding peptides in this mouse and thus enhancement of CTL induction with helper peptides may be evaluated.

A composition comprising a WT1 killer peptide (compound of formula (3)) and a WT1 helper peptide (SEQ ID NO: 18) was mixed with an equal volume of an incomplete Freund's adjuvant, Montanide ISA51VG, to provide an emulsion. The cocktail vaccine thus obtained (referred to as " vaccine" herein after) was intradermally administered to the root of tail of the HLA-A*02:01 transgenic mouse two times with one week interval (1 mg WT1 killer peptide and 0.75 mg WT1 helper peptide per immunization were administered per mouse). One week after the final administration, the vaccinated and unvaccinated mice were euthanized with CO₂ gas and spleens were removed from the mice to prepare spleen cells. The spleen cells were stained with a FITC-labeled anti-CD8 antibody (BD Pharmingen), PE-labeled anti-CD4 antibody (BD Pharmingen), PE-labeled HLA tetramer against a WT1 killer peptide (SEQ ID NO: 8) (MBL), APC-labeled anti-PD-1 antibody (BD Pharmingen, clone J43), APC-labeled isotype control antibody (BD Pharmingen, Hamster IgG2, x), APC-labeled anti-PD-L1 antibody (BioLegends, clone 10F.9G2), and APC-labeled isotype control antibody (eBioscience, Rat IgG2b, x) and analyzed with a flow cytometer MACSQuant Analyzer (Miltenyi Biotec).

The results are shown in Figs. 5-10. Figs. 5 and 8 show expression of PD-1 (Fig. 5) and PD-L1 (Fig. 8) in CD8⁺, tetramer⁺ fraction of spleen cells from a vaccinated mouse (dashed dotted line); CD8⁺, tetramer- fraction of spleen cells from a vaccinated mouse (dashed line); and CD8⁺, tetramer' fraction of spleen cells from an unvaccinated mouse (solid line). The dotted line indicates the results of staining with the isotype control. Figs. 6, 7, 9 and 10 show expression of PD-1 (Figs. 6 and 7) and PD-L1 (Figs. 9 and 10) in CD4⁺ T cells (Figs. 6 and 9, dashed line) and CD4⁻, CD8⁻ cells (Figs. 7 and 10, dashed line) from spleen cells of a vaccinated mouse; and CD4⁺ T cells (Figs. 6 and 9, solid line) and CD4⁻, CD8⁻ cells (Figs. 7 and 10, solid line) from spleen cells of a unvaccinated mouse. The dotted line indicates the results of staining with the isotype control. As shown in the figures, the vaccine administration induced a high PD-1 expression in CD8⁺ T cells, in particular CD8⁺, WT1 tetramer⁺ T cells (Figs. 5-7). PD-L1 was expressed in any type of cells in vaccinated and unvaccinated mice and upregulated in CD4⁺ T cells with the vaccine administration (Figs. 8-10). These results demonstrate that the vaccine administration induces expression of PD-1 and PD-L1 in WT1-specific CD8⁺ T cells and CD4⁺ T cells, respectively.

### Example 4

### Effect of immune checkpoint inhibitor on immune response to tumor cells of WT1 antigen peptide-specific CTLs

As described in Example 3, the composition comprising the WT1 killer peptide and the WT1 helper peptide was emulsified with an equal amount of Montanide and intradermally administered to the root of tail of the HLA-A˙02:01 transgenic mouse (1 mg WT1 killer peptide and 0.75 mg WT1 helper peptide per immunization were administered per mouse) . One week after the final administration, the mouse was euthanized with CO₂ gas and the spleen was removed from the mouse to prepare a suspension of spleen cells using Complete T-cell medium (referred to as "CTM" hereinafter). A WT1 killer peptide (SEQ ID NO: 2) was dissolved with DMSO at 40 mg/mL and diluted with CTM to 500 µg/mL. The peptide solution was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10. To the suspension of spleen cells thus obtained, an anti-PD-l antibody (Bio X cel, clone RMP1-14) or an isotype control antibody (Bio X cell, Rat IgGa, x) was added at 10 µg/mL, and the cells were cultured at 37°C, 5% CO₂ for five days. The cultured spleen cells were washed with 10%FBS RPMI1640 and used as responder cells. Then, the WT1 killer peptide (SEQ ID NO: 2) was dissolved with DMSO at 40 mg/mL and further diluted with 10%FBS RPMI1640 to 100 µg/mL. EL4HHD cells (J Exp Med.1997; 185: 2043-51) were suspended with the peptide-containing RPMI1640 medium or an RPMI1640 medium not containing the peptide and stood for about one hour at 37°C, 5% CO₂. EL4HHD was a cell line established by stably expressing a chimeric HLA molecule called HHD, which has α3 domain of human HLA-A*02:01 instead of that of mouse MHC class I H-2Db, in a mouse lymphoma cell line EL4 S3- Rob (Eur J Immunol.1990; 20: 171-7). After excess peptides were washed out with 10%FBS RPMI1640, the cells were used as stimulator cells. The stimulator EL4HHD cells (1×10⁵ cells/well) and the responder spleen cells cultured for five days (5×10⁵ cells/well) were mixed on U-bottom 96 well plates and cultured for 24 hours at 37°C, 5% CO₂. The concentration of mouse IFN-γ in the culture supernatant was measured with an ELISA kit (R&D Systems).

The IFN-γ production was observed only in the responder cells mixed with the stimulator cells pretreated with the peptide. Also, the IFN-γ production of the responder cells cultured with the anti-PD-1 antibody was higher than that of the responder cells cultured with the isotype control antibody. No IFN-γ production was observed in the responder cells mixed with the stimulator cells untreated with the peptide. Thus, it was suggested WT1 antigen peptide-specific T cells produced IFN-γ. These results demonstrate that WT1 antigen peptide-specific T cells induced by the vaccine administration were activated with the treatment of the anti-PD-1 antibody and thus the immune response to tumor cells was enhanced.

The above Examples demonstrate that WTl-specific T cells are more efficiently induced when a WT1 antigen peptide is combined with an anti-PD-1 or PD-L1 antibody; the expression of immune checkpoint molecules is upregulated in T cells induced by the vaccine administration; and the activity of peptide-specific T cells induced by a WT1 antigen peptide is increased with the treatment of an anti-PD-1 or PD-L1 antibody. Accordingly, the combination therapy of a WT1 antigen peptide and an immune checkpoint inhibitor synergistically increases the therapeutic effects of respective agents and would contribute the improvement of therapeutic effects on cancers and the improvement of QOL.

### Example 5

### Effect of combined agent on immune response to WT1 antigen peptide or tumor cells of WT1 antigen peptide-specific CTLs

A composition comprising a WT1 killer peptide (compound of formula (3)) and a WT1 helper peptide (SEQ ID NO: 18) was emulsified with an equal volume of Montanide and intradermally administered to the root of tail of the HLA-A˙02:01 transgenic mouse (0.5 mg WT1 killer peptide and 0.375 mg WT1 helper peptide were administered per mouse). One week after the administration, the mouse was euthanized with CO₂ gas and the spleen was removed from the mouse to prepare a suspension of spleen cells using CTM. A WT1 killer peptide (SEQ ID NO: 2) was dissolved with DMSO at 40 mg/mL. The WT1 killer peptide solution was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10 and seeded on a U-bottom 96 well plate at 3.85×10⁵ cells/well. LLC-HHD-WT1 tumor cells, established by stably expressing HHD and the WT1 killer peptide (SEQ ID NO: 2) in a mouse Lewis lung carcinoma cell line LLC, were used as tumor cells. X-ray irradiated (50Gy) LLC-HHβ-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the spleen cells at 5×10⁴ cells/well or 3.5×10⁴ cells/well and cultured for about three days at 37°C, 5% CO₂ with CTM (medium), an isotype control antibody, an immune checkpoint inhibitor, a costimulatory molecule agonist antibody, a TLR agonist, or a β-catenin inhibitor. The immune checkpoint inhibitor was an anti-PD-1 (BioLegend, clone 29F.1A12), anti-TIM-3 (BioLegend, clone RMT3-23), anti-CD160 (eBioscience, clone eBioCNX46-3), anti-LAG-3 (BioLegend, clone C9B7W), anti-BTLA (BioLegend, clone 6A6), anit-PD-L1 (eBiosciecne, clone MIH5), anti-HVEM (BioLegend, clone HMHV-1B18), anti-VISTA (BioLegend, clone MH5A), or anti-PVR (Hycult Biotech, clone 3F1) antibody at a final concentration of 10 µg/mL. The costimulatory molecule agonist antibody was an anti-4-7.BB (Bio X Cell, clone LOB12.3), anti-OX-40 (Bio X Cell, clone OX-86), anti-GITR (BioLegend, clone DTA-1), or anti-CD-40 (BioLegend, clone 1C10) antibody at a final concentration of 30 µg/mL. The isotype control antibody was Rat IgG2aκ (BD Pharmingen) for the anti-PD-1, anti-TIM-3, anti-CD160, and anti-CD-40 antibodies; Rat IgG2a (Bio X Cell) for the anit-PD-L1 and anti-PVR antibodies; Armenian Hamster IgG (eBioscience) for the anti-BTLA, anti-HVEM, and anti-VISTA antibodies; Rat IgG1κ (eBiosciecne) for the anti-LAG-3, anti-4-1BB and anti-OX-40 antibodies; and Rat IgG2b (Bio X Cell) for the anti-GITR antibody. The TLR agonist was a TLR3 agonist, PolyI:C HMW (GE Healthcare, final concentration of 30 µg/mL) or PolyI:C LMW (GeneDesign, final concentration of 30 µg/mL); a TLR7 agonist, Imiquimod (final concentration of 10 µg/mL); a TLR7/8 agonist, R848 (final concentration of 1 µmol/L); or a TLR9 agonist, CpG-DDN-D19 (final concentration of 1 µmol/L), CpG-ODN-1826 (final concentration of 1 µmol/L) or CpG-ODN-C583 (final concentration of 1 µmol/L). The β-catenin inhibitor was XAV939 at a final concentration of 5 µmol/L. The concentration of mouse IFN-γ in the culture supernatant was measured with an ELISA kit (R&D Systems).

The results are shown in Figs. 12-15. The IFN-γ production in the culture with the immune checkpoint inhibitor (Figs. 12A-12I) or the costimulatory molecule agonist antibody (Figs. 13A-13D) was higher than that in the culture with the corresponding isotype control antibody. Also, the IFN-γ production in the culture with the TLR agonist (Figs. 14A-14D) or the β-catenin inhibitor (Fig. 15) was higher than that in the culture with the CTM medium. These results demonstrate that immune checkpoint inhibitors, costimulatory molecule agonists, TLR agonists, and β-catenin inhibitors enhance the response to WT1 antigen peptides or tumor cells of WT1 antigen peptide-specific CTLs.

### Example 6

### Effect of combined agent on immunoreactivity of WT1 antigen peptide-specific CTLs from tumor-bearing animal

EL4-A29/Kb-WT1 tumor cells had been established by stably expressing HLA-A2402/Kb, a chimeric HLA molecule having α3 domain of mouse MHC class I H-2Kb instead of that of human HLA-A˙24:02, and a WT1 killer peptide (SEQ ID NO: 4) in mouse lymphoma cell line EL4. The EL4-A24/Kb-WT1 tumor cells were suspended in Hanks' Balanced Salt Solution and intradermally transplanted to the ventral region of the HLA-A˙24:02 transgenic mouse (3×10⁵ cells or 5×10⁵ cells per mouse). One day after and eight days after the transplantation, a composition comprising a WT1 killer peptide (compound of formula (3)) and a WT1 helper peptide (SEQ ID NO: 18) was emulsified with an equal volume of Montanide and intradermally administered to the HLA-A˙24:02 transgenic mouse in two sites, the roots of forelimb and hindlimb (1 mg WT1 killer peptide and 0.75 mg WT1 helper peptide were administered per administration per mouse). Fifteen days after the transplantation, the mouse was euthanized with CO₂ gas and the spleen was removed from the mouse to prepare a suspension of spleen cells using CTM. The spleen cells were seeded on U-bottom 96 well plate at 3.85×10⁵ cells/well and cultured for about three days at 37°C, 5% CO₂ with CTM, an isotype control antibody, an immune checkpoint inhibitor, or a costimulatory molecule agonist antibody. The immune checkpoint inhibitor was an anti-PD-1 (BioLegend, clone 29F.1A12), anti-CTLA-4 (BioLegend, clone UC10-4B9), or anti-TIGIT (Bio X Cell, clone 1G9) antibody at a final concentration of 30 µg/mL. The costimulatory molecule agonist antibody was an anti-ICOS antibody (BioLegend, clone C398.4A) at a final concentration of 30 µg/mL. The isotype control antibody was Rat IgG2ax (BD Pharmingen) for the anti-PD-1 antibody; Armenian Hamster IgG (eBioscience) for the anti-CTLA-4 and anti-ICOS antibodies; and Mouse IgG1 (Bio X Cell) for the anti-TIGIT antibody. The concentration of mouse IFN-γ in the culture supernatant was measured with an ELISA kit (R&D Systems).

The results are shown in Figs. 16-17. The IFN-γ production in the culture with the immune checkpoint inhibitor (Figs. 16A-16C) or the costimulatory molecule agonist antibody (Fig. 17) was higher than that in the culture with the corresponding isotype control antibody. These results demonstrate that immune checkpoint inhibitors and costimulatory molecule agonists enhance the immunoreactivity of WT1 antigen peptide-specific CTLs from tumor-bearing animals.

### Example 7

### Effect of combined agent on immune response to WT1 antigen peptide or tumor cells of WT1 antigen peptide-specific CTLs

A composition comprising a WT1 killer peptide (SEQ ID NO: 2) and a WT1 helper peptide (SEQ ID NO: 11) was emulsified with an equal volume of Montanide and intradermally administered to the root of tail of the HLA-A˙02:01 transgenic mouse (0.3 mg WT1 killer peptide and 0.3 mg WT1 helper peptide were administered per mouse). One week after the administration, the mouse was euthanized with CO₂ gas and the spleen was removed from the mouse to prepare a suspension of spleen cells using CTM. A WT1 killer peptide (SEQ ID NO: 2) was dissolved with DMSO at 40 mg/mL. The WT1 killer peptide solution was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10 and seeded on a U-bottom 96 well plate at 3.85×10⁵ cells/well. LLC-HHD-WT1 tumor cells were used as tumor cells. X-ray irradiated (50Gy) LLC-HHD-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the spleen cells at 3.5×10⁴ cells/well and cultured for about three days at 37°C, 5% CO₂ with CTM, an isotype control antibody, an immune checkpoint inhibitor, or a costimulatory molecule agonist antibody. The immune checkpoint inhibitor was an anti-PD-1 (BioLegend, clone 29F.1A12), anti-B7-H4 (BioLegend, clone HMH4-5G1) or anit-PD-L1 (eBiosciecne, clone MIH5) antibody at a final concentration of 30 µg/mL. The costimulatory molecule agonist antibody was an anti-4-1BB (Bio X Cell, clone LOB12.3) or anti-OX-40 (Bio X Cell, clone OX-86) antibody at a final concentration of 30 µg/mL. The isotype control antibody was Rat IgG2aκ (BD Pharmingen) for the anti-PD-1 antibody; Rat IgG2a (Bio X Cell) for the anit-PD-L1 antibody; Armenian Hamster IgG (eBioscience) for the anti-B7-H4 antibody; and Rat IgG1κ (eBiosciecne) for the anti-4-1BB and anti-OX-40 antibodies. The concentration of mouse IFN-γ in the culture supernatant was measured with an ELISA kit (R&D Systems).

The results are shown in Figs. 18-19. The IFN-γ production in the culture with the immune checkpoint inhibitor (Figs. 18A-18C) or the costimulatory molecule agonist antibody (Figs. 19A-19B) was higher than that in the culture with the corresponding isotype control antibody. These results demonstrate that immune checkpoint inhibitors and costimulatory molecule agonists enhance the response to WT1 antigen peptides or tumor cells of WT1 antigen peptide-specific CTLs.

### Example 8

### Effect of combined agent on immune response to WT1 antigen peptide or tumor cells of WT1 antigen peptide-specific CTLs

A composition comprising a WT1 killer peptide (SEQ ID NO: 2) and a WT1 helper peptide (SEQ ID NO: 14) was emulsified with an equal volume of Montanide and intradermally administered to the root of tail of the HLA-A*02:01 transgenic mouse (0.3 mg WT1 killer peptide and 0.3 mg WT1 helper peptide were administered per mouse). One week after the administration, the mouse was euthanized with CO₂ gas and the spleen was removed from the mouse to prepare a suspension of spleen cells using CTM. A WT1 killer peptide (SEQ ID NO: 2) was dissolved with DMSO at 40 mg/mL. The WT1 killer peptide solution was added to a part of the spleen cells at a final concentration of 100 ug/mL, and the cells were stood for about one hour at 37°C, 5% CO₂. After excess peptides were washed out with CTM, the peptide-pulsed spleen cells and unpulsed spleen cells were mixed at a ratio of 1:10 and seeded on a U-bottom 96 well plate at 3.85×10⁵ cells/well. LLC-HHD-WT1 tumor cells were used as tumor cells. X-ray irradiated (50Gy) LLC-HHD-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the spleen cells at 3.5×10⁴ cells/well and cultured for about three days at 37°C, 5% CO₂ with CTM, an isotype control antibody, an immune checkpoint inhibitor, or a costimulatory molecule agonist antibody. The immune checkpoint inhibitor was an anti-PD-1 (BioLegend, clone 29F.1A12), anti-B7-H4 (BioLegend, clone HMH4-5G1) or anit-PD-L1 (eBiosciecne, clone MIH5) antibody at a final concentration of 30 µg/mL. The costimulatory molecule agonist antibody was an anti-4-1B8 (Bio X Cell, clone LOB12.3) or anti-OX-40 (Bio X Cell, clone OX-86) antibody at a final concentration of 30 µg/mL. The isotype control antibody was Rat IgG2aκ (BD Pharmingen) for the anti-PD-1 antibody; Rat IgG2a (Bio X Cell) for the anit-PD-L1 antibody; Armenian Hamster IgG (eBioscience) for the anti-B7-H4 antibody and Rat IgG1κ (eBiosciecne) for the anti-4-1BB and anti-OX-40 antibodies. The concentration of mouse IFN-γ in the culture supernatant was measured with an ELISA kit (R&D Systems).

The results are shown in Figs. 20-21. The IFN-γ production in the culture with the immune checkpoint inhibitor (Figs. 20A-20C) or the costimulatory molecule agonist antibody (Figs. 21A-21B) was higher than that in the culture with the corresponding isotype control antibody. These results demonstrate that immune checkpoint inhibitors and costimulatory molecule agonists enhance the response to WT1 antigen peptides or tumor cells of WT1 antigen peptide-specific CTLs.

### Example 9

### Effect of combined agent on immune response to WT1 antigen peptide or tumor cells of WT1 antigen peptide-specific CTLs

A composition comprising a WT1 killer peptide (SEQ ID NO: 26) and a WT1 helper peptide (SEQ ID NO: 37) was emulsified with an equal volume of Montanide and intradermally administered to the root of tail of the HLA-A˙02:01 transgenic mouse (0.234 mg WT1 killer peptide and 0.234 mg WT1 helper peptide were administered per mouse). One week after the administration, the mouse was euthanized with CO₂ gas and the spleen was removed from the mouse to prepare a suspension of spleen cells using CTM. A WT1 killer peptide (SEQ ID NO: 26) was dissolved with DMSO at 40 mg/mL. The WT1 killer peptide solution was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10 and seeded on a U-bottom 96 well plate at 3.85×10⁵ cells/well. LLC-HHD-NT1 tumor cells were used as tumor cells. X-ray irradiated (50Gy) LLC-HHD-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the spleen cells at 3.5×10⁴ cells/well and cultured for about three days at 37°C, 5% CO₂ with CTM, an isotype control antibody, or an immune checkpoint inhibitor. The immune checkpoint inhibitor was an anti-PD-1 (BioLegend, clone 29F.1A12) or anit-PD-L1 (eBiosciecne, clone MIH5) antibody at a final concentration of 30 µg/mL. The isotype control antibody was Rat IgG2aκ (BD Pharmingen) for the anti-PD-1 antibody and Rat IgG2a (Bio X Cell) for the anit-PD-L1 antibody. The concentration of mouse IFN-γ in the culture supernatant was measured with an ELISA kit (R&D Systems).

The results are shown in Fig. 22. The IFN-γ production in the culture with the immune checkpoint inhibitor was higher than that in the culture with the corresponding isotype control antibody. These results demonstrate that immune checkpoint inhibitors enhance the response to WT1 antigen peptides or tumor cells of WT1 antigen peptide-specific CTLs.

### Example 10

### Effect of combined agent on immune response to WT1 antigen peptide or tumor cells of WT1 antigen peptide-specific CTLs

A composition comprising a WT1 killer peptide (SEQ ID NO: 5) and a WT1 helper peptide (SEQ ID NO: 11) was emulsified with an equal volume of Montanide and intradermally administered to the root of tail of the HLA-A*02:01 transgenic mouse (0.3 mg WT1 killer peptide and 0.3 mg WT1 helper peptide were administered per mouse). One week after the administration, the mouse was euthanized with CO₂ gas and the spleen was removed from the mouse to prepare a suspension of spleen cells using CTM. The WT1 killer peptide (SEQ ID NO: 5) was dissolved with DMSO at 40 mg/mL. The WT1 killer peptide solution was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10 and seeded on a U-bottom 96 well plate at 3.85×10⁵ cells/well. After excess peptides were washed out with CTM, the peptide-pulsed spleen cells and unpulsed spleen cells were mixed at a ratio of 1:10 and seeded on a U-bottom 96 well plate at 3.85×10⁵ cells/well. LLC-HHD-WT1 tumor cells were used as tumor cells. X-ray irradiated (50Gy) LLC-HHD-WT1 tumor cells were cultured in the presence of a mouse recombinant IFN-γ (100 ng/mL) for about two days and washed with CTM. The LLC-HHD--WT1 tumor cells were seeded on the U-bottom 96 well plate containing the spleen cells at 3.5×10⁴ cells/well and cultured for about three days at 37°C, 5% CO₂ with CTM, an isotype control antibody, an immune checkpoint inhibitor, or a costimulatory molecule agonist antibody. The immune checkpoint inhibitor was an anti-PD-1 (BioLegend, clone 29F.1A12), anti-B7-H4 (BioLegend, clone HMH4-5G1) or anit-PD-L1 (eBiosciecne, clone MIH5) antibody at a final concentration of 30 µg/mL. The costimulatory molecule agonist antibody was an anti-4-1BB (Bio X Cell, clone LOB12.3), anti-OX-40 (Bio X Cell, clone OX-86), or anti-GITR (BioLegend, clone DTA-l) antibody at a final concentration of 30 µg/mL. The isotype control antibody was Rat IgG2ax (BD Pharmingen) for the anti-PD-1 antibody; Rat IgG2a (Bio X Cell) for the anit-PD-L1 antibody; Armenian Hamster IgG (eBioscience) for the anti-B7-H4 antibody; Rat IgG1k (eBiosciecne) for the anti-4-1BB and anti-OX-40 antibodies; and Rat IgG2b (Bio X Cell) for the anti-GITR antibody. The concentration of mouse IFN-γ in the culture supernatant was measured with an ELISA kit (R&D Systems).

The results are shown in Figs. 23-24. The IFN-γ production in the culture with the immune checkpoint inhibitor (Figs. 23A-23C) or the costimulatory molecule agonist antibody (Figs. 24A-24C) was higher than that in the culture with the corresponding isotype control antibody. These results demonstrate that immune checkpoint inhibitors and costimulatory molecule agonists enhance the response to WT1 antigen peptides or tumor cells of WT1 antigen peptide-specific CTLs.

### Example 11

### Enhancement with immune checkpoint inhibitor of in vivo suppression of tumor proliferation by vaccine

The HLA-A*24:02 transgenic mouse (C57BL/6CrHLA-A24/Kb) used in this example expresses a chimeric HLA molecule of human MHC HLA-A24:01 and mouse MHC H-2Kb (Int. J. Cancer 2002; 100: 565-570. In this mouse, CTLs may be induced with human HLA-A*24:02-binding peptides.

EL4-A24/Kb-WT1 tumor cells were suspended in Hanks' Balanced Salt Solution and intradermally transplanted to the ventral region of HLA-A*24:02 transgenic mice (3×10⁵ cells per mouse). The mice received a vehicle (Montanide and phosphate buffered saline) (Group a); Montanide and an anti-PD-1 antibody (Group b); a vaccine and an isotype control antibody (Group c); or a vaccine and an anti-PD-1 antibody (Group d). Five mice per group were used. One day after and eight days after the tumor transplantation, for the mice of Groups a and b, a composition comprising water for injection was emulsified with an equal volume of Montanide and intradermally administered in two sites, the roots of forelimb and hindlimb (0.1 mL per administration per mouse). For the mice of Groups c and d, a composition comprising a WT1 killer peptide (compound of formula (3)) and a WT1 helper peptide (SEQ ID NO: 18) was emulsified with an equal volume of Montanide and intradermally administered to the HLA-A*24:02 transgenic mouse in two sites, the roots of forelimb and hindlimb (0.5 mg WT1 killer peptide and 0.375 mg WT1 helper peptide were administered per administration per mouse). One, four, eight and eleven days after the tumor transplantation, phosphate buffered saline was intraperitoneally administered to the mice of Group a (0.1 mL per administration per mouse). To the mice of Groups b and d, an anti-PD-1 antibody (Bio X Cell, clone RMP1-14) was intraperitoneally administered (0.2 mg per administration per mouse). To the mice of Group c, an isotype control antibody (Bio X Cell, rat IgG2a) was intraperitoneally administered (0.2 mg per administration per mouse). The tumor size was measured 4, 7, 10, 11, 17 and 21 days after the tumor transplantation and the tumor volume and the ratio of animals showing tumor rejection was calculated.

The results are shown in Fig. 25. The anti-PD-1 antibody (Group b) or the WT1 vaccine (Group c) significantly suppressed the tumor proliferation compared to the vehicle (Group a) (parametric Dunnett's multiple test, *: p<0.05). When the anti-PD-1 antibody was used in combination with the WT1 vaccine, the suppression of tumor proliferation was more significant (Group D, **: p<0.01). As shown in Table 1, 11 days after the tumor transplantation, no animal showed tumor rejection in the group received the anti-PD-1 antibody, but the ratio of animals showing tumor rejection was 40% when the anti-PD-1 antibody was combined with the WT1 vaccine. These results demonstrate that combining a WT1 vaccine and an immune checkpoint inhibitor enhances the suppression of tumor proliferation and improves the therapeutic response.

**Table 1: Ratio of animals showing tumor rejection**

| Mice showing tumor rejection (11 days after the tumor transplantation) | | % |
|---|---|---|
| Group a: | vehicle | 0 |
| Group b: | anti-PD-1 antibody | 0 |
| Group c: | vaccine | 20 |
| Group d: | anti-PD-1 antibody + vaccine | 40 |

### Example 12

### Enhancement with immune checkpoint inhibitor of in vivo suppression of tumor proliferation by vaccine

EL4-A24/Kb-WT1 tumor cells were suspended in Hanks' Balanced Salt Solution and intradermally transplanted to the ventral region of HLA-A*24:02 transgenic mice (3×10⁵ cells per mouse). The mice received a vehicle (Montanide and phosphate buffered saline) (Group a); Montanide and an anti-CTLA-4 antibody (Group b); a vaccine and an isotype control antibody (Group c); or a vaccine and an anti-CTLA-4 antibody (Group d). Six mice per group were used. Three, four and ten days after the tumor transplantation, for the mice of Groups a and b, a composition comprising water for injection was emulsified with an equal volume of Montanide and intradermally administered in two sites, the roots of forelimb and hindlimb (0.1 mL per administration per mouse). For the mice of Groups c and d, a composition comprising a WT1 killer peptide (compound of formula (3)) and a WT1 helper peptide (SEQ ID NO: 18) was emulsified with an equal volume of Montanide and intradermally administered to the HLA-A*24:02 transgenic mouse in two sites, the roots of forelimb and hindlimb (0.5 mg WT1 killer peptide and 0.375 mg WT1 helper peptide were administered per administration per mouse). One, four, seven and ten days after the tumor transplantation, phosphate buffered saline was intraperitoneally administered to the mice of Group a (0.1 mL per administration per mouse) . To the mice of Groups b and d, an anti-CTLA-4 antibody (Bio X Cell, clone UC10-4F10-11) was intraperitoneally administered (0.2 mg per administration per mouse). To the mice of Group c, an isotype control antibody (Bio X Cell, Armenian hamster IgG) was intraperitoneally administered (0.2 mg per administration per mouse). The tumor size was measured 21 days after the tumor transplantation and the tumor volume was calculated.

The results are shown in Fig. 26. The anti-CTLA-4 antibody (Group b) or the WT1 vaccine (Group c) did not significantly suppress the tumor proliferation compared to the vehicle (Group a) (parametric Dunnett's multiple test, NS: no significant difference). In contrast, the combination of the anti-CTLA-4 antibody and the WT1 vaccine significantly suppressed the tumor proliferation (Group d, *: p<0.05). These results demonstrate that combining a WT1 vaccine and an immune checkpoint inhibitor enhances the suppression of tumor proliferation.

### Example 13

### Effect of combined agent on immune response to WT1 antigen peptide or tumor cells of WT1 antigen peptide-specific CTLs induced by cocktail vaccine

A composition comprising a WT1 killer peptide (compound of formula (3)) and a WT1 helper peptide (SEQ ID NO: 18) was emulsified with an equal volume of Montanide and intradermally administered to the root of tail of the HLA-A*02:01 transgenic mouse (0.5 mg WT1 killer peptide and 0.375 mg WT1 helper peptide were administered per mouse). Alternatively, a composition comprising a WT1 killer peptide (compound of formula (3)) was emulsified with an equal volume of Montanide and intradermally administered to the root of tail of the HLA-A*02:01 transgenic mouse (0.5 mg WT1 killer peptide were administered per mouse). One week after the administration, the mice were euthanized with CO₂ gas and the spleens were removed from the mice to prepare spleen cells using CTM. The spleen cells were stained with a FITC-labeled anti-CD8 antibody (BD Pharmingen) and PE-labeled HLA tetramer against a WT1 killer peptide (SEQ ID NO: 2) (MBL) and analyzed for killer peptide-specific CTLs with a flow cytometer.

Also, a peptide solution comprising a WT1 killer peptide (SEQ ID NO: 2) was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10, seeded on a U-bottom 96 well plate at 3.85×10⁵ cells/well, and cultured for about three days at 37°C, 5% CO₂. The concentration of mouse IFN-γ in the culture supernatant was measured with an ELISA kit (R&D Systems).

Further, as described in Example 5, X-ray irradiated (50Gy) LLC-HHD-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the spleen cells at 3.5×10⁴ cells/well and cultured for about three days at 37°C, 5% CO₂ with CTM, an isotype control antibody, an immune checkpoint inhibitor, a costimulatory molecule agonist antibody, or a β-catenin inhibitor. The immune checkpoint inhibitor was an anti-PD-1 (BioLegend, clone 29F.1A12), anti-LAG-3 (BioLegend, clone C9B7W), anti-BTLA (BioLegend, clone 6A6), anit-PD-L1 (eBiosciecne, clone MIHS), or anti-VISTA (BioLegend, clone MH5A) antibody at a final concentration of 30 µg/mL. The costimulatory molecule agonist antibody was an anti-4-lBB (Bio X Cell, clone LOB12.3), anti-OX-40 (Bio X Cell, clone OX-86), or anti-GITR (BioLegend, clone DTA-1) antibody at a final concentration of 30 µg/mL. The isotype control antibody was Rat IgG2ax (BD Pharmingen) for the anti-PD-1, antibody; Rat IgG2a (Bio X Cell) for the anit-PD-L1 antibody; Armenian Hamster IgG (eBioscience) for the anti-BTLA and anti-VISTA antibodies; Rat IgGlK (eBiosciecne) for the anti-LAG-3, anti-4-1BB and anti-OX-40 antibodies; and Rat IgG2b (Bio X Cell) for the anti-GITR antibody. The β-catenin inhibitor was XAV939 at a final concentration of 5 µmol/L. The concentration of mouse IFN-γ in the culture supernatant was measured with an ELISA kit (R&D Systems).

The results are shown in Figs. 27-32. The flow cytometric analysis showed that the spleen cells from the mice that received the vaccine comprising the WT1 killer peptide (compound of formula (3)) and the WT1 helper peptide (SEQ ID NO: 18) (referred to as "cocktail vaccine a") contained CTLs specific to the WT1 killer peptide (SEQ ID NO: 2) 1.9 times as many as those in the spleen cells from the mice that received the vaccine comprising the WT1 killer peptide (compound of formula (3)) only (referred to as "killer vaccine a") (Fig. 27). When these spleen cells were cultured in the presence of the WT1 killer peptide (SEQ ID NO: 2), the spleen cells from the mice that received the cocktail vaccine a produced 2 times higher amount of IFN-γ than the spleen cells from the mice that received the killer vaccine a (Fig. 28). In contrast, when these spleen cells were first mixed with tumor cells and then cultured in the presence or absence of the WT1 killer peptide (SEQ ID NO: 2), the IFN-γ production from the spleen cells was decreased compared to the spleen cells that were not mixed with tumor cells prior to the culture with the WT1 killer peptide. Nevertheless, the spleen cells from the mice that received the cocktail vaccine a produced 12 times and 6.9 times higher amount of IFN-γ than the spleen cells from the mice that received the killer vaccine a, when cultured in the presence and absence of the WT1 killer peptide, respectively (Fig. 29). There results demonstrate that CTLs included in the spleen cells from the mouse that received the killer vaccine a were supressed by tumor cells more strongly than CTLs included in the spleen cells from the mouse that received the cocktail vaccine a. Also, when the spleen cells were cultured with an immune checkpoint inhibitor (Fig. 30), a costimulatory molecule agonist antibody (Fig. 31), or a β-catenin inhibitor (Fig. 32) in addition to the WT1 killer peptide in the presence of tumor cells, the spleen cells from the mouse that received the cocktail vaccine a were significantly activated by the combined agent in the same manner as Example 5. In contrast, the spleen cells from the mouse that received the killer vaccine a were not activated. These results demonstrate that it is important to include both a killer peptide and a helper peptide in a WT1 vaccine to increase the response of WT1 antigen peptide-specific CTLs to the WT1 antigen peptide or tumor cells by an immune checkpoint inhibitor, a costimulatory molecule agonist antibody or a β-catenin inhibitor.

### Example 14

### Effect of combined agent on immune response to WT1 antigen peptide or tumor cells of WT1 antigen peptide-specific CTLs induced by cocktail vaccine

A composition comprising a WT1 killer peptide (SEQ ID NO: 2) and a WT1 helper peptide (SEQ ID NO: 11) was emulsified with an equal volume of Montanide and intradermally administered to the root of tail of the HLA-A*02:01 transgenic mouse (0.3 mg WT1 killer peptide and 0.3 mg WT1 helper peptide were administered per mouse). Alternatively, a composition comprising the WT1 killer peptide (SEQ ID NO: 2) was emulsified with an equal volume of Montanide and intradermally administered to the root of tail of the HLA-A*02:01 transgenic mouse (0.3 mg WT1 killer peptide were administered per mouse). One week after the administration, the mice were euthanized with CO₂ gas and spleens were removed from the mice to prepare spleen cells using CTM. The spleen cells were stained with a FITC-labeled anti-CD8 antibody (BD Pharmingen) and PE-labeled HLA tetramer against a WT1 killer peptide (SEQ ID NO: 2) (MBL) and analyzed for killer peptide-specific CTLs with a flow cytometer.

Also, a peptide solution comprising a WT1 killer peptide (SEQ ID NO: 2) was added to a part of the spleen cells at a final concentration of 100 µg/mL, and the cells were stood for about one hour at 37°C, 5% CO₂. After excess peptides were washed out with CTM, the peptide-pulsed spleen cells and unpulsed spleen cells were mixed at a ratio of 1:10, seeded on a U-bottom 96 well plate at 3.85×10⁵ cells/well, and cultured for about three days at 37°C, 5% CO₂. The concentration of mouse IFN-γ in the culture supernatant was measured with an ELISA kit (R&D Systems).

Further, as described in Example 7, X-ray irradiated (50Gy) LLC-HHD-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the spleen cells at 3.5×10⁴ cells/well and cultured for about three days at 37°C, 5% CO₂ with an isotype control antibody, an immune checkpoint inhibitor, or a costimulatory molecule agonist antibody. The immune checkpoint inhibitor was an anti-PD-1 (BioLegend, clone 29F.1A12), anti-B7-H4 (BioLegend, clone HMH4-5G1), or anit-PD-I,1 (eBiosciecne, clone MIH5) antibody at a final concentration of 30 µg/mL. The costimulatory molecule agonist antibody was an anti-4-1BB (Bio X Cell, clone LOB12.3) or anti-OX-40 (Bio X Cell, clone OX-86) antibody at a final concentration of 30 µg/mL. The isotype control antibody was Rat IgG2ak (BD Pharmingen) for the anti-PD-1 antibody; Rat IgG2a (Bio X Cell) for the anit--PD-L1 antibody; Armenian Hamster IgG (e8ioscience) for the anti-B7-H4 antobody; and Rat IgG1κ (eBiosciecne) for the anti-4-1BB and anti-OX-40 antibodies. The concentration of mouse IFN-γ in the culture supernatant was measured with an ELISA kit (R&D Systems).

The results are shown in Figs. 33-39. The flow cytometric analysis showed that the spleen cells from the mouse that received the vaccine comprising the WT1 killer peptide (SEQ ID NO: 2) and the WT1 helper peptide (SEQ ID NO: 11) (referred to as "cocktail vaccine b") contained CTLs specific to the WT1 killer peptide (SEQ ID NO: 2) 1.3 times as many as those in the spleen cells from the mice that received the vaccine comprising the WT1 killer peptide (SEQ ID NO: 2) only (referred to as "killer vaccine b") (Fig. 33). When these spleen cells were cultured in the presence of the WT1 killer peptide (SEQ ID NO: 2), the spleen cells from the mouse that received the cocktail vaccine b produced 2.6 times higher amount of IFN-γ than the spleen cells from the mice that received the killer vaccine b (Figs. 34A-34B). When these spleen cells were first mixed with tumor cells and then cultured in the presence or absence of the WT1 killer peptide (SEQ ID NO: 2), the IFN-γ production from the spleen cells was decreased compared to the spleen cells that were not mixed with tumor cells prior to the culture with the WT1 killer peptide. When the spleen cells were cultured with an immune checkpoint inhibitor (Figs. 35-37) or a costimulatory molecule agonist antibody (Figs. 38-39) in addition to the WT1 killer peptide in the presence of tumor cells, the spleen cells from the mouse that received the cocktail vaccine b were significantly activated by the combined agent in the same manner as Example 7. In contrast, the spleen cells from the mouse that received the killer vaccine b were not activated. These results demonstrate that it is important to include both a killer peptide and a helper peptide in a WT1 vaccine to increase the response of WT1 antigen peptide-specific CTLs to WT1 antigen peptides or tumor cells by an immune checkpoint inhibitor or a costimulatory molecule agonist antibody.

### INDUSTRIAL APPLICABILITY

The invention is useful in the pharmaceutical field, for example in the development or manufacture of therapeutic and prophylactic compositions for cancer.

### SEQUENCE FREE TEXT

SEQ ID NO: 2 peptide
SEQ ID NO: 3 peptide
SEQ ID NO: 4 peptide
SEQ ID NO: 5 peptide
SEQ ID NO: 6 peptide
SEQ ID NO: 7 peptide
SEQ ID NO: 8 peptide
SEQ ID NO: 9 peptide
SEQ ID NO: 10 peptide
SEQ ID NO: 11 peptide
SEQ ID NO: 12 peptide
SEQ ID NO: 13 peptide
SEQ ID NO: 14 peptide
SEQ ID NO: 15 peptide
SEQ ID NO: 16 peptide
SEQ ID NO: 17 peptide
SEQ ID NO: 18 peptide
SEQ ID NO: 19 peptide
SEQ ID NO: 20 peptide
SEQ ID NO: 21 peptide
SEQ ID NO: 22 peptide
SEQ ID NO: 23 peptide
SEQ ID NO: 24 peptide
SEQ ID NO: 25 peptide
SEQ ID NO: 26 peptide
SEQ ID NO: 27 peptide
SEQ ID NO: 28 peptide
SEQ ID NO: 29 peptide
SEQ ID NO: 30 peptide
SEQ ID NO: 31 peptide
SEQ ID NO: 32 peptide
SEQ ID NO: 33 peptide
SEQ ID NO: 34 peptide
SEQ ID NO: 35 peptide
SEQ ID NO: 36 peptide
SEQ ID NO: 37 peptide
SEQ ID NO: 38 peptide
SEQ ID NO: 39 peptide
SEQ ID NO: 40 peptide
SEQ ID NO: 41 peptide
SEQ ID NO: 42 peptide
SEQ ID NO: 43 peptide
in compound of formula 1: SEQ ID NO:41 is bound via a C-C disulfide bond to SEQ ID NO:42
in compound of formula 2: SEQ ID NO:43 is bound via a C-C disulfide bond to SEQ ID NO:4
in compound of formula 3: SEQ ID NO:41 is bound via a C-C disulfide bond to SEQ ID NO:4
SEQ ID NO: 44 peptide
SEQ ID NO: 45 peptide
SEQ ID NO: 46 peptide

## Claims

1. A pharmaceutical composition for use in treating or preventing cancer, wherein said pharmaceutical composition is to be parenterally administered by injection, comprising:
(i) a WT1 antigen peptide or a pharmaceutically acceptable salt thereof, wherein the pharmaceutical composition is used in combination with an immunomodulator,
(ii) an immunomodulator, wherein the pharmaceutical composition is used in combination with a WT1 antigen peptide or a pharmaceutically acceptable salt thereof, or
(iii)a WT1 antigen peptide or a pharmaceutically acceptable salt thereof and an immunomodulator,
wherein the pharmaceutical composition further comprises a WT1 helper peptide or a pharmaceutically acceptable salt thereof, or the pharmaceutical composition is used in combination with a WT1 helper peptide or a pharmaceutically acceptable salt thereof,
wherein said WT1 antigen peptide or a pharmaceutically acceptable salt thereof is
a peptide consisting of the amino acid sequence selected from
RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6),
RVPGVAPTL (SEQ ID NO: 7),
VLDFAPPGA,(SEQ ID NO: 8),
C-CMTWNQMNL (SEQ ID NO: 9) (wherein the bond within C-C is a disulfide bond),
C-CYTWNQMNL (SEQ ID NO: 10) (wherein the bond within C-C is a disulfide bond),
RYFPNAPYL (SEQ ID NO: 21), and
YMFPNAPYL (SEQ ID NO: 26);
a peptide comprising an altered amino acid sequence of the amino acid sequence selected from SEQ ID NOS: 2-10, 21 and 26 that comprises deletion, substitution, or addition of one to several amino acids in the amino acid sequence and having a CTL induction activity; or
a compound selected from the group consisting of the compound of formula (1): 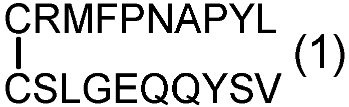
(wherein the bond within C-C is a disulfide bond), the compound of formula (2): 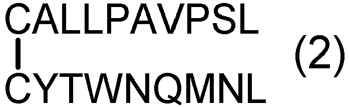
(wherein the bond within C-C is a disulfide bond), and the compound of formula (3): 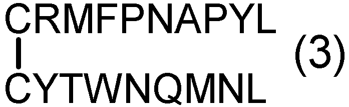
(wherein the bond within C-C is a disulfide bond);
or a pharmaceutically acceptable salt thereof, and
the WT1 helper peptide or a pharmaceutically acceptable salt thereof is
a peptide consisting of the amino acid sequence selected from
KRYFKLSHLQMHSRKH (SEQ ID NO: 11),
SGQARMFPNAPYLPSCLES (SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20), and
SGQAYMFPNAPYLPSCLES (SEQ ID NO: 37); or
a peptide comprising an altered amino acid sequence of the amino acid sequence selected from SEQ ID NOs: 11-20 and 37 that comprises deletion, substitution, or addition of one to several amino acids in the amino acid sequence and having a helper T cell induction activity; or a pharmaceutically acceptable salt thereof, and
the immunomodulator is at least one agent selected from the group consisting of
(a) an immune checkpoint inhibitor,
the immune checkpoint inhibitor is at least one agent directed to a molecule selected from the group consisting of
(1) CTLA-4,
(2) PD-1,
(3) LAG-3,
(4) BTLA,
(5) KIR,
(6) TIM-3,
(7) PD-L1,
(8) PD-L2,
(9) B7-H3,
(10) B7-H4,
(11) HVEM,
(12) GAL9,
(13) CD160,
(14) VISTA,
(15) BTNL2,
(16) TIGIT,
(17) PVR,
(18) BTN1A1,
(19) BTN2A2,
(20) BTN3A2, and
(21) CSF-1R,
(b) a costimulatory molecule agonist,
the costimulatory molecule agonist is at least one agent directed to a molecule selected from the group consisting of
(1) 4-1BB,
(2) 4-1BB-L,
(3) OX40,
(4) OX40-L,
(5) GITR,
(6) CD28,
(7) CD40,
(8) CD40-L,
(9) ICOS,
(10) ICOS-L,
(11) LIGHT, and
(12) CD27
(c) a Toll-like receptor (TLR) agonist,
the TLR agonist is at least one agent selected from the group consisting of
(1) a TLR1/2 agonist,
(2) a TLR2 agonist,
(3) a TLR3 agonist,
(4) a TLR4 agonist,
(5) a TLR5 agonist,
(6) a TLR6/2 agonist,
(7) a TLR7 agonist,
(8) a TLR7/8 agonist,
(9) a TLR7/9 agonist,
(10) a TLR8 agonist,
(11) a TLR9 agonist, and
(12) a TLR11 agonist, and
(d) a low-molecular inhibitor
the low-molecular inhibitor is at least one agent selected from the group consisting of
(1) a β-catenin inhibitor,
(2) a IDO inhibitor,
(3) a COX-2 inhibitor,
(4) a CXCR4 inhibitor,
(5) a STATS inhibitor, and
(6) a multikinase inhibitor.

2. The pharmaceutical composition for use according to claim 1, wherein said pharmaceutical composition is to be parenterally administered by injection,
wherein the WT1 antigen peptide or a pharmaceutically acceptable salt thereof is
the compound of formula (3): 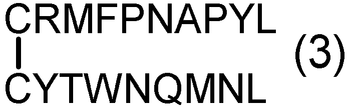
(wherein the bond within C-C is a disulfide bond);
or a pharmaceutically acceptable salt thereof.

3. The pharmaceutical composition for use according to claim 1 or 2, wherein said pharmaceutical composition is to be parenterally administered by injection,
wherein the WT1 helper peptide or a pharmaceutically acceptable salt thereof is
a peptide consisting of the amino acid sequence selected from KRYFKLSHLQMHSRKH (SEQ ID NO: 11),
SGQARMFPNAPYLPSCLES (SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20), and
SGQAYMFPNAPYLPSCLES (SEQ ID NO: 37) or
a pharmaceutically acceptable salt thereof.

4. The pharmaceutical composition for use according to any one of claims 1-3, wherein said pharmaceutical composition is to be parenterally administered by injection,
wherein the WT1 helper peptide or a pharmaceutically acceptable salt thereof is
WAPVLDFAPPGASAYGSL (SEQ ID NO: 18),
or a pharmaceutically acceptable salt thereof.

5. The pharmaceutical composition for use according to any one of claims 1-4, wherein said pharmaceutical composition is to be parenterally administered by injection, wherein the pharmaceutical composition is used as a cancer vaccine.

6. The pharmaceutical composition for use according to any one of claims 1-5, wherein said pharmaceutical composition is to be parenterally administered by injection, wherein the immune checkpoint inhibitor is at least one agent directed to a molecule selected from the group consisting of CTLA-4, PD-1, LAG-3, BTLA, TIM-3, PD-L1, B7-H4, HVEM, CD160, VISTA, TIGIT and PVR.

7. The pharmaceutical composition for use according to any one of claims 1-6, wherein said pharmaceutical composition is to be parenterally administered by injection, wherein the immune checkpoint inhibitor is an antibody.

8. The pharmaceutical composition for use according to claim 7, wherein said pharmaceutical composition is to be parenterally administered by injection, wherein the immune checkpoint inhibitor is an antibody against PD-1 or PD-L1.

9. The pharmaceutical composition for use according to claim 8, wherein said pharmaceutical composition is to be parenterally administered by injection, wherein the antibody against PD-1 is Nivolumab or Pembrolizumab.

10. The pharmaceutical composition for use according to claim 8, wherein said pharmaceutical composition is to be parenterally administered by injection, wherein the antibody against PD-L1 is Durvalumab, MPDL3280A or BMS-936559.

11. The pharmaceutical composition for use according to any one of claims 1-5, wherein said pharmaceutical composition is to be parenterally administered by injection, wherein the costimulatory molecule agonist is at least one agent directed to a molecule selected from the group consisting of 4-1BB, OX40, GITR, CD40 and ICOS.

12. The pharmaceutical composition for use according to any one of claims 1-5, wherein said pharmaceutical composition is to be parenterally administered by injection, wherein the immune activating agent is at least one of Toll-like receptor (TLR) agonists selected from the group consisting of a TLR3 agonist, a TLR7 agonist, a TLR7/8 agonist and a TLR9 agonist.

13. The pharmaceutical composition for use according to any one of claims 1-5, wherein said pharmaceutical composition is to be parenterally administered by injection, wherein the low-molecular inhibitor is a β-catenin inhibitor.

14. The pharmaceutical composition for use according to any one of claims 1-13, wherein said pharmaceutical composition is to be parenterally administered by injection, wherein the cancer is selected from the group consisting of leukemia, myelodysplastic syndrome, multiple myeloma, malignant lymphoma, gastric cancer, colorectal cancer, lung cancer, breast cancer, germ cell cancer, liver cancer, skin cancer, urinary bladder cancer, prostate cancer, uterine cancer, cervical cancer, ovarian cancer, brain tumor, bone cancer, pancreatic cancer, cancer of the head or neck, cutaneous or intraocular malignant melanoma, rectal cancer, cancer of the anal region, testicular cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia such as acute myeloid leukemia, chronic myeloid leukemia, acute lymphoblastic leukemia, or chronic lymphocytic leukemia, childhood solid tumor, lymphocytic lymphoma, cancer of the kidney or ureter, carcinoma of the renal pelvis, central nervous system (CNS) tumor, primary CNS lymphoma, tumor angiogenesis, spinal tumor, brainstem glioma, pituitary adenoma, Kaposi's sarcoma, epidermoid cancer, squamous cell cancer, T-cell lymphoma, glioblastoma multiforme, malignant melanoma, non-small cell lung cancer, renal cell carcinoma, and asbestos-induced cancer

15. A kit for use in treating or preventing cancer, comprising the WT1 antigen peptide or a pharmaceutically acceptable salt thereof, the WT1 helper peptide or a pharmaceutically acceptable salt thereof, and the immunomodulator as defined in any one of claims 1-14 which are to be parenterally administered by injection.

## Patentansprüche

1. Pharmazeutische Zusammensetzung zur Verwendung bei der Behandlung oder Vorbeugung von Krebs, wobei die pharmazeutische Zusammensetzung parenteral durch Injektion zu verabreichen ist, umfassend:
(i) ein WT1-Antigenpeptid oder ein pharmazeutisch verträgliches Salz davon, wobei die pharmazeutische Zusammensetzung in Kombination mit einem Immunmodulator verwendet wird,
(ii) ein Immunmodulator, wobei die pharmazeutische Zusammensetzung in Kombination mit einem WT1-Antigenpeptid oder einem pharmazeutisch verträglichen Salz davon verwendet wird, oder
(iii) ein WT1-Antigenpeptid oder ein pharmazeutisch verträgliches Salz davon und ein Immunmodulator,
wobei die pharmazeutische Zusammensetzung des Weiteren ein WT1-Helferpeptid oder ein pharmazeutisch verträgliches Salz davon umfasst, oder die pharmazeutische Zusammensetzung in Kombination mit einem WT1-Helferpeptid oder einem pharmazeutisch verträglichen Salz davon verwendet wird,
wobei das WT1-Antigenpeptid oder ein pharmazeutisch verträgliches Salz davon ein Peptid ist, das aus der Aminosäuresequenz besteht, die ausgewählt ist aus:
RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6),
RVPGVAPTL (SEQ ID NO: 7),
VLDFAPPGA (SEQ ID NO: 8),
C-CMTWNQMNL (SEQ ID NO: 9) (wobei die Bindung innerhalb C-C eine Disulfid-Brücke ist),
C-CYTWNQMNL (SEQ ID NO: 10) (wobei die Bindung innerhalb C-C eine Disulfid-Brücke ist),
RYFPNAPYL (SEQ ID NO: 21) und
YMFPNAPYL (SEQ ID NO: 26);
einem Peptid, das eine veränderte Aminosäuresequenz der Aminosäuresequenz ausgewählt aus SEQ ID NOS: 2-10, 21 und 26 umfasst, die eine Deletion, Substitution oder Hinzufügung von einer bis zu mehreren Aminosäuren in der Aminosäuresequenz umfasst und CTL-Induktions-Aktivität aufweist; oder
einer Verbindung ausgewählt aus der Gruppe bestehend aus der Verbindung der Formel (1): 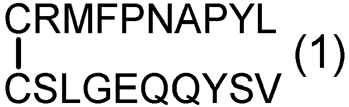
(wobei die Bindung innerhalb C-C eine Disulfid-Brücke ist), der Verbindung der Formel (2): 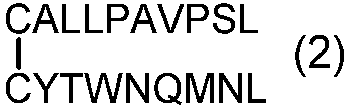
(wobei die Bindung innerhalb C-C eine Disulfid-Brücke ist), und der Verbindung der Formel (3): 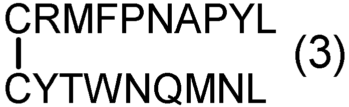
(wobei die Bindung innerhalb C-C eine Disulfid-Brücke ist);
oder einem pharmazeutisch verträglichen Salz davon, und
das WT1-Helferpeptid oder das pharmazeutisch verträgliche Salz davon ein Peptid ist, das aus der Aminosäuresequenz besteht, die ausgewählt ist aus:
KRYFKLSHLQMHSRKH (SEQ ID NO: 11),
SGQARMFPNAPYLPSCLES(SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20) und
SGQAYMFPNAPYLPSCLES (SEQ ID NO: 37); oder
einem Peptid, das eine veränderte Aminosäuresequenz der Aminosäuresequenz ausgewählt aus SEQ ID NOS: 11-20 und 37 umfasst, die eine Deletion, Substitution oder Hinzufügung von einer bis zu mehreren Aminosäuren in der Aminosäuresequenz umfasst und eine T-Helferzell-Induktions-Aktivität aufweist; oder einem pharmazeutisch verträglichen Salz davon, und
der Immunmodulator mindestens ein Agens ist, ausgewählt aus der Gruppe bestehend aus:
(a) einem Immun-Checkpoint-Inhibitor,
wobei der Immun-Checkpoint-Inhibitor mindestens ein Agens ist, das auf ein Molekül gerichtet ist, das ausgewählt ist aus der Gruppe bestehend aus:
(1) CTLA-4,
(2) PD-1,
(3) LAG-3,
(4) BTLA,
(5) KIR,
(6) TIM-3,
(7) PD-L1,
(8) PD-L2,
(9) B7-H3,
(10) B7-H4,
(11) HVEM,
(12) GAL9,
(13) CD160,
(14) VISTA,
(15) BTNL2,
(16) TIGIT,
(17) PVR,
(18) BTN1A1,
(19) BTN2A2,
(20) BTN3A2 und
(21) CSF-1R;
(b) einem co-stimulatorischen Molekül-Agonisten,
wobei der co-stimulatorische Molekül-Agonist mindestens ein Agens ist, das auf ein Molekül gerichtet ist, das ausgewählt ist aus der Gruppe bestehend aus:
(1) 4-1BB,
(2) 4-1BB-L,
(3) OX40,
(4) OX40-L,
(5) GITR,
(6) CD28,
(7) CD40,
(8) CD40-L,
(9) ICOS,
(10) ICOS-L,
(11) LIGHT und
(12) CD27;
(c) einem Toll-like-Rezeptor (TLR)-Agonisten,
wobei der TLR-Agonist mindestens ein Agens ist, das ausgewählt ist aus der Gruppe bestehend aus:
(1) einem TLR1/2-Agonisten,
(2) einem TLR2-Agonisten,
(3) einem TLR3-Agonisten,
(4) einem TLR4-Agonisten,
(5) einem TLR5-Agonisten,
(6) einem TLR6/2-Agonisten,
(7) einem TLR7-Agonisten,
(8) einem TLR7/8-Agonisten,
(9) einem TLR7/9-Agonisten,
(10) einem TLR8-Agonisten,
(11) einem TLR9-Agonisten und
(12) einem TLR11-Agonisten; und
(d) einem niedermolekularen Inhibitor,
wobei der niedermolekulare Inhibitor mindestens ein Agens ist, das ausgewählt ist aus der Gruppe bestehend aus:
(1) einem β-Catenin-Inhibitor,
(2) einem IDO-Inhibitor,
(3) einem COX-2-Inhibitor,
(4) einem CXCR4-Inhibitor,
(5) einem STAT3-Inhibitor und
(6) einem Multikinase-Inhibitor.

2. Die pharmazeutische Zusammensetzung zur Verwendung nach Anspruch 1, wobei die pharmazeutische Zusammensetzung parenteral durch Injektion zu verabreichen ist,
wobei das WT1-Antigenpeptid oder ein pharmazeutisches Salz davon die Verbindung der Formel (3): 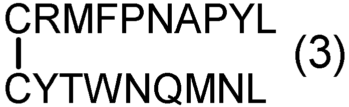
(wobei die Bindung innerhalb C-C eine Disulfid-Brücke ist);
oder ein pharmazeutisch verträgliches Salz davon ist.

3. Pharmazeutische Zusammensetzung zur Verwendung nach Anspruch 1 oder 2, wobei die pharmazeutische Zusammensetzung parenteral durch Injektion zu verabreichen ist,
wobei das WT1-Helferpeptid oder ein pharmazeutisch verträgliches Salz davon ein Peptid, das aus der Aminosäuresequenz besteht, ausgewählt aus:
KRYFKLSHLQMHSRKH (SEQ ID NO: 11),
SGQARMFPNAPYLPSCLES(SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20) und
SGQAYMFPNAPYLPSCLES (SEQ ID NO: 37); oder
ein pharmazeutisch verträgliches Salz davon ist.

4. Pharmazeutische Zusammensetzung zur Verwendung nach einem der Ansprüche 1 bis 3, wobei die pharmazeutische Zusammensetzung parenteral durch Injektion zu verabreichen ist,
wobei das WT1-Helferpeptid oder ein pharmazeutisch verträgliches Salz davon WAPVLDFAPPGASAYGSL (SEQ ID NO: 18);
oder ein pharmazeutisch verträgliches Salz davon ist.

5. Pharmazeutische Zusammensetzung zur Verwendung nach einem der Ansprüche 1 bis 4, wobei die pharmazeutische Zusammensetzung parenteral durch Injektion zu verabreichen ist, wobei die pharmazeutische Zusammensetzung als Krebs-Impfstoff verwendet wird.

6. Pharmazeutische Zusammensetzung zur Verwendung nach einem der Ansprüche 1 bis 5, wobei die pharmazeutische Zusammensetzung parenteral durch Injektion zu verabreichen ist, wobei der Immun-Checkpoint-Inhibitor mindestens ein Agens ist, das auf ein Molekül gerichtet ist, das ausgewählt ist aus der Gruppe bestehend aus CTLA-4, PD-1, LAG-3, BTLA, TIM-3, PD-L1, B7-H4, HVEM, CD160, VISTA, TIGIT und PVR.

7. Pharmazeutische Zusammensetzung zur Verwendung nach einem der Ansprüche 1 bis 6, wobei die pharmazeutische Zusammensetzung parenteral durch Injektion zu verabreichen ist, wobei der Immun-Checkpoint-Inhibitor ein Antikörper ist.

8. Pharmazeutische Zusammensetzung zur Verwendung nach Anspruch 7, wobei die pharmazeutische Zusammensetzung parenteral durch Injektion zu verabreichen ist, wobei der Immun-Checkpoint-Inhibitor ein Antikörper gegen PD-1 oder PD-L1 ist.

9. Pharmazeutische Zusammensetzung zur Verwendung nach Anspruch 8, wobei die pharmazeutische Zusammensetzung parenteral durch Injektion zu verabreichen ist, wobei der Antikörper gegen PD-1 Nivolumab oder Pembrolizumab ist.

10. Pharmazeutische Zusammensetzung zur Verwendung nach Anspruch 8, wobei die pharmazeutische Zusammensetzung parenteral durch Injektion zu verabreichen ist, wobei der Antikörper gegen PD-L1 Durvalumab, MPDL3280A oder BMS-936559 ist.

11. Pharmazeutische Zusammensetzung zur Verwendung nach einem der Ansprüche 1 bis 5, wobei die pharmazeutische Zusammensetzung parenteral durch Injektion zu verabreichen ist, wobei der co-stimulatorische Molekül-Agonist mindestens ein Agens ist, das auf ein Molekül gerichtet ist, das ausgewählt ist aus der Gruppe bestehend aus 4-1BB, OX40, GITR, CD40 und ICOS.

12. Pharmazeutische Zusammensetzung zur Verwendung nach einem der Ansprüche 1 bis 5, wobei die pharmazeutische Zusammensetzung parenteral durch Injektion zu verabreichen ist, wobei das immun-aktivierende Agens mindestens einer der Toll-like-Rezeptor (TLR)-Agonisten ist, der ausgewählt ist aus der Gruppe bestehend aus einem TLR3-Agonisten, einem TLR7-Agonisten, einem TLR7/8-Agonisten und einem TLR9-Agonisten.

13. Pharmazeutische Zusammensetzung zur Verwendung nach einem der Ansprüche 1 bis 5, wobei die pharmazeutische Zusammensetzung parenteral durch Injektion zu verabreichen ist, wobei der niedermolekulare Inhibitor ein β-Catenin-Inhibitor ist.

14. Pharmazeutische Zusammensetzung zur Verwendung nach einem der Ansprüche 1 bis 13, wobei die pharmazeutische Zusammensetzung parenteral durch Injektion zu verabreichen ist, wobei die Krebserkrankung ausgewählt ist aus der Gruppe bestehend aus Leukämie, myelodysplastischem Syndrom, multiplem Myelom, malignem Lymphom, Magenkrebs, kolorektalem Karzinom, Lungenkrebs, Brustkrebs, Keimzelltumor, Leberkrebs, Hautkrebs, Blasenkrebs, Prostatakrebs, Uteruskarzinom, Gebärmutterhalskrebs, Eierstockkrebs, Hirntumor, Knochenkrebs, Bauchspeicheldrüsenkrebs, Kopf-Hals-Karzinom, Hautmelanom oder intraokulärem malignem Melanom, Rektumkarzinom, Krebs in der Analregion, Hodenkrebs, Eileiterkrebs, Endometriumkarzinom, Zervixkarzinom, Vaginalkarzinom, Vulvakarzinom, Hodgkin-Lymphom, Non-Hodgkin-Lymphom, Speiseröhrenkrebs, Dünndarmkrebs, endokrinen Tumoren, Schilddrüsenkrebs, Tumoren der Nebenschilddrüsen, Nebennierenkrebs, Weichteilsarkom, Harnröhrenkrebs, Peniskrebs, chronischer oder akuter Leukämie wie z.B. akuter myeloischer Leukämie, chronischer myeloischer Leukämie, akuter lymphoblastischer Leukämie oder chronischer lymphatischer Leukämie, soliden Tumoren bei Kindern, lymphozytischem Lymphom, Nieren- oder Harnleiterkrebs, Nierenbeckenkarzinom, Tumor des Zentralnervensystems (ZNS-Tumor), primärem ZNS-Lymphom, Tumorangiogenese, spinalem Tumor, Hirnstammgliom, Hypophysenadenom, Kaposi-Sarkom, Plattenepithelkarzinom, Epithelioma spinocellulare, T-Zell-Lymphom, Gliablastoma multiforma, malignem Melanom, nicht-kleinzelligem Lungenkrebs, Nierenzellkarzinom und Asbestose-induziertem Krebs.

15. Kit zur Verwendung bei der Behandlung oder Vorbeugung von Krebs, umfassend das WT1-Antigenpeptid oder ein pharmazeutisch verträgliches Salz davon, das WT1-Helferpeptid oder ein pharmazeutisch verträgliches Salz davon und den Immunmodulator wie in einem der Ansprüche 1 bis 14 definiert, die parenteral durch Injektion zu verabreichen sind.

## Revendications

1. Composition pharmaceutique pour une utilisation dans le traitement ou la prévention d'un cancer, laquelle composition pharmaceutique est destinée à être administrée par voie parentérale par injection, comprenant :
(i) un peptide antigène de WT1 ou un sel pharmaceutiquement acceptable de celui-ci, la composition pharmaceutique étant utilisée en combinaison avec un immunomodulateur,
(ii) un immunomodulateur, la composition pharmaceutique étant utilisée en combinaison avec un peptide antigène de WT1 ou un sel pharmaceutiquement acceptable de celui-ci, ou
(iii) un peptide antigène de WT1 ou un sel pharmaceutiquement acceptable de celui-ci et un immunomodulateur,
laquelle composition pharmaceutique comprend en outre un peptide auxiliaire de WT1 ou un sel pharmaceutiquement acceptable de celui-ci, ou laquelle composition pharmaceutique est utilisée en combinaison avec un peptide auxiliaire de WT1 ou un sel pharmaceutiquement acceptable de celui-ci,
dans laquelle ledit peptide antigène de WT1 ou un sel pharmaceutiquement acceptable de celui-ci est
un peptide consistant en la séquence d'acides aminés choisie parmi RMFPNAPYL (SEQ ID NO : 2),
CMTWNQMNL (SEQ ID NO : 3),
CYTWNQMNL (SEQ ID NO : 4),
ALLPAVPSL (SEQ ID NO : 5),
SLGEQQYSV (SEQ ID NO : 6),
RVPGVAPTL (SEQ ID NO : 7),
VLDFAPPGA, (SEQ ID NO: 8),
C-CMTWNQMNL (SEQ ID NO : 9) (dans laquelle la liaison dans C-C est une liaison disulfure),
C-CYTWNQMNL (SEQ ID NO : 10) (dans laquelle la liaison dans C-C est une liaison disulfure),
RYFPNAPYL (SEQ ID NO : 21), et
YMFPNAPYL (SEQ ID NO : 26) ;
un peptide comprenant une séquence d'acides aminés altérée de la séquence d'acides aminés choisie parmi les SEQ ID NO : 2 à 10, 21 et 26 qui comprend une délétion, une substitution ou une addition d'un à plusieurs acides aminés dans la séquence d'acides aminés et ayant une activité d'induction de CTL ; ou
un composé choisi dans le groupe constitué par
le composé de formule (1) : 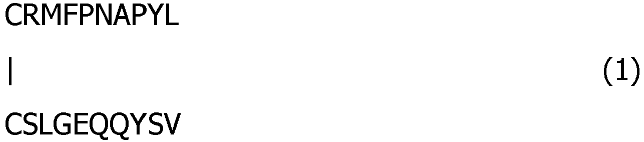
(dans laquelle la liaison dans C-C est une liaison disulfure), le composé de formule (2) : 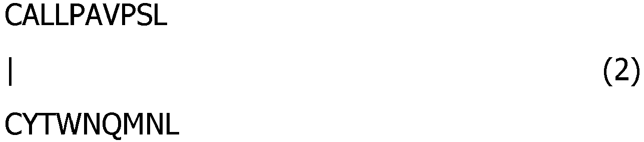
(dans laquelle la liaison dans C-C est une liaison disulfure), et le composé de formule (3) : 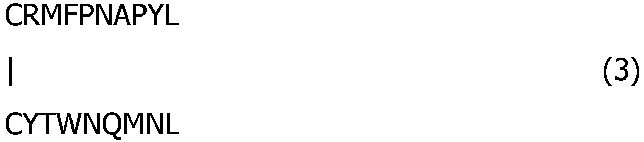
(dans laquelle la liaison dans C-C est une liaison disulfure) ;
ou un sel pharmaceutiquement acceptable de celui-ci, et
le peptide auxiliaire de WT1 ou un sel pharmaceutiquement acceptable de celui-ci est
un peptide consistant en la séquence d'acides aminés choisie parmi
KRYFKLSHLQMHSRKH (SEQ ID NO : 11),
SGQARMFPNAPYLPSCLES (SEQ ID NO : 12),
RSDELVRHHNMHQRNMTKL (SEQ ID NO : 13),
PGCNKRYFKLSHLQMHSRKHTG (SEQ ID NO : 14),
CNKRYFKLSHLQMHSRK (SEQ ID NO : 15),
CNKRYFKLSHLQMHSRKH (SEQ ID NO : 16),
CNKRYFKLSHLQMHSRKHTG (SEQ ID NO : 17),
WAPVLDFAPPGASAYGSL (SEQ ID NO : 18),
CWAPVLDFAPPGASAYGSL (SEQ ID NO : 19),
WAPVLDFAPPGASAYGSLC (SEQ ID NO : 20), et
SGQAYMFPNAPYLPSCLES (SEQ ID NO : 37) ; ou
un peptide comprenant une séquence d'acides aminés altérée de la séquence d'acides aminés choisie parmi les SEQ ID NO : 11 à 20 et 37 qui comprend une délétion, une substitution ou une addition d'un à plusieurs acides aminés dans la séquence d'acides aminés et ayant une activité d'induction de cellules T auxiliaires ;
ou un sel pharmaceutiquement acceptable de celui-ci, et
l'immunomodulateur est au moins un agent choisi dans le groupe constitué par
(a) un inhibiteur de point de contrôle immunitaire,
l'inhibiteur de point de contrôle immunitaire qui est au moins un agent dirigé vers une molécule choisie dans le groupe constitué par
(1) CTLA-4,
(2) PD-1,
(3) LAG-3,
(4) BTLA,
(5) KIR,
(6) TIM-3,
(7) PD-L1,
(8) PD-L2,
(9) B7-H3,
(10) B7-H4,
(11) HVEM,
(12) GAL9,
(13) CD160,
(14) VISTA,
(15) BTNL2,
(16) TIGIT,
(17) PVR,
(18) BTN1A1,
(19) BTN2A2,
(20) BTN3A2, et
(21) CSF-1R,
(b) un agoniste moléculaire de costimulation,
l'agoniste moléculaire de costimulation est au moins un agent dirigé vers une molécule choisie dans le groupe constitué par
(1) 4-1BB,
(2) 4-1BB-L,
(3) OX40,
(4) OX40-L,
(5) GITR,
(6) CD28,
(7) CD40,
(8) CD40-L,
(9) ICOS,
(10) ICOS-L,
(11) LIGHT, et
(12) CD27,
(c) un agoniste de récepteur de type Toll (TLR),
l'agoniste de TLR est au moins un agent choisi dans le groupe constitué par
(1) un agoniste de TLR1/2,
(2) un agoniste de TLR2,
(3) un agoniste de TLR3,
(4) un agoniste de TLR4,
(5) un agoniste de TLR5,
(6) un agoniste de TLR6/2,
(7) un agoniste de TLR7,
(8) un agoniste de TLR7/8,
(9) un agoniste de TLR7/9,
(10) un agoniste de TLR8,
(11) un agoniste de TLR9, et
(12) un agoniste de TLR11, et
(d) un inhibiteur de faible poids moléculaire,
l'inhibiteur de faible poids moléculaire est au moins un agent choisi dans le groupe constitué par
(1) un inhibiteur de β-caténine,
(2) un inhibiteur d'IDO,
(3) un inhibiteur de COX-2,
(4) un inhibiteur de CXCR4,
(5) un inhibiteur de STAT3, et
(6) un inhibiteur de multikinase.

2. Composition pharmaceutique pour une utilisation selon la revendication 1, laquelle composition pharmaceutique est destinée à être administrée par voie parentérale par injection,
dans laquelle le peptide antigène de WT1 ou un sel pharmaceutiquement acceptable de celui-ci est
le composé de formule (3) : 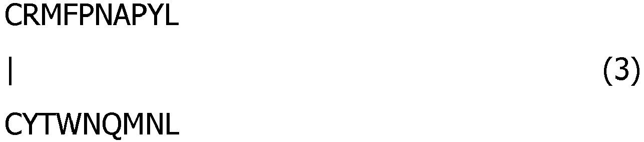
(dans laquelle la liaison dans C-C est une liaison disulfure) ;
ou un sel pharmaceutiquement acceptable de celui-ci.

3. Composition pharmaceutique pour une utilisation selon la revendication 1 ou 2, laquelle composition pharmaceutique est destinée à être administrée par voie parentérale par injection,
dans laquelle le peptide auxiliaire de WT1 ou un sel pharmaceutiquement acceptable de celui-ci est
un peptide consistant en la séquence d'acides aminés choisie parmi
KRYFKLSHLQMHSRKH (SEQ ID NO : 11),
SGQARMFPNAPYLPSCLES (SEQ ID NO : 12),
RSDELVRHHNMHQRNMTKL (SEQ ID NO : 13),
PGCNKRYFKLSHLQMHSRKHTG (SEQ ID NO : 14),
CNKRYFKLSHLQMHSRK (SEQ ID NO : 15),
CNKRYFKLSHLQMHSRKH (SEQ ID NO : 16),
CNKRYFKLSHLQMHSRKHTG (SEQ ID NO : 17),
WAPVLDFAPPGASAYGSL (SEQ ID NO : 18),
CWAPVLDFAPPGASAYGSL (SEQ ID NO : 19),
WAPVLDFAPPGASAYGSLC (SEQ ID NO : 20), et
SGQAYMFPNAPYLPSCLES (SEQ ID NO : 37),
ou un sel pharmaceutiquement acceptable de celui-ci.

4. Composition pharmaceutique pour une utilisation selon l'une quelconque des revendications 1 à 3, laquelle composition pharmaceutique est destinée à être administrée par voie parentérale par injection,
dans laquelle le peptide auxiliaire de WT1 ou un sel pharmaceutiquement acceptable de celui-ci est
WAPVLDFAPPGASAYGSL (SEQ ID NO : 18),
ou un sel pharmaceutiquement acceptable de celui-ci.

5. Composition pharmaceutique pour une utilisation selon l'une quelconque des revendications 1 à 4, laquelle composition pharmaceutique est destinée à être administrée par voie parentérale par injection, laquelle composition pharmaceutique est utilisée en tant que vaccin anticancéreux.

6. Composition pharmaceutique pour une utilisation selon l'une quelconque des revendications 1 à 5, laquelle composition pharmaceutique est destinée à être administrée par voie parentérale par injection, dans laquelle l'inhibiteur de point de contrôle immunitaire est au moins un agent dirigé vers une molécule choisie dans le groupe constitué par CTLA-4, PD-1, LAG-3, BTLA, TIM-3, PD-L1, B7-H4, HVEM, CD160, VISTA, TIGIT et PVR.

7. Composition pharmaceutique pour une utilisation selon l'une quelconque des revendications 1 à 6, laquelle composition pharmaceutique est destinée à être administrée par voie parentérale par injection, dans laquelle l'inhibiteur de point de contrôle immunitaire est un anticorps.

8. Composition pharmaceutique pour une utilisation selon la revendication 7, laquelle composition pharmaceutique est destinée à être administrée par voie parentérale par injection, dans laquelle l'inhibiteur de point de contrôle immunitaire est un anticorps dirigé contre PD-1 ou PD-L1.

9. Composition pharmaceutique pour une utilisation selon la revendication 8, laquelle composition pharmaceutique est destinée à être administrée par voie parentérale par injection, dans laquelle l'anticorps dirigé contre PD-1 est le Nivolumab ou le Pembrolizumab.

10. Composition pharmaceutique pour une utilisation selon la revendication 8, laquelle composition pharmaceutique est destinée à être administrée par voie parentérale par injection, dans laquelle l'anticorps dirigé contre PD-L1 est le Durvalumab, MPDL3280A ou BMS-936559.

11. Composition pharmaceutique pour une utilisation selon l'une quelconque des revendications 1 à 5, laquelle composition pharmaceutique est destinée à être administrée par voie parentérale par injection, dans laquelle l'agoniste moléculaire de costimulation est au moins un agent dirigé vers une molécule choisie dans le groupe constitué par 4-1BB, OX40, GITR, CD40 et ICOS.

12. Composition pharmaceutique pour une utilisation selon l'une quelconque des revendications 1 à 5, laquelle composition pharmaceutique est destinée à être administrée par voie parentérale par injection, dans laquelle l'agent d'activation immunitaire est au moins l'un des agonistes de récepteur de type Toll (TLR) choisis dans le groupe constitué par un agoniste de TLR3, un agoniste de TLR7, un agoniste de TLR7/8 et un agoniste de TLR9.

13. Composition pharmaceutique pour une utilisation selon l'une quelconque des revendications 1 à 5, laquelle composition pharmaceutique est destinée à être administrée par voie parentérale par injection, dans laquelle l'inhibiteur de faible poids moléculaire est un inhibiteur de β-caténine.

14. Composition pharmaceutique pour une utilisation selon l'une quelconque des revendications 1 à 13, laquelle composition pharmaceutique est destinée à être administrée par voie parentérale par injection, dans laquelle le cancer est choisi dans le groupe constitué par une leucémie, un syndrome myélodysplasique, un myélome multiple, un lymphome malin, un cancer gastrique, un cancer colorectal, un cancer pulmonaire, un cancer mammaire, un cancer à cellules germinales, un cancer du foie, un cancer de la peau, un cancer de la vessie, un cancer de la prostate, un cancer utérin, un cancer du col, un cancer ovarien, une tumeur cérébrale, un cancer des os, un cancer du pancréas, un cancer de la tête ou du cou, un mélanome malin cutané ou intraoculaire, un cancer rectal, un cancer de la région anale, un cancer testiculaire, un carcinome des trompes de Fallope, un carcinome de l'endomètre, un carcinome du col, un carcinome du vagin, un carcinome de la vulve, une maladie de Hodgkin, un lymphome non hodgkinien, un cancer de l'œsophage, un cancer de l'intestin grêle, un cancer du système endocrinien, un cancer de la glande thyroïde, un cancer de la glande parathyroïde, un cancer de la glande surrénale, un sarcome de tissu mou, un cancer de l'urètre, un cancer du pénis, une leucémie chronique ou aiguë telle qu'une leucémie myéloïde aiguë, une leucémie myéloïde chronique, une leucémie lymphoblastique aiguë, ou une leucémie lymphocytaire chronique, une tumeur solide chez un enfant, un lymphome lymphocytaire, un cancer rénal ou de l'uretère, un carcinome du pelvis rénal, une tumeur du système nerveux central (CNS), un lymphome du CNS primaire, une angiogenèse tumorale, une tumeur spinale, un gliome du tronc cérébral, un adénome pituitaire, un sarcome de Kaposi, un cancer épidermoïde, un cancer à cellules squameuses, un lymphome à cellules T, un glioblastome multiforme, un mélanome malin, un cancer pulmonaire non à petites cellules, un carcinome à cellules rénales, et un cancer induit par l'amiante.

15. Trousse pour une utilisation dans le traitement ou la prévention d'un cancer, comprenant le peptide antigène de WT1 ou un sel pharmaceutiquement acceptable de celui-ci, le peptide auxiliaire de WT1 ou un sel pharmaceutiquement acceptable de celui-ci, et l'immunomodulateur tels que définis dans l'une quelconque des revendications 1 à 14 qui sont destinés à être administrés par voie parentérale par injection.
